# FASCICULE DE BREVET EUROPEEN

(11) **EP 1 919 474 B1**
(45) Date de publication et mention de la délivrance du brevet: **05.08.2009**
(21) Numéro de dépôt: 06808267.6
(22) Date de dépôt: 31.08.2006
(51) Int. Cl.: A61K 31/437, C07D 471/04, A61P 3/06

(54) **DERIVES DE PYRROLOPYRIDINE ET LEURS UTILISATIONS COMME MODULATEURS DES RECEPTEURS PPAR**
PYRROLOPYRIDIN-DERIVATE UND DEREN VERWENDUNG ALS PPAR-REZEPTORMODULATOREN
PYRROLOPYRIDINE DERIVATIVES AND USE OF SAME AS PPAR RECEPTOR MODULATORS

(30) Priorité: 01.09.2005 US 713459 P; 14.10.2005 FR 0510482
(43) Date de publication de la demande: 14.05.2008
(73) Titulaire: LABORATOIRES FOURNIER SA, 21000 Dijon (FR)
(72) Inventeur: BOUBIA, Benaïssa, F-21850 Saint Apollinaire (FR); BARTH, Martine, F-21380 Asnieres les Dijon (FR); BINET, Jean, F-21121 Fontaine les Dijon (FR); DODEY, Pierre, F-21121 Fontaine les Dijon (FR); LEGENDRE, Christiane, F-21370 Velard Sur Ouche (FR); POUPARDIN-OLIVIER, Olivia, F-21490 Varois et Chaignot (FR)
(74) Mandataire: Hubert, Philippe
(86) Numéro de dépôt international: PCT/FR2006/050827
(87) Numéro de publication internationale: WO 2007/026104

(56) Documents cités:
- WO-A-03/044018
- WO-A-20/04005253
- WO-A-20/05009958

## Description

La présente invention concerne de nouveaux composés de pyrrolopyridine, leur procédé de fabrication ainsi que leur utilisation en thérapeutique pour la prévention ou le traitement de pathologies impliquant les récepteurs nucléaires de type PPAR.

### Art antérieur

En thérapeutique, il est connu que les maladies du système cardiovasculaire sont un facteur de risque important pour la santé. Ces maladies sont fréquemment la conséquence d'un taux élevé de cholestérol et/ou de triglycérides et il est donc important de maintenir ces taux inférieurs à des valeurs couramment admises par le corps médical.

Dans le cas du cholestérol, il est en particulier nécessaire d'évaluer les quantités de cholestérol liées aux différentes lipoprotéines afin d'adapter les traitements pour abaisser les taux de cholestérol lié aux LDL tout en maintenant le cholestérol lié aux HDL. Parmi les familles de composés utilisés pour réguler ces paramètres, on connaît les statines qui sont des inhibiteurs de l'HMG CoA réductase et qui permettent essentiellement de traiter des taux trop élevés en LDL-cholestérol, et les composés de la famille des fibrates, qui agissent en activant les récepteurs nucléaires PPARα (peroxisome proliferator activated receptor alpha) et qui permettent de baisser les taux de triglycérides et de cholestérol.

L'étude des récepteurs nucléaires PPAR a conduit à l'identification de 3 sous-types appelés PPARα, PPARγ et PPARδ. Ces différents récepteurs, en se liant à certains fragments précis de l'ADN, régulent l'expression de gènes cibles qui codent pour des protéines intervenant dans les mécanismes de régulation du métabolisme lipidique (voir par exemple Current Topics in Medicinal Chemistry, 2003, 3, (14), 1649-1661).

Ainsi :
- le PPARα est exprimé essentiellement dans le foie et est impliqué dans le catabolisme des acides gras en régulant la β- et la ω-oxydation (J. Lipid. Res. (1996)37, 907-925);
- les PPARγ sont exprimés principalement dans les tissus adipeux et sont impliqués dans les mécanismes de régulation de la glycémie;
- le PPARδ est exprimé de façon ubiquitaire, mais est présent principalement au niveau des reins, des muscles squelettiques, du coeur et de l'intestin .Comme les autres récepteurs de type PPAR, le PPARδ forme un hétérodimère avec le RXR (retinoïd X receptor) et est alors capable de se lier à certains éléments des gènes cibles du noyau et de contrôler les facteurs de transcription. Parmi les différentes études consacrées à ce récepteur nucléaire, il a été par exemple démontré que l'activation du PPARδ permet d'augmenter le taux de HDL-cholesterol chez la souris db/db (FEBS letters (2000), 473, 333-336) et le singe rhésus obèse insulino-dépendant, et favorise l'efflux de cholestérol via l'Apo A1 dans les cellules THP-1-humaines, (Proc. Nat. Ac. Sci. USA (2001), 98, 5306-5311).

Le traitement du diabète non insulino-dépendant de type 2 reste insatisfaisant en dépit de la mise sur le marché de nombreux dérivés hypoglycémiants oraux destinés à faciliter la sécrétion d'insuline et à favoriser son action au niveau des tissus cibles périphériques. Les agonistes PPARγ sont généralement décrits pour améliorer la sensibilité à l'insuline, comme ceci a déjà été observé avec les Thiazolidinediones (TZD).

De nouveaux agonistes de PPARs sont développés dans les traitements du diabète de type 2 et/ou des dyslipidémies. Parmi les nouveaux agonistes PPARs plusieurs sont activateurs d'au moins deux des trois sous types PPAR α, δ, γ.

L'augmentation de la fréquence de ces pathologies appelle au développement de nouveaux agents thérapeutiques actifs dans ces maladies.: des composés présentant une excellente activité hypoglycémiante et hypolipémiante en évitant les effets secondaires observés avec les thiazolidinediones sont par conséquent très utiles dans le traitement et/ou la prophylaxie de ces pathologies et particulièrement indiqués dans le traitement du diabète non insulino-dépendant de type 2 pour réduire l'insulino-résistance périphérique et normaliser la glycémie.

Suite à l'étude de ces différents récepteurs nucléaires, il apparaît que des composés qui seraient agonistes à la fois due 2, et de préférence 3, sous-types de récepteurs PPAR, pourraient présenter un profil pharmacologique extrêmement intéressant pour traiter simultanément des pathologies telles que les hyperlipidémies, les hypercholestérolémies, le diabète, ainsi que les différentes maladies du système cardiovasculaire qui sont la conséquence d'un syndrome métabolique.

La présente invention concerne des composés activateurs ou modulateurs des récepteurs PPARs. Ces composés répondent aux critères pharmacologiques cités ci-dessus.

Parmi les documents de l'art antérieur mentionnant des composés proches, on connaît par exemple les documents WO 97/28149, WO 04/060871, WO 05/016335, WO 05/016881 qui décrivent des agonistes des récepteurs PPAR δ, le document WO 01/60807 qui décrit des agonistes des récepteurs PPAR α ou encore les documents WO 05/009958 et WO 05/056522 qui proposent des composés de l'indole actifs sur les récepteurs PPAR.

On citera encore les documents WO 02/071827 et Bioorganic and Med. Chem. Letter Vol.14 (11) p.259-2763 (06/2004), qui décrivent des dérivés modulateurs des récepteurs RXR et leur utilisation en thérapeutique pour traiter les pathologies impliquées dans le syndrome métabolique.

Par ailleurs, divers composés de pyrrolopyridine ont été décrits dans l'art antérieur comme par exemple certains intermédiaires divulgués dans le document WO 98/25611 dont les composés revendiqués sont actifs contre les thromboses.

### Objet de l'invention

La présente invention concerne de nouveaux composés dérivés de la pyrrolopyridine qui sont des activateurs des PPAR, et sont choisis parmi
i) les composés de formule : 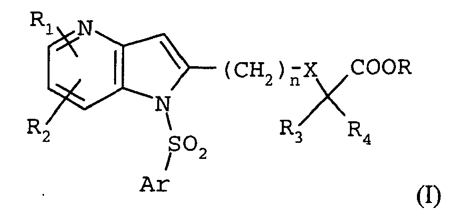 dans laquelle :
   R₁ et R₂ représentent chacun indépendamment un atome d'hydrogène, un atome d'halogène, un groupe alkyle en C₁-C₄, alcoxy en C₁-C₄ ou un groupe CF₃,
   R₃ et R₄ représentent chacun indépendamment un atome d'hydrogène ou un groupe alkyle en C₁-C₄,
   R représente un atome d'hydrogène ou un groupe alkyle en C₁-C₃,
   n= 1, 2 ou 3
   X représente une liaison simple ou un atome d'oxygène,
   Ar représente un noyau aromatique ou hétéroaromatique choisi parmi les groupes phényle, pyrazolyle, imidazolyle, thiazolyle, oxazolyle, isoxazolyle, furanyle, thiényle, pyrrolyle, pyridinyle, biphényle, naphtyle, 1,2,3,4-tétrahydronaphtalènyle, isoquinolinyle, quinolinyle, 1,2,3,4-tétrahydroquinolinyle, benzimidazolyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle, ou benzoxazolyle, éventuellement substitué par un ou plusieurs (par exemple 2 ou 3) substituants choisis parmi les atomes d'halogène, les groupes alkyle en C₁-C₆, alcoxy en C₁-C₄, trifluorométhyle, trifluorométhoxy, nitro, acétyle, acétylamino, dialkylamino dans lequel chaque groupe alkyle comprend 1 à 3 atomes de carbone ou amino, ou les hétérocycles oxazolyle, thiazolyle, pyrazolyle, pyrrolidinyle, pyridinyle, pyrimidinyle, méthyl-pyrimidinyle ou morpholinyle,
ii) leurs sels pharmaceutiquement acceptables.

Selon un deuxième aspect, l'invention concerne les composés précités pour leur utilisation en tant que substances pharmacologiquement actives, ainsi que les compositions pharmaceutiques les contenant.

En outre, l'invention concerne l'utilisation d'au moins un composé de formule (I) ou l'un de ses sels pharmaceutiquement acceptable en tant que principe actif pour la préparation d'un médicament destiné à une utilisation en thérapeutique, notamment pour lutter contre les hypercholestérolémies, les hyperlipidémies, les hypertriglycéridémies, les dyslipidémies, l'insulinorésistance, le diabète ou l'obésité ainsi que les maladies cardiovasculaires qui sont la conséquence d'un déséquilibre des lipoprotéines sériques. Les composés selon l'invention sont également utiles comme principes actifs de médicaments destinés à prévenir ou traiter les maladies liées à un dysfonctionnement endothélial, l'athérosclérose, l'infarctus du myocarde, l'hypertension, les problèmes cérébrovasculaires, certaines maladies inflammatoires comme par exemple l'arthrite rhumatoïde, et les neuro-dégénérescences comme notamment la maladie d'Alzheimer ou la maladie de Parkinson.

### Description détaillée

Dans la présente description, on entend par groupe alkyle en C₁-Cₙ (n étant un nombre entier) une chaîne hydrocarbonée ayant de 1 à n atomes de carbone linéaire, ramifiée ou partiellement ou totalement cyclique, la partie cyclique ayant au moins 3 atomes de carbone. Par exemple et sans limitation, un groupe alkyle en C₁-C₆ peut être un groupe méthyle, éthyle, propyle, butyle, pentyle, hexyle, 1-méthyléthyle, 1-méthylpropyle, 2-méthylpropyle, 1,1-diméthyléthyle, 1-méthylbutyle, 1,1-diméthylpropyle, 1-méthylpentyle, 1,1-diméthylbutyle, cyclopropyle, cyclobutyle, cyclopentyle, cyclohexyle ou cyclopentylméthyle. Par groupe alcoxy en C₁-Cₙ (n étant un nombre entier), on entend un groupe RO-, dans lequel R représente un groupe alkyle ayant 1 à n atomes de carbone tel que défini précédemment. Par halogène, on entend un atome de fluor, chlore, brome ou iode, les atomes de fluor et de chlore étant préférés.

Les composés de formule (I) dans laquelle R représente un atome d'hydrogène sont des acides carboxyliques qui peuvent être utilisés sous la forme d'acides libres ou sous la forme de sels, lesdits sels étant obtenus par combinaison de l'acide avec une base minérale ou organique non toxique pharmaceutiquement acceptable. Parmi les bases minérales, on peut utiliser par exemple les hydroxydes de sodium, de potassium, de magnésium ou de calcium. Parmi les bases organiques, on peut utiliser par exemple les amines, les aminoalcools, des acides aminés basiques tels que la lysine ou l'arginine ou encore des composés porteurs d'une fonction ammonium quaternaire tels que par exemple la bétaïne ou la choline.

Les composés de formule (I) dans laquelle les substituants R₃ et R₄ sont différents présentent un centre d'asymétrie. Pour ces composés, l'invention couvre aussi bien le composé racémique que chacun des isomères optiques considérés séparément.

Une famille particulière de composés selon l'invention comprend les composés de formule I précitée dans laquelle :
R₁ et R₂ représentent chacun indépendamment un atome d'hydrogène, un atome d'halogène ou un groupe CF₃,
R₃ et R₄ représentent chacun indépendamment un atome d'hydrogène ou un groupe alkyle en C₁-C₄,
R représente un atome d'hydrogène ou un groupe alkyle en C₁-C₃,
n= 1 ou 2,
X représente une liaison simple ou un atome d'oxygène,
Ar représente un noyau aromatique ou hétéroaromatique choisi parmi les groupes phényle, naphtyle, benzothiazolyle, 3,4-dihydro-1,4-benzoxazinyle, 1,3-benzodioxolyle, 2,3-dihydrobenzodioxinyle ou benzoxazolyle, éventuellement substitué par un ou plusieurs substituants choisis parmi les groupes alkyle en C₁-C₆, alcoxy en C₁-C₄ ou amino.

Parmi les composés de formule I selon l'invention, on préfère ceux dans laquelle :
R₁ et R₂ représentent chacun indépendamment un atome d'hydrogène, un atome d'halogène, un groupe alkyle en C₁-C₄, alcoxy en C₁-C₄ ou un groupe CF₃,
R₃ et R₄ représentent chacun indépendamment un atome d'hydrogène ou un groupe alkyle en C₁-C₄,
R représente un atome d'hydrogène ou un groupe alkyle en C₁-C₃,
n= 1, 2 ou 3
X représente une liaison simple ou un atome d'oxygène,
Ar représente un noyau aromatique ou hétéroaromatique choisi parmi les groupes phényle, pyridinyle, biphényle, naphtyle, quinolinyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle, ou benzoxazolyle, éventuellement substitué par un ou plusieurs (par exemple 2 ou 3) substituants choisis parmi les atomes d'halogène, les groupes alkyle en C₁-C₆, alcoxy en C₁-C₄, trifluorométhyle, trifluorométhoxy, nitro, acétyle, acétylamino, dialkylamino dans lequel chaque groupe alkyle comprend 1 à 3 atomes de carbone ou amino, ou les hétérocycles oxazolyle, thiazolyle, pyrazolyle, pyrrolidinyle, pyridinyle, pyrimidinyle, méthyl-pyrimidinyle ou morpholinyle.

Parmi les composés selon l'invention, on préfère encore ceux dans lesquels Ar représente un groupe phényle. On préfère également les composés dans lesquels R₁ représente un atome de chlore ou un groupe trifluorométhyle.

Les composés selon l'invention peuvent être préparés selon un premier procédé consistant à :
a) effectuer une réaction d'halogénation, préférentiellement une iodation, d'une aminopyridine de formule 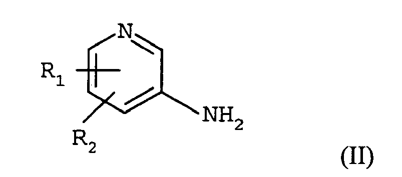 dans laquelle :
   R₁ et R₂ représentent chacun indépendamment un atome d'hydrogène, un atome de chlore de brome ou de fluor, un groupe alkyle en C₁-C₄, alcoxy en C₁-C₄ ou un groupe trifluorométhyle,
   à l'aide d'un agent halogénant, tel que par exemple l'iode en présence de sulfate d'argent ou le dichloroiodate de benzyltriméthylammonium, dans un solvant, tel que le dichlorométhane ou un alcool aliphatique, à température ambiante, pendant 5 à 24 heures pour obtenir le composé de formule 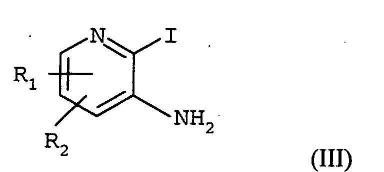
   dans laquelle :
   R₁ et R₂ conservent la même signification que dans les composés de départ ;
b) faire réagir selon la réaction dite de SONOGASHIRA (voir par exemple : Tet. Lett., 1975, 4467), le composé de formule III avec un dérivé acétylénique de formule 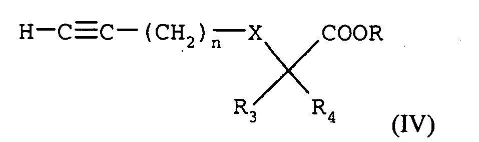 dans laquelle :
   n = 1, 2 ou 3 ;
   R₃ et R₄ représentent chacun indépendamment un atome d'hydrogène ou un groupe alkyle en C₁-C₄ ;
   R représente un groupe alkyle en C₁-C₃ ;
   X représente une liaison simple ou un atome d'oxygène ;
   en présence d'iodure cuivreux, d'un catalyseur à base de palladium, tel que par exemple le tetrakis(triphénylphosphine)palladium ou le dichloro-bis(triphénylphosphine)palladium, et d'une base organique comme par exemple la triéthylamine, dans un solvant comme par exemple le diméthylformamide (DMF) à une température comprise entre 0 et 60 °C pendant 2 à 24 heures, pour obtenir le composé de formule 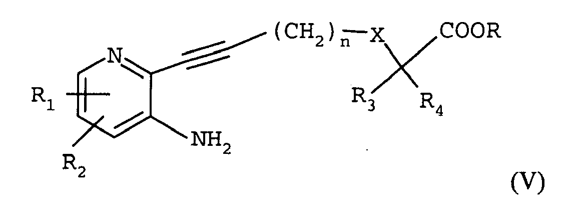
   dans laquelle:
   R₁, R₂, n, X, R₃, R₄ et R conservent la même signification que dans les composés de départ ;
c) faire réagir le composé de formule V avec un chlorure d'arylsulfonyle de formule 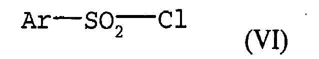 dans laquelle :
   Ar représente un noyau aromatique ou hétéroaromatique choisi parmi les groupes phényle, pyrazolyle, imidazolyle, thiazolyle, oxazolyle, isoxazolyle, furanyle, thiényle, pyrrolyle, pyridinyle, biphényle, naphtyle, 1,2,3,4-tétrahydronaphtalènyle, quinolinyle, isoquinolinyle, 1,2,3,4-tetrahydroquinolinyle, benzimidazolyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle, ou benzoxazolyle, éventuellement substitué par un ou plusieurs (par exemple 2 ou 3) substituants choisis parmi les atomes d'halogène, les groupes alkyle en C₁-C₆, alcoxy en C₁-C₄, trifluorométhyle, trifluorométhoxy, nitro, acétyle, acétylamino, dialkylamino ou amino, ou les hétérocycles oxazolyle, thiazolyle, pyrazolyle, pyrrolidinyle, pyridinyle, pyrimidinyle, méthyl-pyrimidinyle ou morpholinyle,
   en présence de pyridine, éventuellement dans un solvant, tel que le dichlorométhane, à température ambiante, pendant 10 à 120 mn, pour obtenir le composé de formule 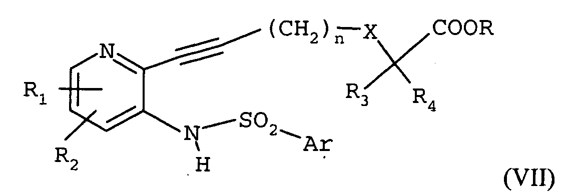
   dans laquelle :
   R₁, R₂, n, X, R₃, R₄, R et Ar conservent la même signification que dans les composés de départ ;
d) effectuer une cyclisation du composé de formule VII, par exemple par action de l'acétate de cuivre II (voir par exemple J. Org. Chem., 2004, 69 (4), 1126-1136), dans un solvant, tel que le 1,2-dichloroéthane, à une température proche de la température de reflux du solvant, pendant 4 à 24 heures, pour obtenir le composé de formule 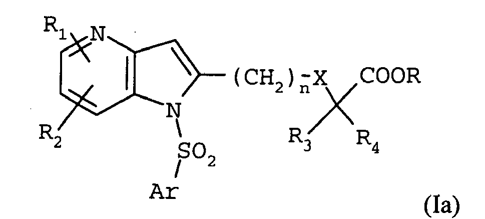 dans laquelle :
   R₁, R₂, n, X, R₃, R₄, R et Ar conservent la même signification que dans les composés de départ ;
e) si nécessaire, hydrolyser la fonction ester du composé de formule Ia, par exemple par action d'une base minérale, telle que la soude ou la lithine, selon des modes opératoires bien connus de l'homme du métier, pour obtenir, après traitement acide, le composé de formule I sous sa forme d'acide libre:

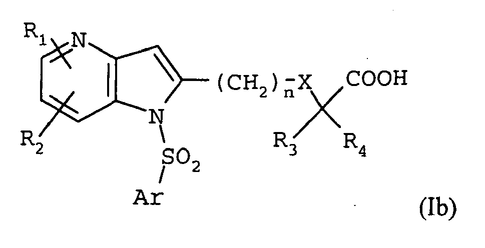

Selon une première variante du procédé de préparation, les composés de formule I peuvent être obtenus par une série de réactions consistant à :
a) faire réagir le composé de formule (III) 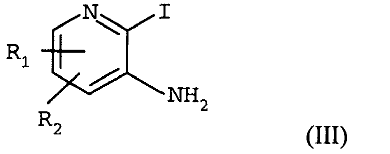 tel qu'obtenu ci-dessus, avec un chlorure d'arylsulfonyle de formule 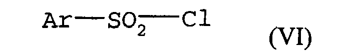 dans laquelle :
   Ar représente un noyau aromatique ou hétéroaromatique choisi parmi les groupes phényle, pyrazolyle, imidazolyle, thiazolyle, oxazolyle, isoxazolyle, furanyle, thiényle, pyrrolyle, pyridinyle, biphényle, naphtyle, 1,2,3,4-tétrahydronaphtalènyle, quinolinyle, isoquinolinyle, 1,2,3,4-tetrahydroquinolinyle, benzimidazolyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle, ou benzoxazolyle, éventuellement substitué par un ou plusieurs (par exemple 2 ou 3) substituants choisis parmi les atomes d'halogène, les groupes alkyle en C₁-C₆, alcoxy en C₁-C₄, trifluorométhyle, trifluorométhoxy, nitro, acétyle, acétylamino, dialkylamino ou amino, ou les hétérocycles oxazolyle, thiazolyle, pyrazolyle, pyrrolidinyle, pyridinyle, pyrimidinyle, méthyl-pyrimidinyle ou morpholinyle,
   dans un solvant, tel que par exemple le diméthylformamide, de préférence en présence d'une base aprotique, telle que par exemple la pyridine, à température ambiante et pendant 1 à 12 heures, pour obtenir le composé de formule (VIII) 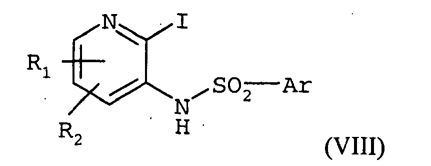
   dans laquelle :
   R₁, R₂ et Ar conservent la même signification que dans les composés de départ ;
b) faire réagir le composé de formule VIII avec un dérivé acétylénique de formule 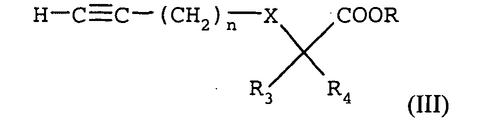 dans laquelle :
   n= 1, 2 ou 3 ;
   R₃ et R₄ représentent chacun indépendamment un atome d'hydrogène ou un groupe alkyle en C₁-C₄ ;
   R représente un groupe alkyle en C₁-C₃ ;
   X représente une liaison simple ou un atome d'oxygène;
      dans des conditions analogues à celles décrites pour l'étape b) du procédé général précédent,
      pour obtenir le composé de formule

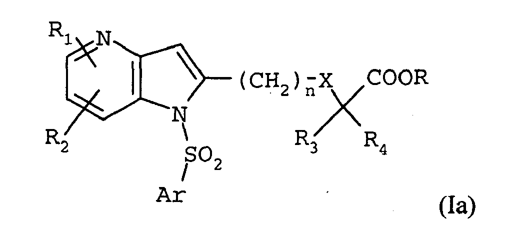

      dans laquelle R₁, R₂, n, X, R₃, R₄, R et Ar conservent la même signification que dans les composés de départ ;
c) si nécessaire, hydrolyser la fonction ester du composé de formule Ia, par exemple par action d'une base minérale, telle que la soude ou la lithine, selon des modes opératoires bien connus de l'homme du métier, pour obtenir, après traitement acide, le composé de formule I sous sa forme d'acide libre :

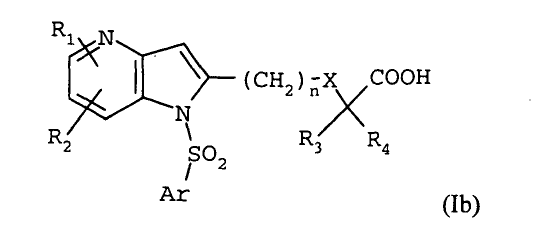

Les composés de l'invention sous forme de sels d'un acide de formule Ib avec une base minérale ou organique, peuvent être obtenus de façon classique, en utilisant les méthodes bien connues de l'homme de métier, par exemple en mélangeant des quantités stoechiométriques de l'acide et de la base dans un solvant, tel que par exemple l'eau ou un mélange hydroalcoolique, et en lyophilisant ensuite la solution obtenue.

Dans certaines des étapes réactionnelles décrites ci-dessus, il est possible de remplacer avantageusement les méthodes de chauffage traditionnelles par un chauffage au moyen de micro-ondes en utilisant des réacteurs adaptés à ce mode de réaction. Dans ce cas, l'homme du métier comprendra que les durées de "chauffage" seront considérablement réduites, par comparaison aux durées nécessaires avec un chauffage classique.
Les exemples suivants de préparation de composés selon la formule (I) permettront de mieux comprendre l'invention.
Dans ces exemples, qui ne sont pas limitatifs de la portée de l'invention, on désigne par « préparation » les exemples décrivant la synthèse de composés intermédiaires et par « exemples » ceux décrivant la synthèse de composés de formule (I) selon l'invention. Parmi les abréviations, « mM » signifie millimole, THF signifie tétrahydrofurane, DMF représente le diméthylformamide, DME signifie le 1,2-diméthoxyéthane, DCM représente le dichlorométhane et PdCl₂dppf signifie le dichloro-1,1'-bis(diphénylphosphino)ferrocène-palladium(II).

Les points de fusion sont mesurés au banc Kofler ou à l'aide d'un appareil Mettler et les valeurs spectrales de Résonance Magnétique Nucléaire sont caractérisées par le déplacement chimique calculé par rapport au TMS, par le nombre de protons associés au signal et par la forme du signal (s pour singulet, d pour doublet, dd pour doublet dédoublé, t pour triplet, q pour quadruplet, quin pour quintuplet, m pour multiplet). La fréquence de travail et le solvant utilisés sont indiqués pour chaque composé. La température ambiante est de 20°C ± 5°C.

### PREPARATION I

### 3-amino-6-chloro-2-iodopyridine

On mélange 23,2 g (180,5 mM) de 5-amino-2-chloropyridine dans 70 ml de dichlorométhane (DCM) et 180 ml de méthanol et on ajoute 21,6 g (216 mM) de carbonate de calcium et 75,3 g (226 mM) de dichloroiodure de benzyltriméthylammonium. Le mélange réactionnel est agité à température ambiante pendant 16 heures, puis filtré pour éliminer les sels minéraux. Le filtrat est dilué à l'eau et extrait par du DCM. La phase organique obtenue est lavée par une solution de chlorure de sodium, puis par une solution saturée de thiosulfate de sodium, séchée sur sulfate de magnésium et concentrée sous pression réduite. On obtient une huile que l'on purifie par chromatographie sur gel de silice en éluant à l'aide d'un mélange cyclohexane/acétate d'éthyle (80/20 puis 70/30;v/v). On obtient ainsi 13,9 g du produit attendu sous forme d'un solide orange (rendement = 30%).
F = 148°C.

### PREPARATION II

### 3-[di(benzènesulfonyl)amino]-6-chloro-2-iodopyridine

On agite pendant 60 heures à température ambiante un mélange de 13,75 g (54 mM) du composé obtenu selon la préparation I et 27,6 ml (216 mM) de chlorure de benzènesulfonyle dans 30 ml de pyridine. Le mélange réactionnel est ensuite dilué à l'eau et extrait plusieurs fois par de l'acétate d'éthyle. La phase organique est lavée par une solution d'acide chlorhydrique N, puis par une solution de chlorure de sodium, séchée sur sulfate de magnésium et concentrée sous pression réduite. On obtient le composé attendu sous forme d'un solide beige (rendement = 96%).
F=231°C.

### PREPARATION III

### N-(6-chloro-2-iodo-3-pyridinyl)benzènesulfonamide

On mélange 15,46 g (29 mM) du composé obtenu selon la préparation II dans 170 ml de dioxane et on ajoute 77 ml d'une solution aqueuse de potasse 3 M. Le mélange réactionnel est agité à doux reflux du solvant pendant 1 heure, puis concentré sous pression réduite. On obtient un solide beige que l'on reprend en suspension dans 200 ml d'eau. Le mélange est acidifié jusqu'à pH 4 environ par addition d'acide chlorhydrique puis extrait par du dichlorométhane (DCM). La phase organique est séchée sur sulfate de magnésium puis concentrée sous pression réduite. On obtient ainsi 10,32 g du produit attendu sous forme d'un solide beige (rendement = 91 %).
F = 132°C.

### Exemple 1

### Acide 5-chloro-1-(phénylsulfonyl)-1H-pyrrolo[3,2-b]pyridine-2-propanoïque, méthyl ester

On mélange 10,32 g (26,2 mM) du composé obtenu selon la préparation III, 30 ml de diméthylformamide, 460 mg (0,65 mM) de dichlorobis(triphénylphosphine)-palladium, 250 mg (1,3 mM) d'iodure cuivreux et 20 ml de diéthylamine. On ajoute ensuite sous agitation, à température ambiante, 3,5 g (31,25 mM) d'ester méthylique de l'acide 4-pentynoïque et le mélange réactionnel est agité pendant 1 heure à léger reflux du solvant. Le mélange réactionnel est ensuite dilué à l'eau et extrait plusieurs fois par de l'acétate d'éthyle. La phase organique est lavée par une solution de chlorure de sodium, séchée sur sulfate de magnésium et concentrée sous pression réduite. L'huile brune obtenue est purifiée par chromatographie sur gel de silice en éluant à l'aide d'un mélange cyclohexane/acétate d'éthyle (8/2 ; v/v). On obtient ainsi 8,02 g du produit attendu sous forme d'un solide jaune (rendement : 81 %).
F = 108-115°C.

### Exemple 2

### Acide 5-chloro-1-(phénylsulfonyl)-1H-pyrrolo[3,2-b]pyridine-2-propanoïque

On mélange 1,5 g (4 mM) de l'ester obtenu selon l'exemple 1 dans 5 ml de tétrahydrofurane et on ajoute une solution de 332 mg (7,9 mM) de lithine (LiOH, H₂O) dans 4 ml d'eau. Le milieu réactionnel est agité pendant 2 heures à température ambiante puis acidifié jusqu'à pH 3 par de l'acide chlorhydrique et extrait par du DCM. La phase organique est séchée sur sulfate de magnésium et concentrée sous pression réduite. On obtient une huile légèrement jaune qui cristallise sous forme d'un solide blanc (rendement = 77 %).
F = 185-187°C.

### PREPARATION IV

### 3-amino-2-iodo-6-(trifluorométhyl)pyridine

On ajoute sous agitation et à température ambiante, 3,85 g (12,3 mM) de sulfate d'argent à une solution de 2 g (12,3 mM) de 5-amino-2-(trifluorométhyl)pyridine dans 100 ml d'éthanol. On ajoute ensuite 3,13 g d'iode et on maintient le mélange réactionnel sous agitation à température ambiante pendant 24 heures. Le solide en suspension dans le milieu est éliminé par filtration et le filtrat est concentré sous pression réduite. Le résidu d'évaporation est repris par 200 ml de dichlorométhane et lavé par une solution de soude à 5%, puis par de l'eau et séché sur sulfate de magnésium. La solution obtenue est concentrée sous pression réduite et on obtient ainsi 3,42 g du composé attendu sous forme d'un solide rose (rendement = 96 %).
F = 127°C.

### PREPARATION V

### Acide 6-(3-amino-6-chloro-2-pyridinyl)-5-hexynoïque, méthyl ester

On mélange 5 g (19,6 mM) du composé obtenu selon la préparation I, 20 ml de diméthylformamide, 345 mg (0,49 mM) dé dichlorobis(triphénylphosphine)-palladium, 187 mg (0,98 mM) d'iodure cuivreux et 10 ml de diéthylamine. On ajoute ensuite sous agitation, à température ambiante, 2,97 g (23,5 mM) d'ester méthylique de l'acide 5-hexynoïque et le mélange réactionnel est agité pendant 1 heure à léger reflux du solvant. Le mélange réactionnel est ensuite dilué à l'eau et extrait plusieurs fois par de l'acétate d'éthyle. La phase organique est lavée par une solution de chlorure de sodium, séchée sur sulfate de magnésium et concentrée sous pression réduite. Le composé huileux résiduel obtenu est purifié par chromatographie sur gel de silice en éluant à l'aide d'un mélange cyclohexane/acétate d'éthyle (9/1 ; v/v). On obtient ainsi 4,15 g du produit attendu sous forme d'une huile (rendement : 84 %).
¹H RMN (300 MHz, DMSO) δ : 1,83 (quin, 2H) ; 2,47 (t, 2H) ; 2,54 (t, 2H) ; 3,60 (s, 3H) ; 5,63 (s, 2H) ; 7,10 (s, 2H).

### PREPARATION VI

### Acide 6-[3-[(6-benzothiazolylsulfonyl)amino]-6-chloro-2-pyridinyl]-5-hexynoïque, méthyl ester

On prépare une solution de 1 g (4 mM) du composé obtenu selon la préparation V dans 10 ml de pyridine et on ajoute 1,1 g (4,7 mM) de chlorure de 6-benzothiazolesulfonyle. Le mélange est agité pendant 3 heures à température ambiante puis dilué avec de l'eau et extrait par l'acétate d'éthyle. La phase organique est lavée deux fois par une solution d'acide chlorhydrique N, puis à l'eau, séchée sur sulfate de magnésium et concentrée sous pression réduite. L'huile résiduelle est purifiée par chromatographie sur gel de silice en éluant à l'aide d'un mélange cyclohexane/acétate d'éthyle (7/3 ; v/v). On obtient ainsi 1,07 g du composé attendu sous forme d'un solide jaune (rendement = 60 %).
F = 134°C.

### Exemple 3

### Acide 1-(6-benzothiazolylsulfonyl)-5-chloro-1H-pyrrolo[3,2-b]pyridine-2-butanoïque, méthyl ester

Dans un tube-réacteur pour micro-ondes, on prépare un mélange de 1 g (2,22 mM) d'ester obtenu selon la préparation VI dans 3 ml de 1,2-dichloroéthane et on ajoute 403 mg (2,22 mM) d'acétate de cuivre (cuivrique). Le mélange est chauffé sous micro-ondes à 150°C pendant 30 minutes, puis refroidi, dilué avec 6 ml de dichlorométhane et filtré sur papier Whatman. Le filtrat est concentré sous pression réduite et le produit brut est purifié par chromatographie sur gel de silice en éluant avec un mélange cyclohexane/acétate d'éthyle (8/2 ; v/v). On obtient 500 mg du composé attendu sous forme d'un solide jaune (rendement = 50 %).
F = 55°C.

### Exemple 4

### Acide 1-(6-benzothiazolylsulfonyl)-5-chloro-1H-pyrrolo[3,2-b]pyridine-2-butanoïque

En opérant de façon analogue à l'exemple 2, au départ du composé obtenu selon l'exemple 3, on obtient le produit attendu sous forme d'un solide beige (rendement = 95 %).
F = 178°C.

### PREPARATION VII

### N-[2-iodo-6-(trifluorométhyl)-3-pyridinyl]benzènesulfonamide

On ajoute progressivement, sous agitation et à température ambiante, 7 g (40 mM) de chlorure de benzènesulfonyle à une solution de 2,88 g (10 mM) du composé obtenu selon la préparation IV dans 25 ml de pyridine. Le mélange réactionnel est agité à température ambiante pendant 24 heures, puis versé sur 300 ml d'acide chlorhydrique N glacé. Le précipité obtenu est séparé par filtration et lavé à l'eau sur le filtre, puis agité dans un ballon avec 40 ml de dioxane et 10 ml d'une solution aqueuse 3 M d'hydroxyde de potassium, à doux reflux du solvant, pendant 2 heures. Ce mélange réactionnel est refroidi, dilué avec 300 ml d'eau, acidifié jusqu'à pH 1,5 environ à l'aide d'acide chlorhydrique concentré, puis extrait par du dichlorométhane. La phase organique obtenue est lavée à l'eau puis séchée sur sulfate de magnésium et concentrée sous pression réduite. On obtient ainsi 3,7 g du produit attendu sous forme d'un solide blanc (rendement = 89 %).
F = 131°C.

### PREPARATION VIII

### Acide 5-[3-amino-6-chloro-2-pyridinyl]-4-pentynoïque, méthyl ester

En opérant de façon analogue à la préparation V, au départ de l'ester méthylique de l'acide 4-pentynoïque, on obtient le produit attendu sous forme d'un solide jaune (rendement = 77 %).
F = 96-100°C.

En opérant de façon analogue à la préparation VI, au départ des chlorures de sulfonyle adéquats, on obtient les composés suivants :

### PREPARATION IX

### Acide 6-[3-[(1,3-benzodioxol-5-ylsulfonyl)amino]-6-chloro-2-pyridinyl]-5-hexynoïque, méthyl ester

Solide marron, rendement = 91 %.
F = 123°C.

### PREPARATION X

### Acide 6-[3-[(2-amino-5-benzothiazolylsulfonyl)amino]-6-chloro-2-pyridinyl]-5-hexynoïque, méthyl ester

Solide blanc, rendement = 37 %.
F = 66-72°C.

### PREPARATION XI

### Acide 6-[6-chloro-3-[(3,5-diméthylphényl)sulfonylamino]-2-pyridinyl]-5-hexynoïque, méthyl ester

Huile orange, rendement = 98 %.
¹H RMN (300 MHz, DMSO) δ : 1,74 (quin, 2H) ; 2,30 (s, 6H) ; 2,40 (t, 2H) ; 2,42 (t, 2H) ; 3,61 (s, 3H) ; 7,28 (s, 1H) ; 7,33 (s, 2H) ; 7,45 (d, 1H) ; 7,70 (d, 2H) ; 10,07 (s, 1H).

### PREPARATION XII

### Acide 6-[6-chloro-3-[(2,5-diméthoxyphényl)sulfonylamino]-2-pyridinyl]-5-hexynoïque, méthyl ester

Solide orange, rendement = 84 %.
F = 78-82°C.

### PREPARATION XIII

### Acide 6-[6-chloro-3-[(1-naphtalènyl)sulfonylamino]-2-pyridinyl]-5-hexynoïque, méthyl ester

Solide orange, rendement = 98 %.
F = 117°C.

### PREPARATION XIV

### Acide 6-[6-chloro-3-[(3,4-dihydro-4-méthyl-2H-1,4-benzoxazin-7-yl)sulfonylamino]-2-pyridinyl]-5-hexynoïque, méthyl ester

Huile brune, rendement = 74 %.
¹H RMN (300 MHz, DMSOd₆) δ : 1,75 (quin, 2H) ; 2,41 (m, 4H) ; 2,78 (s, 3H) ; 3,28 (m, 2H) ; 3,61 (s, 3H) ; 4,27 (m, 2H) ; 6,78 (d, 1H) ; 6,88 (s, 1H) ; 6,89 (d, 1 H) ; 7,44 (d, 1H) ; 7,71 (d, 1H) ; 9,80 (s, 1H).

En opérant de façon analogue à la préparation VI, au départ de l'ester obtenu selon la préparation VIII et des chlorures de sulfonyle adéquats, on obtient les composés suivants :

### PREPARATION XV

### Acide 5-[3-[(5-benzodioxolylsulfonyl)amino]-6-chloro-2-pyridinyl]-4-pentynoïque, méthyl ester

Solide marron, rendement = 92 %.
F = 133°C.

### PREPARATION XVI

### Acide 5-[3-[(6-benzothiazolylsulfonyl)amino]-6-chloro-2-pyridinyl]-4-pentynoïque, méthyl ester

Solide jaune, rendement = 49 %.
F = 137°C.

### PREPARATION XVII

### Acide 2-[[3-(3-amino-6-chloro-2-pyridinyl)-2-propynyl]oxy]propanoïque, éthyl ester

En opérant de façon analogue à la préparation V, au départ de l'ester éthylique de l'acide 2-(2-propynyloxy)propanoïque, on obtient le produit attendu sous forme d'un solide brun (rendement = 73 %).
F = 70°C.

### PREPARATION XVIII

### N-(6-chloro-2-iodo-3-pyridinyl)-3-méthoxy-benzènesulfonamide

En opérant de façon analogue à la préparation VII, au départ du composé obtenu selon la préparation I et de chlorure de 3-méthoxybenzènesulfonyle, on obtient le produit attendu sous forme d'un solide blanc (rendement = 96 %).
F = 154°C.

### PREPARATION XIX

### Acide 2-[[3-[3-amino-6-(trifluorométhyl)-2-pyridinyl]-2-propynyl]oxy]-2-méthylpropanoïque, méthyl ester

En opérant de façon analogue à la préparation V, au départ du composé obtenu selon la préparation IV et d'ester méthylique de l'acide 2-méthyl-2-(2-propynyloxy)propanoïque, on obtient le produit attendu sous forme d'une huile jaune (rendement = 56 %).
¹H RMN (250 MHz, DMSOd₆) δ : 7,50 (d, 1H) ; 7,18 (d, 1H) ; 6,23 (s, 2H) ; 4,44 (s, 2H) ; 3,68 (s, 3H) ; 1,42 (s, 6H).

### PREPARATION XX

### Acide 2-[[3-[3-amino-6-(trifluorométhyl)-2-pyridinyl]-2-propynyl]oxy] propanoïque, éthyl ester

On mélange 800 mg (2,78 mM) du composé obtenu selon la préparation IV, 78 mg (0,28 mM) de tricyclohexylphosphine, 20 ml de diméthylformamide, 97 mg (0,14 mM) de dichlorobis(triphénylphosphine)-palladium, 26 mg (0,14 mM) d'iodure cuivreux et 8 ml de t-butylamine. On ajoute ensuite sous agitation, à température ambiante, 1,09 g (7 mM) d'ester éthylique de l'acide 2-(2-propynyloxy)propanoïque et le mélange réactionnel est agité pendant 20 heures à 45-50°C. Le mélange réactionnel est ensuite refroidi, dilué à l'eau et extrait plusieurs fois par de l'acétate d'éthyle. Les phases organiques rassemblées sont lavées par une solution de chlorure de sodium, séchées sur sulfate de magnésium et concentrées sous pression réduite. Le produit brut obtenu est purifié par chromatographie sur gel de silice en éluant à l'aide d'un mélange toluène/acétate d'éthyle (9/1 puis 8/2 ; v/v). On obtient ainsi le produit attendu sous forme d'une huile (rendement : 76 %).
¹H RMN (300 MHz, DMSOd₆) δ : 7,51 (d, 1H) ; 7,18 (d, 1H) ; 6,27 (s, 2H) ; 4,58 (d, 1H) ; 4,48 (d, 1H) ; 4,23 (q, 1H) ; 4,12 (q, 2H) ; 1,31 (d, 3H) ; 1,20 (t, 3H).

### PREPARATION XXI

### Acide 5-[3-amino-6-(trifluorométhyl)-2-pyridinyl]-4-pentynoïque, méthyl ester

En opérant de façon analogue à la préparation V, au départ du composé obtenu selon la préparation IV et d'ester méthylique de l'acide 4-pentynoïque, on obtient le produit attendu sous forme d'un solide ocre (rendement = 81 %).
F =90°C.

### PREPARATION XXII

### Acide 6-[3-amino-6-(trifluorométhyl)-2-pyridinyl]-5-hexynoïque, méthyl ester

En opérant de façon analogue à la préparation XXI, au départ de l'ester méthylique de l'acide 5-hexynoïque, on obtient le produit attendu sous forme d'un solide brun (rendement = 94 %).
F = 49 °C.

### PREPARATION XXIII

### Acide 6-(3-amino-6-chloro-2-pyridinyl)-6-heptynoïque, méthyl ester

En opérant de façon analogue à la préparation V, au départ de l'ester méthylique de l'acide 6-heptynoïque, on obtient le produit attendu sous forme d'une huile jaune (rendement = 85 %).
¹H RMN (300 MHz, DMSOd₆) δ : 7,09 (s, 2H) ; 5,60 (s, 2H) ; 3,60 (s, 3H) ; 2,50 (m, 2H) ; 2,37 (t, 2H) ; 1,63 (m, 4H).

En opérant de façon analogue à la préparation VII, au départ du composé obtenu selon la préparation I et des chlorures de sulfonyle adéquats, on obtient les aryles ou hétéroarylsulfonamides suivants :

### PREPARATION XXIV

### N-(6-chloro-2-iodo-3-pyridinyl)-4-éthyl-benzènesulfonamide

Solide jaune, rendement = 88 %.
F = 141 °C.

### PREPARATION XXV

### N-(6-chloro-2-iodo-3-pyridinyl)-4-méthoxy-benzènesulfonamide

Solide brun, rendement = 98 %.
F = 93 °C.

### PREPARATION XXVI

### N-(6-chloro-2-iodo-3-pyridinyl)-2,3-dichloro-benzènesulfonamide

Solide beige, rendement = 99 %.
F > 260 °C.

### PREPARATION XXVII

### N-(6-chloro-2-iodo-3-pyridinyl)-4-(1-méthyléthyl)-benzènesulfonamide

Solide jaune, rendement = 93 %.
F = 132 °C.

### PREPARATION XXVIII

### N-(6-chloro-2-iodo-3-pyridiliyl)-1-naphthalènesulfonamide

Solide blanc, rendement = 54 %.
F = 135 °C.

### PREPARATION XXIX

### N-(6-chloro-2-iodo-3-pyridinyl)-8-quinoleïnesulfonamide

Solide rose, rendement = 14 %.
F = 199 °C.

### PREPARATION XXX

### N-(6-chloro-2-iodo-3-pyridinyl)-2,5-diméthoxy-benzènesulfonamide

Solide beige, rendement = 99 %.
F = 147 °C.

### PREPARATION XXXI

### N-(6-chloro-2-iodo-3-pyridinyl)-1,3-benzodioxole-5-sulfonamide

Solide blanc, rendement = 95 %.
F = 187 °C.

### PREPARATION XXXII

### N-(6-chloro-2-iodo-3-pyridinyl)-2,3-dihydro-1,4-benzodioxine-6-sulfonamide

Solide jaune, rendement = 94 %.
F=122°C.

### PREPARATION XXXIII

### N-(6-chloro-2-iodo-3-pyridinyl)-2,3-dihydro-5-benzofuranesulfonamide

Solide blanc, rendement = 94 %.
F = 187 °C.

### PREPARATION XXXIV

### N-(6-chloro-2-iodo-3-pyridinyl)-3,5-diméthyl-benzènesulfonamide

Solide beige, rendement = 99 %.
F = 138 °C.

### PREPARATION XXXV

### N-(6-chloro-2-iodo-3-pyridinyl)-3,4-dihydro-4-méthyl-2H-1,4-benzoxazine-7-sulfonamide

Solide blanc, rendement = 99 %.
F = 139 °C.

### PREPARATION XXXVI

### N-(2-bromo-6-méthyl-3-pyridinyl)benzènesulfonamide

En opérant de façon analogue à la préparation VII, au départ de la 3-amino-2-bromo-6-méthylpyridine, on obtient le produit attendu sous forme d'un solide beige (rendement = 95 %).
¹H RMN (250 MHz, DMSOd₆) δ : 10,05 (s, 1H) ; 7,70 (m, 3H) ; 7,61 (d, 2H) ; 7,47 (d, 1H) ; 7,25 (d, 1H) ; 2,39 (s, 3H).

### PREPARATION XXXVII

### 3-amino-2-bromo-6-méthoxypyridine

On prépare un mélange de 1,83 g (14,7 mM) de 5-amino-2-méthoxypyridine et 1,21 g (14,7 mM) d'acétate de sodium dans 12 ml d'acide acétique et on ajoute doucement, sous agitation et en maintenant à température ambiante, 0,75 ml (14,7 mM) de brome. Le mélange réactionnel est maintenu sous agitation pendant 30 mn, à température ambiante, puis on ajoute 200 ml d'une solution saturée de thiosulfate de sodium. La phase aqueuse obtenue est extraite 2 fois par de l'acétate d'éthyle. Les phases organiques rassemblées sont séchées sur sulfate de magnésium et concentrées sous pression réduite. On obtient 3g du composé attendu sous forme d'un solide violet (rendement quantitatif).
1H RMN (250 MHz, DMSOd₆) δ : 7,18 (d, 1H) ; 6,64 (d, 1H) ; 4,91 (s, 2H) ; 3,71 (s, 3H).

### PREPARATION XXXVIII

### (2-bromo-6-méthoxy-3-pyridinyl)benzènesulfonamide

En opérant de façon analogue à la préparation VII, au départ de la 3-amino-2-bromo-6-méthoxypyridine, on obtient le produit attendu sous forme d'un solide brun (rendement = 50 %).
¹H RMN (250 MHz, DMSOd₆) δ : 9,92 (s, 1H) ; 7,67 (m, 3H) ; 7,57 (m, 2H) ; 7,45 (d, 1H) ; 6,84 (d, 1H) ; 3,80 (s, 3H).

### PREPARATION XXXIX

### 3-amino-2-iodo-6-méthoxypyridine

En opérant de façon analogue à la préparation IV, au départ de la 5-amino-2-méthoxypyridine, on obtient le produit attendu sous forme d'une huile marron (rendement = 6 %).
¹H RMN (300 MHz, DMSOd₆) δ : 7,08 (d, 1H) ; 6,62 (d, 1H) ; 4,81 (s, 2H) ; 3,71 (s, 3H).

### PREPARATION XL

### (2-iodo-6-méthoxy-3-pyridinyl)benzènesulfonamide

En opérant de façon analogue à la préparation VII, au départ de la 3-amino-2-iodo-6-méthoxypyridine, on obtient le produit attendu sous forme d'un solide brun (rendement = 91 %).
¹H RMN (250 MHz, DMSOd₆) δ : 9,84 (s, 1H) ; 7,65 (m, 5H) ; 7,17 (d, 1H) ; 6,77 (d, 1H) ; 3,79 (s, 3H).

### PREPARATION XLI

### N-(2-bromo-6-méthyl-3-pyridinyl)-6-benzothiazolesulfonamide

En opérant de façon analogue à la préparation VII, au départ de la 3-amino-2-bromo-6-méthylpyridine et du chlorure de 6-benzothiazolesulfonyle, on obtient le produit attendu sous forme d'une pâte beige (rendement = 35 %).
¹H RMN (250 MHz, DMSOd₆) δ 10,20 (s, 1H) ; 9,63 (s, 1H) ; 8,61 (d, 1H) ; 8,25 (d, 1H) ; 7,85 (dd, 1H) ; 7,49 (d, 1H) ; 7,26 (d, 1H) ; 2,39 (s, 3H).

### PREPARATION XLII

### N-(6-chloro-2-iodo-3-pyridinyl)-3-pyridinesulfonamide

En opérant de façon analogue à la préparation XVIII, au départ du chlorure de 3-pyridinesulfonyle, on obtient le produit attendu sous forme d'un composé pâteux utilisé sans purification particulière pour la préparation du composé selon l'exemple 252.

### PREPARATION XLIII

### N-(6-chloro-2-iodo-3-pyridinyl)-6-quinoléïnesulfonamide

En opérant de façon analogue à la préparation XVIII au départ du chlorure de 6-quinoléïnesulfonyle, on obtient le produit attendu sous forme d'un solide blanc (rendement = 99%)
F > 250°C.

### PREPARATION XLIV

### N-(2-iodo-6-chloro-3-pyridinyl)-6-benzothiazolesulfonamide

En opérant de façon analogue à l'exemple 50, au départ de la 3-amino-2-iodo-6-chloropyridine et du chlorure de 6-benzothiazolesulfonyle, on obtient le produit attendu sous forme d'un solide orange (rendement = 33 %).
F = 191 °C.

### PREPARATION XLV

### Acide 5-(trifluorométhyl)-1H-pyrrolo[3,2-b]pyridine-2-butanoïque, méthyl ester

En opérant de façon analogue à l'exemple 3, au départ de l'ester méthylique obtenu selon la préparation XXII, on obtient le produit attendu sous forme d'un solide beige (rendement = 66 %).
F = 137°C.

### PREPARATION XLVI

### Acide 6-[6-chloro-3-[[(4-fluoro-3-nitrophényl)sulfonyl]amino]-2-pyridinyl]-5-hexynoïque, méthyl ester

En opérant de façon analogue à la préparation VI, au départ du chlorure de 4-fluoro-3-nitrobenzènesulfonyle, on obtient le produit attendu sous forme d'une huile jaune (rendement = 31 %).
¹H RMN (300 MHz, DMSOd₆) δ : 10,63 (s, 1H) ; 8,41 (dd, 1H) ; 8,06 (m, 1H) ; 7,75 (m, 2H) ; 7,46 (d, 1H) ; 3,61 (s, 3H) ; 2,39 (m, 4H) ; 1,72 (quin, 2H).

### PREPARATION XLVII

### Acide 6-[3-[[(2,1,3-benzothiadiazol-4-yl)sulfonyl]amino]-6-chloro-2-pyridinyl]-5-hexynoïque, méthyl ester

En opérant de façon analogue à la préparation VI, au départ du chlorure de 2,1,3-benzothiadiazole-4-sulfonyle, on obtient le composé attendu sous forme d'un solide beige (rendement = 64 %).
¹H RMN (250 MHz, DMSOd₆) δ : 10,36 (s, 1H) ; 8,40 (d, 1H) ; 8,18 (d, 1H) ; 7,84 (m, 2H) ; 7,48 (d, 1H) ; 3,62 (s, 3H) ; 2,26 (t, 2H) ; 2,01 (t, 2H) ; 1,47 (quin, 2H).

### Exemple 5

### Acide 2-[[5-chloro-1-(phénylsulfonyl)-1H-pyrrolo[3,2-b]pyridin-2-yl]-méthoxy]propanoïque, éthyl ester

En opérant de façon analogue à l'exemple 1, au départ de l'ester éthylique de l'acide 2-(2-propynyloxy)propanoïque, on obtient le composé attendu sous forme d'une huile jaune (rendement = 62 %).
¹H RMN (300 MHz, DMSOd₆) δ : 1,18 (t, 3H) ; 1,31 (d, 3H) ; 4,12 (q, 2H) ; 4,24 (q, 1H) ; 4,92 (d, 1H) ; 5,05 (d, 1H) ; 6,95 (s, 1H) ; 7,43 (d, 1H) ; 7,60 t, 2H) ; 7,74 (t, 1H) ; 8,02 (d, 2H) ; 8,42 (d, 1H).

### Exemple 6

### Acide 2-[[5-chloro-1-(phénylsulfonyl)-1H-pyrrolo[3,2-b]pyridin-2-yl]-méthoxy]propanoïque

En opérant de façon analogue à l'exemple 2, au départ de l'ester obtenu selon l'exemple 5, on obtient le composé attendu sous forme d'un solide blanc (rendement = 57 %).
F = 153-155°C.

### Exemple 7

### Acide 2-[[5-chloro-1-(phénylsulfonyl)-1H-pyrrolo[3,2-b]pyridin-2-yl]-méthoxy]-2-méthylpropanoïque, méthyl ester

En opérant de façon analogue à l'exemple 1, au départ de l'ester méthylique de l'acide 2-méthyl-2-(2-propynyloxy)propanoïque, on obtient le composé attendu sous forme d'un solide blanc (rendement = 20 %).
F = 84-87°C.

### Exemple 8

### Acide 2-[[5-chloro-1-(phénylsulfonyl)-1H-pyrrolo[3,2-b]pyridin-2-yl]-méthoxy]-2-méthylpropanoïque

En opérant de façon analogue à l'exemple 2, au départ de l'ester obtenu selon l'exemple 7, on obtient le composé attendu sous forme d'un solide blanc (rendement = 57 %).
F = 183-185°C.

### Exemple 9

### Acide 5-chloro-1-(phénylsulfonyl)-1H-pyrrolo[3,2-b]pyridine-2-butanoïque, méthyl ester

En opérant de façon analogue à l'exemple 1, au départ de l'ester méthylique de l'acide 5-hexynoïque, on obtient le composé attendu sous forme d'un solide jaune (rendement = 38 %).
F = 85-90°C.

### Exemple 10

### Acide 5-chloro-1-(phénylsulfonyl)-1H-pyrrolo[3,2-b]pyridine-2-butanoïque

En opérant de façon analogue à l'exemple 2, au départ de l'ester obtenu selon l'exemple 9, on obtient le composé attendu sous forme d'un solide beige (rendement = 38 %).
F = 160-164°C.

### Exemple 11

### Acide 3-(1-benzènesulfonyl-5-chloro-1H-pyrrolo[3,2-b]pyridin-2-yl)-2,2-diméthylpropanoïque, méthyl ester

En opérant de façon analogue à l'exemple 1, au départ de l'ester méthylique de l'acide 2,2-diméthyl-4-pentynoïque, on obtient le composé attendu sous forme d'une huile jaune (rendement = 82 %).
¹H RMN (300 MHz, DMSOd₆) δ : 1,22 (s, 6H) ; 3,41 (s, 2H) ; 3,62 (s, 3H) ; 6,58 (s, 1H) ; 7,40 (d, 1H) ; 7,59 (t, 2H) ; 7,72 (t, 1H) ; 7,82 (d, 2H) ; 8,42 (d, 1H).

### Exemple 12

### Acide 3-(1-benzènesulfonyl-5-chloro-1H-pyrrolo[3,2-b]pyridin-2-yl)-2,2-diméthylpropanoïque

En opérant de façon analogue à l'exemple 2, au départ de l'ester obtenu selon l'exemple 11, on obtient le composé attendu sous forme d'un solide blanc (rendement = 18 %).
F = 208-211°C.

### Exemple 13

### Acide 2-[[1-(phénylsulfonyl)-5-(trifluorométhyl)-1H-pyrrolo[3,2-b]pyridin-2-yl]méthoxy]propanoïque, éthyl ester

En opérant de façon analogue à l'exemple 1, au départ du composé obtenu selon la préparation VII et de l'ester éthylique de l'acide 2-(2-propynyloxy)-propanoïque, on obtient le composé attendu sous forme d'une huile incolore (rendement = 63 %).
¹H RMN (300 MHz, DMSOd₆) δ : 1,17 (t, 3H) ; 1,31 (d, 3H) ; 4,11 (q, 2H) ; 4,23 (q, 1H) ; 4,97 (d, 1H) ; 5,10 (d, 1H) ; 7,12 (s, 1H) ; 7,61 (t, 2H) ; 7,75 (t, 1H) ; 7,80 (d, 1H) ; 8,06 (d, 2H) ; 8,63 (d, 1H).

### Exemple 14

### Acide 2-[[1-(phénylsulfonyl)-5-(trifluorométhyl)-1H-pyrrolo[3,2-b]pyridin-2-yl]méthoxy]propanoïque

En opérant de façon analogue à l'exemple 2, au départ de l'ester obtenu selon l'exemple 13, on obtient le composé attendu sous forme d'un solide blanc (rendement = 56 %).
F = 53-57°C.

### Exemple 15

### Acide 2-[[1-(phénylsulfonyl)-5-(trifluorométhyl)-1H-pyrrolo[3,2-b]pyridin-2-yl]méthoxy]-2-méthylpropanoïque, méthyl ester

En opérant de façon analogue à l'exemple 13, au départ du composé obtenu selon la préparation VII et de l'ester méthylique de l'acide 2-méthyl-2-(2-propynyloxy)propanoïque, on obtient le composé attendu sous forme d'une huile jaune (rendement = 30 %).
¹H RMN (300 MHz, DMSOd₆) δ : 1,44 (s, 6H) ; 3,65 (s, 3H) ; 4,93 (s, 2H) ; 7,10 (s, 1H) ; 7,63 (t, 2H) ; 7,76 (t, 1 H) ; 7,83 (d, 1 H) ; 8,05 (d, 2H) ; 8,64 (d, 1 H).

### Exemple 16

### Acide 2-[[1-(phénylsulfonyl)-5-(trifluorométhyl)-1H-pyrrolo[3,2-b]pyridin-2-yl]méthoxy]-2-méthylpropanoïque

En opérant de façon analogue à l'exemple 2, au départ de l'ester obtenu selon l'exemple 15, on obtient le composé attendu sous forme d'un solide beige (rendement = 45 %).
F = 122-125°C.

### Exemple 17

### Acide 1-(phénylsulfonyl)-5-(trifluorométhyl)-1H-pyrrolo[3,2-b]pyridine-2-propanoïque, méthyl ester

En opérant de façon analogue à l'exemple 13, au départ de l'ester méthylique de l'acide 4-pentynoïque, on obtient le composé attendu sous forme d'un solide beige (rendement = 91 %).
F = 119°C.

### Exemple 18

### Acide 1-(phénylsulfonyl)-5-(trifluorométhyl)-1H-pyrrolo[3,2-b]pyridine-2-propanoïque

En opérant de façon analogue à l'exemple 2, au départ de l'ester obtenu selon l'exemple 17, on obtient le composé attendu sous forme d'un solide blanc (rendement = 53 %).
F = 180 °C.

### Exemple 19

### Acide 1-(phénylsulfonyl)-5-(trifluorométhyl)-1H-pyrrolo[3,2-b]pyridine-2-butanoïque, méthyl ester

En opérant de façon analogue à l'exemple 13, au départ de l'ester méthylique de l'acide 5-hexynoïque, on obtient le composé attendu sous forme d'un solide beige (rendement = 88 %).
F = 95°C.

### Exemple 20

### Acide 1-(phénylsulfonyl)-5-(trifluorométhyl)-1H-pyrrolo[3,2-b]pyridine-2-butanoïque

En opérant de façon analogue à l'exemple 2, au départ de l'ester obtenu selon l'exemple 19, on obtient le composé attendu sous forme d'un solide blanc (rendement = 58 %).
F = 168°C.

### Exemple 21

### Acide 1-[(2-amino-6-benzothiazolyl)sulfonyl]-5-chloro-1H-pyrrolo[3,2-b]pyridine-2-butanoïque, méthyl ester

En opérant de façon analogue à l'exemple 3, au départ du composé obtenu selon la préparation X, on obtient le composé attendu sous forme d'un solide jaune (rendement = 63 %).
F = 85-90°C.

### Exemple 22

### Acide 1-[(2-amino-6-benzothiazolyl)sulfonyl]-5-chloro-1H-pyrrolo[3,2-b]-pyridine-2-butanoïque

En opérant de façon analogue à l'exemple 2, au départ du composé obtenu selon l'exemple 21, on obtient le produit attendu sous forme d'un solide blanc (rendement = 82 %).
F > 250°C.

### Exemple 23

### Acide 1-(1,3-benzodioxol-5-ylsulfonyl)-5-chloro-1H-pyrrolo[3,2-b]pyridine-2-butanoïque, méthyl ester

En opérant de façon analogue à l'exemple 3, au départ du composé obtenu selon la préparation IX, on obtient le composé attendu sous forme d'un solide jaune (rendement = 74 %).
F = 120°C.

### Exemple 24

### Acide 1-(1,3-benzodioxol-5-ylsulfonyl)-5-chloro-1H-pyrrolo[3,2-b]pyridine-2-butanoïque

En opérant de façon analogue à l'exemple 2, au départ du composé obtenu selon l'exemple 23, on obtient le produit attendu sous forme d'un solide beige (rendement = 95 %).
F = 160°C.

### Exemple 25

### Acide 5-chloro-1-[(3,5-diméthylphényl)sulfonyl]-1H-pyrrolo[3,2-b]pyridine-2-butanoïque, méthyl ester

En opérant de façon analogue à l'exemple 3, au départ du composé obtenu selon la préparation XI, on obtient le composé attendu sous forme d'un solide brun (rendement = 99 %).
F = 138°C.

### Exemple 26

### Acide 5-chloro-1-[(3,5-diméthylphényl)sulfonyl]-1H-pyrrolo[3,2-b]pyridine-2-butanoïque

En opérant de façon analogue à l'exemple 2, au départ du composé obtenu selon l'exemple 25, on obtient le produit attendu sous forme d'un solide beige (rendement = 88 %).
F = 200°C.

### Exemple 27

### Acide 5-chloro-1-[(2,5-diméthoxyphényl)sulfonyl]-1H-pyrrolo[3,2-b]pyridine-2-butanoïque, méthyl ester

En opérant de façon analogue à l'exemple 3, au départ du composé obtenu selon la préparation XII, on obtient le composé attendu sous forme d'un solide beige (rendement = 94 %).
F = 102°C.

### Exemple 28

### Acide 5-chloro-1-[(2,5-diméthoxyphényl)sulfonyl]-1H-pyrrolo[3,2-b]pyridine-2-butanoïque

En opérant de façon analogue à l'exemple 2, au départ du composé obtenu selon l'exemple 27, on obtient le produit attendu sous forme d'un solide blanc (rendement = 95 %).
F = 227-231°C.

### Exemple 29

### Acide 5-chloro-1-(1-naphthalénylsulfonyl)-1H-pyrrolo[3,2-b]pyridine-2-butanoïque, méthyl ester

En opérant de façon analogue à l'exemple 3, au départ du composé obtenu selon la préparation XIII, on obtient le composé attendu sous forme d'une huile jaune (rendement = 86 %).
¹H RMN (300 MHz, DMSOd₆) δ : 1,83 (quin, 2H) ; 2,34 (t, 2H) ; 2,88 (t, 2H) ; 3,52 (s, 3H) ; 6,83 (s, 1H) ; 7,39 (d, 1H) ; 7,71 (m, 4H) ; 8,14 (m, 1H) ; 8,30 (m, 1H) ; 8,37 (m, 2H).

### Exemple 30

### Acide 5-chloro-1-(1-naphthalénylsulfonyl)-1H-pyrrolo[3,2-b]pyridine-2-butanoïque

En opérant de façon analogue à l'exemple 2, au départ du composé obtenu selon l'exemple 29, on obtient le produit attendu sous forme d'un solide blanc (rendement = 96 %).
F = 202-206°C.

### Exemple 31

### Acide 5-chloro-1-[(3,4-dihydro-4-méthyl-2H-1,4-benzoxazin-7-yl)sulfonyl]-1H-pyrrolo[3,2-b]pyridine-2-butanoïque, méthyl ester

En opérant de façon analogue à l'exemple 3, au départ du composé obtenu selon la préparation XIV, on obtient le composé attendu sous forme d'un solide blanc (rendement = 78 %).
F = 106-110°C.

### Exemple 32

### Acide 5-chloro-1-[(3,4-dihydro-4-méthyl-2H-1,4-benzoxazin-7-yl)sulfonyl]-1H-pyrrolo[3,2-b]pyridine-2-butanoïque

En opérant de façon analogue à l'exemple 2, au départ du composé obtenu selon l'exemple 31, on obtient le produit attendu sous forme d'un solide blanc (rendement = 98 %).
F = 180-183°C.

### Exemple 33

### Acide 1-(1,3-benzodioxol-5-ylsulfonyl)-5-chloro-1H-pyrrolo[3,2-b]pyridine-2-propanoïque, méthyl ester

En opérant de façon analogue à l'exemple 3, au départ du composé obtenu selon la préparation XV, on obtient le composé attendu sous forme d'une huile jaune (rendement = 76 %).
¹H RMN (300 MHz, DMSOd₆) δ : 2,84 (t, 2H) ; 3,31 (t, 2H) ; 3,47 (t, 2H) ; 3,62 (s, 3H) ; 6,16 (s, 2H) ; 6,72 (s, 1H) ; 7,09 (d, 1H) ; 7,37 (d, 1H) ; 7,38 (s, 1H) ; 7,51 (dd, 1H) ; 8,40 (s, 1H).

### Exemple 34

### Acide 1-(1,3-benzodioxol-5-ylsulfonyl)-5-chloro-1H-pyrrolo[3,2-b]pyridine-2-propanoïque

En opérant de façon analogue à l'exemple 2, au départ du composé obtenu selon l'exemple 33, on obtient le produit attendu sous forme d'un solide marron (rendement = 98 %).
F = 186°C.

### Exemple 35

### Acide 1-(6-benzothiazolylsulfonyl)-5-chloro-1H-pyrrolo[3,2-b]pyridine-2-propanoïque, méthyl ester

En opérant de façon analogue à l'exemple 3, au départ du composé obtenu selon la préparation XVI, on obtient le composé attendu sous forme d'un solide blanc (rendement = 57 %).
F = 146°C.

### Exemple 36

### Acide 1-(6-benzothiazolylsulfonyl)-5-chloro-1H-pyrrolo[3,2-b]pyridine-2-propanoïque

En opérant de façon analogue à l'exemple 2, au départ du composé obtenu selon l'exemple 35, on obtient le produit attendu sous forme d'un solide blanc (rendement = 90 %).
F = 248°C.

### Exemple 37

### Acide 5-chloro-1-(phénylsulfonyl)-1H-pyrrolo[3,2-b]pyridine-2-propanoïque, sel de sodium

On mélange 1,46 g (4 mM) de l'acide obtenu selon l'exemple 2 dans 12 ml de tétrahydrofurane et on ajoute 8 ml (4 mM) d'une solution d'hydroxyde de sodium 0,5N dans l'eau. Le milieu réactionnel est agité pendant 2 heures à température ambiante puis concentré sous pression réduite. Le résidu huileux est trituré dans du méthanol et le précipité blanc formé est séparé par filtration et séché sous vide. On obtient le sel attendu sous forme d'un solide blanc pulvérulent (rendement = 98 %).
F = 200°C.

### Exemple 38

### Acide 2-[[1-(2,1,3-benzothiadiazol-5-ylsulfonyl)-5-chloro-1H-pyrrolo[3,2-b]pyridin-2-yl]méthoxy]propanoïque; éthyl ester

En opérant de façon analogue à la préparation VI, au départ de l'ester obtenu selon la préparation XVII et du chlorure de 2,1,3-benzothiadiazole-5-sulfonyle, on obtient le composé attendu sous forme d'un solide orange (rendement = 46 %).
F = 77-79°C.

### Exemple 39

### Acide 2-[[1-(2,1,3-benzothiadiazol-5-ylsulfonyl)-5-chloro-1H-pyrrolo[3,2-b]pyridin-2-yl]méthoxy]propanoïque

En opérant de façon analogue à l'exemple 2, au départ de l'ester obtenu selon l'exemple 38, on obtient le composé attendu sous forme d'un solide blanc (rendement = 16 %).
F = 192-194°C.

### Exemple 40

### Acide 2-[[5-chloro-1-[(2-méthyl-7-benzothiazolyl)sulfonyl]-1H-pyrrolo[3,2-b]pyridin-2-yl]méthoxy]propanoïque, éthyl ester

En opérant de façon analogue à la préparation VI, au départ de l'ester obtenu selon la préparation XVII et du chlorure de 2-méthyl-7-benzothiazolesulfonyle, on obtient le composé attendu sous forme d'une huile incolore (rendement = 50 %).
¹H RMN (300 MHz, DMSOd₆) δ: 8,32 (d, 1H) ; 8,27 (d, 1H) ; 7,94 (d, 1H) ; 7,67 (t, 1H) ; 7,43 (d, 1H) ; 7,00 (s, 1H) ; 5,02 (d, 1H) ; 4,88 (d, 1H) ; 4,13 (q, 1H) ; 4,07 (q, 2H) ; 2,85 (s, 3H) ; 1,15 (m, 6H).

### Exemple 41

### Acide 2-[[5-chloro-1-[(2-méthyl-7-benzothiazolyl)sulfonyl]-1H-pyrrolo[3,2-b]pyridin-2-yl]méthoxy]propanoïque

En opérant de façon analogue à l'exemple 2, au départ de l'ester obtenu selon l'exemple 40, on obtient le composé attendu sous forme d'un solide blanc (rendement = 28 %).
F = 203-205°C.

### Exemple 42

### Acide 2-[[5-chloro-1-[(3-méthoxyphényl)sulfonyl]-1H-pyrrolo[3,2-b]pyridin-2-yl]méthoxy]-2-méthylpropanoïque, méthyl ester

En opérant de façon analogue à l'exemple 1, au départ du composé obtenu selon la préparation XVIII et de l'ester méthylique de l'acide 2-méthyl-2-(2-propynyloxy)propanoïque, on obtient le composé attendu sous forme d'un solide blanc (rendement = 33 %).
F = 153°C.

### Exemple 43

### Acide 2-[[5-chloro-1-[(3-méthoxyphényl)sulfonyl]-1H-pyrrolo[3,2-b]pyridin-2-yl]méthoxy]-2-méthylpropanoïque

On prépare une solution de 186 mg (0,41 mM) de l'ester obtenu selon l'exemple 42 dans 7,5 ml d'acide acétique et on ajoute, sous agitation et à température ambiante, 0,75 ml d'acide chlorhydrique concentré. Le milieu réactionnel est porté à léger reflux pendant 18 heures puis concentré sous pression réduite. Le résidu d'évaporation est repris à l'eau et extrait par du dichlorométhane. La phase organique est lavée à l'eau, séchée sur sulfate de magnésium et concentrée sous pression réduite. Le produit brut est purifié par chromatographie sur gel de silice en éluant à l'aide d'un mélange dichlorométhane/méthanol (gradient de 99/1 à 90/10, v/v). On obtient ainsi l'acide attendu sous forme d'un solide beige (rendement = 70%).
F= 130°C.

### Exemple 44

### Acide 2-[[1-[(2-acétylamino-6-benzothiazolyl)sulfonyl]-5-chloro-1H-pyrrolo[3,2-b]pyridin-2-yl]méthoxy]propanoïque, éthyl ester

En opérant de façon analogue à la préparation VI, au départ de l'ester obtenu selon la préparation XVII et du chlorure de 2-acétylamino-6-benzothiazolesulfonyle, on obtient le composé attendu sous forme d'un solide blanc (rendement = 25 %).
F = 100-102°C.

### Exemple 45

### Acide 2-[[1-[(2-acétylamino-6-benzothiazolyl)sulfonyl]-5-chloro-1H-pyrrolo[3,2-b]pyridin-2-yl]méthoxy]propanoïque

En opérant de façon analogue à l'exemple 43, au départ de l'ester obtenu selon l'exemple 44, on obtient le composé attendu sous forme d'un solide blanc (rendement = 25 %).
F = 257-260°C.

### Exemple 46

### Acide 2-[[1-[(2-amino-6-benzothiazolyl)sulfonyl]-5-chloro-1H-pyrrolo[3,2-b]pyridin-2-yl]méthoxy]propanoïque, éthyl ester

En opérant de façon analogue à la préparation VI, au départ de l'ester obtenu selon la préparation XVII et du chlorure de 2-amino-6-benzothiazolesulfonyle, on obtient le composé attendu sous forme d'un solide jaune (rendement = 25 %).
F = 86-88°C.

### Exemple 47

### Acide 2-[[1-[(2-amino-6-benzothiazolyl)sulfonyl]-5-chloro-1H-pyrrolo[3,2-b]pyridin-2-yl]méthoxy]propanoïque

En opérant de façon analogue à l'exemple 43, au départ de l'ester obtenu selon l'exemple 46, on obtient le composé attendu sous forme d'un solide blanc (rendement = 65 %).
F = 218-220°C.

### Exemple 48

### Acide 2-[[1-(6-benzothiazolylsulfonyl)-5-(trifluorométhyl)-1H-pyrrolo[3,2-b]pyridin-2-yl]méthoxy]-2-méthylpropanoïque, méthyl ester

En opérant de façon analogue à la préparation VI, au départ du composé obtenu selon la préparation XIX et du chlorure de 6-benzothiazolesulfonyle, on obtient le composé attendu sous forme d'un solide blanc (rendement = 13 %).
F = 124-127°C.

### Exemple 49

### Acide 2-[[1-(6-benzothiazolylsulfonyl)-5-(trifluorométhyl)-1H-pyrrolo[3,2-b]pyridin-2-yl]méthoxy]-2-méthylpropanoïque

En opérant de façon analogue à l'exemple 2, au départ de l'ester obtenu selon l'exemple 48, on obtient le composé attendu sous forme d'un solide blanc (rendement = 16 %).
F = 180-182°C.

### Exemple 50

### Acide 2-[[1-(6-benzothiazolylsulfonyl)-5-chloro-1H-pyrrolo[3,2-b]pyridin-2-yl]méthoxy]propanoïque, éthyl ester

On prépare une solution de 350 mg (1,24 mM) de l'ester obtenu selon la préparation XVII dans 8 ml de pyridine et on ajoute, sous agitation et à 0°C, 550 mg (2,35 mM) de chlorure de 6-benzothiazolesulfonyle. Le milieu réactionnel est agité à température ambiante pendant 24 heures puis dilué par de l'acétate d'éthyle. Cette phase organique est lavée par une solution d'acide chlorhydrique 2N puis à l'eau, séchée sur sulfate de magnésium et concentrée sous pression réduite. L'huile obtenue est reprise dans 10 ml de THF et on ajoute progressivement à cette solution refroidie à 0°C, 2,6 ml (2,6 mM) de fluorure de tétrabutylammonium en solution 1M dans le THF. Le mélange réactionnel est agité pendant 24 heures à 4°C, puis dilué dans du DCM. La phase organique obtenue est lavée par une solution d'acide chlorhydrique N, puis à l'eau, séchée sur sulfate de magnésium et concentrée sous pression réduite. Le produit brut est purifié par chromatographie sur gel de silice en éluant à l'aide d'un mélange toluène/acétate d'éthyle (95/5 puis 9/1 ; v/v). On obtient ainsi le produit attendu sous forme d'huile jaune (rendement = 31%).
¹H RMN (250 MHz, DMSOd₆) δ : 9,68 (s, 1H) ; 9,11 (d, 1H) ; 8,50 (d, 1H) ; 8,23 (d, 1H) ; 8,10 (dd, 1H) ; 7,43 (d, 1H) ; 6,96 (s, 1H) ; 5,08 (d, 1H) ; 4,96 (d, 1H) ; 4,25 (q, 1H) ; 4,12 (q, 2H) ; 1,28 (d, 3H) ; 1,19 (t, 3H).

### Exemple 51

### Acide 2-[[1-(6-benzothiazolylsulfonyl)-5-chloro-1H-pyrrolo[3,2-b]pyridin-2-yl]méthoxy]propanoïque

En opérant de façon analogue à l'exemple 2, au départ de l'ester obtenu selon l'exemple 50, on obtient le composé attendu sous forme d'un solide blanc (rendement = 40 %).
F = 179°C.

### Exemple 52

### Acide 2-[[1-(6-benzothiazolylsulfonyl)-5-(trifluorométhyl)-1H-pyrrolo[3,2-b]pyridin-2-yl]méthoxy]propanoïque, éthyl ester

On prépare une solution de 140 mg (0,443 mM) du composé obtenu selon la préparation XX dans 2 ml de pyridine et on ajoute 228 mg (0,98 mM) de chlorure de 6-benzothiazolesulfonyle. Le mélange réactionnel est agité à température ambiante pendant 48 heures puis dilué par de l'acétate d'éthyle. La phase organique obtenue est lavée successivement à l'eau, avec de l'acide chlorhydrique N, à nouveau à l'eau, avec une solution de bicarbonate de sodium et enfin par une solution de chlorure de sodium. Après séchage sur sulfate de magnésium et concentration, on obtient 242 mg d'ester éthylique de l'acide 2-[[3-[3-(6-benzothiazolylsulfonylamino)-6-(trifluorométhyl)-2-pyridinyl]-2-propynyl]oxy]-propanoïque que l'on reprend dans 5 ml de DCM dans un tube réacteur adapté pour chauffage sous micro-ondes. On ajoute 100 mg (0,5 mM) d'acétate de cuivre monohydraté et on chauffe ce mélange à 150°C pendant 15 minutes. Le milieu réactionnel est refroidi, filtré et concentré sous pression réduite. Après purification par chromatographie sur colonne de silice en éluant à l'aide d'un mélange toluène/acétate d'éthyle (9/1 ; v/v), on obtient le composé attendu sous forme d'un solide beige (rendement = 42 %).
F = 96°C.

### Exemple 53

### Acide 2-[[1-(6-benzothiazolylsulfonyl)-5-(trifluorométhyl)-1H-pyrrolo[3,2-b]pyridin-2-yl]méthoxy]propanoïque

En opérant de façon analogue à l'exemple 2, au départ de l'ester obtenu selon l'exemple 52, on obtient le composé attendu sous forme d'un solide beige (rendement = 50 %).
F = 124°C.

### Exemple 54

### Acide 5-chloro-1-[(2,5-diméthoxyphényl)sulfonyl]-1H-pyrrolo[3,2-b]pyridine-2-propanoïque, méthyl ester

En opérant de façon analogue à l'exemple 52, au départ du composé obtenu selon la préparation VIII et du chlorure de 2,5-diméthoxybenzènesulfonyle, on obtient le composé attendu sous forme d'un solide beige (rendement = 77 %).
F = 127°C.

### Exemple 55

### Acide 5-chloro-1-[(2,5-diméthoxyphényl)sulfonyl]-1H-pyrrolo[3,2-b]pyridine-2-propanoïque

En opérant de façon analogue à l'exemple 2, au départ de l'ester obtenu selon l'exemple 54, on obtient le composé attendu sous forme d'un solide blanc (rendement = 71 %).
F = 204-209°C.

### Exemple 56

### Acide 1-[(2-amino-6-benzothiazolyl)sulfonyl]-5-chloro-1H-pyrrolo[3,2-b]pyridine-2-propanoïque, méthyl ester

En opérant de façon analogue à l'exemple 52, au départ de l'ester obtenu selon la préparation VIII et du chlorure de 2-amino-6-benzothiazolesulfonyle, on obtient le composé attendu sous forme d'un solide jaune (rendement = 38 %).
F = 205-215°C.

### Exemple 57

### Acide 1-[(2-amino-6-benzothiazolyl)sulfonyl]-5-chloro-1H-pyrrolo[3,2-b]-pyridine-2-propanoïque

En opérant de façon analogue à l'exemple 2, au départ de l'ester obtenu selon l'exemple 56, on obtient le composé attendu sous forme d'un solide beige (rendement = 57 %).
F > 260°C.

### Exemple 58

### Acide 5-chloro-1-[(3,5-diméthylphényl)sulfonyl]-1H-pyrrolo[3,2-b]pyridine-2-propanoïque, méthyl ester

En opérant de façon analogue à l'exemple 52, au départ du composé obtenu selon la préparation VIII et du chlorure de 3,5-diméthylbenzènesulfonyle, on obtient le composé attendu sous forme d'un solide blanc (rendement = 86 %).
F = 182-185°C.

### Exemple 59

### Acide 5-chloro-1-[(3,5-diméthylphényl)sulfonyl]-1H-pyrrolo[3,2-b]pyridine-2-propanoïque

En opérant de façon analogue à l'exemple 2, au départ de l'ester obtenu selon l'exemple 58, on obtient le composé attendu sous forme d'un solide blanc (rendement = 99 %).
F = 181-186°C.

### Exemple 60

### Acide 5-chloro-1-[(3,4-dihydro-4-méthyl-2H-1,4-benzoxazin-7-yl)sulfonyl]-1H-pyrrolo[3,2-b]pyridine-2-propanoïque, méthyl ester

En opérant de façon analogue à l'exemple 52, au départ du composé obtenu selon la préparation VIII et du chlorure de 3,4-dihydro-4-méthyl-2*H*-1,4-benzoxazine-7-sulfonyle, on obtient le composé attendu sous forme d'une huile jaune (rendement = 87 %).
¹H RMN (300 MHz, DMSOd₆) δ : 8,43 (d, 1H) ; 7,37 (d, 1H) ; 7,07 (dd, 1H) ; 6,94 (d, 1H) ; 6,81 (d, 1H) ; 6,69 (s, 1H) ; 4,25 (m, 2H) ; 3,62 (s, 3H) ; 3,26 (m, 4H) ; 2,86 (m, 2H) ; 2,82 (s, 3H).

### Exemple 61

### Acide 5-chloro-1-[(3,4-dihydro-4-méthyl-2H-1,4-benzoxazin-7-yl)sulfonyl]-1H-pyrrolo[3,2-b]pyridine-2-propanoïque

En opérant de façon analogue à l'exemple 2, au départ de l'ester obtenu selon l'exemple 60, on obtient le composé attendu sous forme d'un solide blanc (rendement = 98 %).
F = 175-185°C.

### Exemple 62

### Acide 5-chloro-1-(8-quinolinylsulfonyl)-1H-pyrrolo[3,2-b]pyridine-2-propanoïque, méthyl ester

En opérant de façon analogue à l'exemple 52, au départ du composé obtenu selon la préparation VIII et du chlorure de 8-quinoléïnesulfonyle, on obtient le composé attendu sous forme d'un solide blanc (rendement = 59 %).
F = 120°C.

### Exemple 63

### Acide 5-chloro-1-(8-quinolinylsulfonyl)-1H-pyrrolo[3,2-b]pyridine-2-propanoïque

En opérant de façon analogue à l'exemple 2, au départ de l'ester obtenu selon l'exemple 62, on obtient le composé attendu sous forme d'un solide blanc (rendement = 98 %).
F = 183°C.

### Exemple 64

### Acide 5-chloro-1-(1-naphthalénylsulfonyl)-1H-pyrrolo[3,2-b]pyridine-2-propanoïque, méthyl ester

En opérant de façon analogue à l'exemple 52, au départ du composé obtenu selon la préparation VIII et du chlorure de 1-naphthalènesulfonyle, on obtient le composé attendu sous forme d'une huile jaune (rendement = 66 %).
¹H RMN (250 MHz, DMSOd₆) δ : 8,34 (m, 3H) ; 8,15 (m, 1H) ; 7,69 (m, 4H) ; 7,39 (d, 1H) ; 6,81 (s, 1H) ; 3,56 (s, 3H) ; 3,12 (t, 2H) ; 2,74 (t, 2H).

### Exemple 65

### Acide 5-chloro-1-(1-naphthalénylsulfonyl)-1H-pyrrolo[3,2-b]pyridine-2-propanoïque

En opérant de façon analogue à l'exemple 2, au départ de l'ester obtenu selon l'exemple 64, on obtient le composé attendu sous forme d'un solide blanc (rendement = 86 %).
F = 93°C.

### Exemple 66

### Acide 1-[(1-acétyl-2,3-dihydro-1H-indol-5-yl)sulfonyl]-5-chloro-1H-pyrrolo-[3,2-b]pyridine-2-propanoïque, méthyl ester

En opérant de façon analogue à l'exemple 52, au départ du composé obtenu selon la préparation VIII et du chlorure de 1-acétyl-2,3-dihydro-1*H*-indole-5-sulfonyle, on obtient le composé attendu sous forme d'un solide brun (rendement = 93 %).
F = 66°C.

### Exemple 67

### Acide 1-[(1-acétyl-2,3-dihydro-1H-indol-5-yl)sulfonyl]-5-chloro-1H-pyrrolo-[3,2-b]pyridine-2-propanoïque

En opérant de façon analogue à l'exemple 2, au départ de l'ester obtenu selon l'exemple 66, on obtient le composé attendu sous forme d'un solide jaune (rendement = 37 %).
F = 216°C

### Exemple 68

### Acide 5-chloro-1-[3-(trifluorométhoxy)phénylsulfonyl]-1H-pyrrolo[3,2-b]-pyridine-2-propanoïque, méthyl ester

En opérant de façon analogue à l'exemple 52, au départ du composé obtenu selon la préparation VIII et du chlorure de 3-(trifluorométhoxy)benzènesulfonyle, on obtient le composé attendu sous forme d'un solide orange (rendement = 71 %).
F = 102°C.

### Exemple 69

### Acide 5-chloro-1-[3-(trifluorométhoxy)phénylsulfonyl]-1H-pyrrolo[3,2-b]-pyridine-2-propanoïque

En opérant de façon analogue à l'exemple 2, au départ de l'ester obtenu selon l'exemple 68, on obtient le composé attendu sous forme d'un solide jaune (rendement = 9 %).
F = 146°C.

### Exemple 70

### Acide 1-[(3,5-diméthylphényl)sulfonyl]-5-(trifluorométhyl)-1H-pyrrolo[3,2-b]pyridine-2-propanoïque, méthyl ester

En opérant de façon analogue à l'exemple 52, au départ du composé obtenu selon la préparation XXI et du chlorure de 3,5-diméthylbenzènesulfonyle, on obtient le composé attendu sous forme d'un solide beige (rendement = 51 %).
¹H RMN (250 MHz, DMSOd₆) δ : 8,60 (d, 1H) ; 7,79 (d, 1H) ; 7,57 (s, 2H) ; 7,39 (s, 1H) ; 6,90 (s, 1H) ; 3,63 (s, 3H) ; 3,33 (t, 2H) ; 2,87 (t, 2H) ; 2,31 (s, 6H).

### Exemple 71

### Acide 1-[(3,5-diméthylphényl)sulfonyl]-5-(trifluorométhyl)-1H-pyrrolo[3,2-b]pyridine-2-propanoïque

En opérant de façon analogue à l'exemple 2, au départ de l'ester obtenu selon l'exemple 70, on obtient le composé attendu sous forme d'un solide blanc (rendement = 91 %).
F = 185-189°C.

### Exemple 72

### Acide 1-(8-quinolinylsulfonyl)-5-(trifluorométhyl)-1H-pyrrolo[3,2-b]pyridine-2-propanoïque, méthyl ester

En opérant de façon analogue à l'exemple 52, au départ du composé obtenu selon la préparation XXI et du chlorure de 8-quinoleïnesulfonyle, on obtient le composé attendu sous forme d'un solide blanc (rendement = 51 %).
¹H RMN (300 MHz, DMSOd₆) δ : 8,72 (m, 2H) ; 8,45 (m, 3H) ; 7,88 (t, 1H) ; 7,72 (d, 1H) ; 7,62 (dd, 1H) ; 6,76 (s, 1H) ; 3,60 (s, 3H) ; 3,54 (t, 2H) ; 2,88 (t, 2H)

### Exemple 73

### Acide 1-(8-quinolinylsulfonyl)-5-(trifluorométhyl)-1H-pyrrolo[3,2-b]pyridine-2-propanoïque

En opérant de façon analogue à l'exemple 2, au départ de l'ester obtenu selon l'exemple 72, on obtient le composé attendu sous forme d'un solide blanc (rendement = 56 %).
F = 216-217°C.

### Exemple 74

### Acide 1-(1-naphthalénylsulfonyl)-5-(trifluorométhyl)-1H-pyrrolo[3,2-b]-pyridine-2-propanoïque, méthyl ester

En opérant de façon analogue à l'exemple 52, au départ du composé obtenu selon la préparation XXI et du chlorure de 1-naphthalènesulfonyle, on obtient le composé attendu sous forme d'un solide beige (rendement = 63 %).
¹H RMN (250 MHz, DMSOd₆) δ : 8,56 (d, 1H) ; 8,36 (m, 2H) ; 8,17 (m, 1H) ; 7,72 (m, 5H) ; 6,98 (s, 1H) ; 3,56 (s, 3H) ; 3,15 (t, 2H) ; 2,77 (t, 2H).

### Exemple 75

### Acide 1-(1-naphthalénylsulfonyl)-5-(trifluorométhyl)-1H-pyrrolo[3,2-b]pyridine-2-propanoïque

En opérant de façon analogue à l'exemple 2, au départ de l'ester obtenu selon l'exemple 74, on obtient le composé attendu sous forme d'un solide blanc (rendement = 42 %).
F = 116-117°C.

### Exemple 76

### Acide 5-chloro-1-(8-quinolinylsulfonyl)-1H-pyrrolo[3,2-b]pyridine-2-butanoïque, méthyl ester

En opérant de façon analogue à l'exemple 52, au départ du composé obtenu selon la préparation V et du chlorure de 8-quinoleïnesulfonyle, on obtient le composé attendu sous forme d'un solide blanc (rendement = 25 %).
F = 64°C.

### Exemple 77

### Acide 5-chloro-1-(8-quinolinylsulfonyl)-1H-pyrrolo[3,2-b]pyridine-2-butanoïque

En opérant de façon analogue à l'exemple 2, au départ de l'ester obtenu selon l'exemple 76, on obtient le composé attendu sous forme d'un solide blanc (rendement = 99 %).
F = 186-189°C.

### Exemple 78

### Acide 5-chloro-1-[(2,2-difluoro-1,3-benzodioxol-5-yl)sulfonyl]-1H-pyrrolo[3,2-b]pyridine-2-butanoïque, méthyl ester

En opérant de façon analogue à l'exemple 76, au départ du chlorure de 2,2-difluoro-1,3-benzodioxole-5-sulfonyle, on obtient le composé attendu sous forme d'une huile incolore (rendement = 95 %).
¹H RMN (250 MHz, DMSOd₆) δ : 8,43 (d, 1H) ; 8,09 (d, 1H) ; 7,83 (dd, 1H) ; 7,60 (d, 1H) ; 7,38 (d, 1H) ; 6,77 (s, 1H) ; 3,59 (s, 3H) ; 3,07 (t, 2H) ; 2,45 (t, 2H) ; 1,97 (m, 2H).

### Exemple 79

### Acide 5-chloro-1-[(2,2-difluoro-1,3-benzodioxol-5-yl)sulfonyl]-1H-pyrrolo-[3,2-b]pyridine-2-butanoïque

En opérant de façon analogue à l'exemple 2, au départ de l'ester obtenu selon l'exemple 78, on obtient le composé attendu sous forme d'un solide blanc (rendement = 78 %).
F = 216°C.

### Exemple 80

### Acide 5-chloro-1-[(2-méthyl-5-benzothiazolyl)sulfonyl]-1H-pyrrolo[3,2-b]pyridine-2-butanoïque, méthyl ester

En opérant de façon analogue à l'exemple 76, au départ du chlorure de 2-méthyl-5-benzothiazolesulfonyle, on obtient le composé attendu sous forme d'un solide beige (rendement = 79 %).
F = 114°C.

### Exemple 81

### Acide 5-chloro-1-[(2-méthyl-5-benzothiazolyl)sulfonyl]-1H-pyrrolo[3,2-b]pyridine-2-butanoïque

En opérant de façon analogue à l'exemple 2, au départ de l'ester obtenu selon l'exemple 80, on obtient le composé attendu sous forme d'un solide beige (rendement = 98 %).
F = 173°C.

### Exemple 82

### Acide 5-chloro-1-[(2-méthyl-6-benzothiazolyl)sulfonyl]-1H-pyrrolo[3,2-b]pyridine-2-butanoïque, méthyl ester

En opérant de façon analogue à l'exemple 76, au départ du chlorure de 2-méthyl-6-benzothiazolesulfonyle, on obtient le composé attendu sous forme d'un solide beige (rendement = 69 %).
F = 117°C.

### Exemple 83

### Acide 5-chloro-1-[(2-méthyl-6-benzothiazolyl)sulfonyl]-1H-pyrrolo[3,2-b]pyridine-2-butanoïque

En opérant de façon analogue à l'exemple 2, au départ de l'ester obtenu selon l'exemple 82, on obtient le composé attendu sous forme d'un solide jaune (rendement = 99 %).
F = 184°C.

### Exemple 84

### Acide 5-chloro-1-[(2-méthyl-7-benzothiazolyl)sulfonyl]-1H-pyrrolo[3,2-b]pyridine-2-butanoïque, méthyl ester

En opérant de façon analogue à la préparation VI, au départ du composé obtenu selon la préparation V et du chlorure de 2-méthyl-7-benzothiazolesulfonyle, on obtient le composé attendu sous forme d'un solide jaune (rendement = 48 %).
F = 138°C.

### Exemple 85

### Acide 5-chloro-1-[(2-méthyl-7-benzothiazolyl)sulfonyl]-1H-pyrrolo[3,2-b]pyridine-2-butanoïque

En opérant de façon analogue à l'exemple 2, au départ de l'ester obtenu selon l'exemple 84, on obtient le composé attendu sous forme d'un solide beige (rendement = 90 %).
F = 225°C.

### Exemple 86

### Acide 5-chloro-1-[(2,3-dihydro-5-benzofuranyl)sulfonyl]-1H-pyrrolo[3,2-b]pyridine-2-butanoïque, méthyl ester

En opérant de façon analogue à l'exemple 76, au départ du chlorure de 2,3-dihydro-5-benzofuranesulfonyle, on obtient le composé attendu sous forme d'un solide jaune (rendement = 82 %).
F = 201°C.

### Exemple 87

### Acide 5-chloro-1-[(2,3-dihydro-5-benzofuranyl)sulfonyl]-1H-pyrrolo[3,2-b]pyridine-2-butanoïque

En opérant de façon analogue à l'exemple 2, au départ de l'ester obtenu selon l'exemple 86, on obtient le composé attendu sous forme d'un solide blanc (rendement = 98 %).
F = 225°C.

### Exemple 88

### Acide 5-chloro-1-[(3,5-dichlorophényl)sulfonyl]-1H-pyrrolo[3,2-b]pyridine-2-butanoïque, méthyl ester

En opérant de façon analogue à l'exemple 76, au départ du chlorure de 3,5-dichlorobenzènesulfonyle, on obtient le composé attendu sous forme d'un solide blanc (rendement = 66 %).
F = 112-114°C.

### Exemple 89

### Acide 5-chloro-1-[(3,5-dichlorophényl)sulfonyl]-1H-pyrrolo[3,2-b]pyridine-2-butanoïque

En opérant de façon analogue à l'exemple 2, au départ de l'ester obtenu selon l'exemple 88, on obtient le composé attendu sous forme d'un solide blanc (rendement = 28 %).
F = 164-165°C

### Exemple 90

### Acide 1-[(2-acétylamino-6-benzothiazolyl)sulfonyl]-5-chloro-1H-pyrrolo[3,2-b]pyridine-2-butanoïque, méthyl ester

En opérant de façon analogue à l'exemple 76, au départ du chlorure de 2-acétylamino-6-benzothiazolesulfonyle, on obtient le composé attendu sous forme d'un solide jaune (rendement = 35 %).
F = 274°C.

### Exemple 91

### Acide 1-[(2-acétylamino-6-benzothiazolyl)sulfonyl]-5-chloro-1H-pyrrolo[3,2-b]pyridine-2-butanoïque

En opérant de façon analogue à l'exemple 2, au départ de l'ester obtenu selon l'exemple 90, on obtient le composé attendu sous forme d'un solide blanc (rendement = 87 %).
F = 270°C.

### Exemple 92

### Acide 1-[(2-amino-6-benzoxazolyl)sulfonyl]-5-chloro-1H-pyrrolo[3,2-b]-pyridine-2-butanoïque, méthyl ester

En opérant de façon analogue à l'exemple 76, au départ du chlorure de 2-amino-6-benzoxazolesulfonyle, on obtient le composé attendu sous forme d'un solide blanc (rendement = 35 %).
F = 241°C.

### Exemple 93

### Acide 1-[(2-amino-6-benzoxazolyl)sulfonyl]-5-chloro-1H-pyrrolo[3,2-b]-pyridine-2-butanoïque

En opérant de façon analogue à l'exemple 2, au départ de l'ester obtenu selon l'exemple 92, on obtient le composé attendu sous forme d'un solide blanc (rendement = 96 %).
F = 273°C.

### Exemple 94

### Acide 5-chloro-1-[(2-fluorophényl)sulfonyl]-1H-pyrrolo[3,2-b]pyridine-2-butanoïque, méthyl ester

En opérant de façon analogue à l'exemple 76, au départ du chlorure de 2-fluorobenzènesulfonyle, on obtient le composé attendu sous forme d'un solide brun (rendement = 87 %).
F = 83°C.

### Exemple 95

### Acide 5-chloro-1-[(2-fluorophényl)sulfonyl]-1H-pyrrolo[3,2-b]pyridine-2-butanoïque

En opérant de façon analogue à l'exemple 2, au départ de l'ester obtenu selon l'exemple 94, on obtient le composé attendu sous forme d'un solide beige (rendement = 92 %).
F = 176°C.

### Exemple 96

### Acide 5-chloro-1-[[2-(trifluorométhyl)phényl]sulfonyl]-1H-pyrrolo[3,2-b]-pyridine-2-butanoïque, méthyl ester

En opérant de façon analogue à la préparation VI, au départ du composé obtenu selon la préparation V et du chlorure de 2-(trifluorométhyl)benzènesulfonyle, on obtient le composé attendu sous forme d'un solide jaune (rendement = 39 %).
F = 127°C.

### Exemple 97

### Acide 5-chloro-1-[[2-(trifluorométhyl)phényl]sulfonyl]-1H-pyrrolo[3,2-b]pyridine-2-butanoïque

En opérant de façon analogue à l'exemple 2, au départ de l'ester obtenu selon l'exemple 96, on obtient le composé attendu sous forme d'un solide jaune (rendement = 89 %).
F = 171°C.

### Exemple 98

### Acide 1-([1,1'-biphényl]-3-ylsulfonyl)-5-chloro-1H-pyrrolo[3,2-b]pyridine-2-butanoïque, méthyl ester

En opérant de façon analogue à la préparation VI, au départ du composé obtenu selon la préparation V et du chlorure de [1,1'-biphényl]-3-sulfonyle, on obtient le composé attendu sous forme d'un solide blanc (rendement = 68 %).
¹H RMN (300 MHz, DMSOd₆) δ : 8,49 (d, 1H) ; 8,03 (m, 2H) ; 7,78 (d, 1H) ; 7,66 (m, 3H) ; 7,47 (m, 3H) ; 7,39 (d, 1H) ; 6,79 (s, 1H) ; 3,55 (s, 3H) ; 3,11 (t, 2H) ; 2,44 (t, 2H) ; 2,01 (m, 2H).

### Exemple 99

### Acide 1-([1,1'-biphényl]-3-ylsulfonyl)-5-chloro-1H-pyrrolo[3,2-b]pyridine-2-butanoïque

En opérant de façon analogue à l'exemple 2, au départ de l'ester obtenu selon l'exemple 98, on obtient le composé attendu sous forme d'un solide blanc (rendement = 23 %).
F = 147-149°C.

### Exemple 100

### Acide 5-chloro-1-[(4-fluorophényl)sulfonyl]-1H-pyrrolo[3,2-b]pyridine-2-butanoïque, méthyl ester

En opérant de façon analogue à la préparation VI, au départ du composé obtenu selon la préparation V et du chlorure de 4-fluorobenzènesulfonyle, on obtient le composé attendu sous forme d'un solide jaune (rendement = 29 %).
F = 119°C.

### Exemple 101

### Acide 5-chloro-1-[(4-fluorophényl)sulfonyl]-1H-pyrrolo[3,2-b]pyridine-2-butanoïque

En opérant de façon analogue à l'exemple 2, au départ de l'ester obtenu selon l'exemple 100, on obtient le composé attendu sous forme d'un solide beige (rendement = 66 %).
F = 180 °C.

### Exemple 102

### Acide 5-chloro-1-[(3-fluorophényl)sulfonyl]-1H-pyrrolo[3,2-b]pyridine-2-butanoïque, méthyl ester

En opérant de façon analogue à l'exemple 76, au départ du chlorure de 3-fluorobenzènesulfonyle, on obtient le composé attendu sous forme d'un solide brun (rendement = 76 %).
F = 104 °C.

### Exemple 103

### Acide 5-chloro-1-[(3-fluorophényl)sulfonyl]-1H-pyrrolo[3,2-b]pyridine-2-butanoïque

En opérant de façon analogue à l'exemple 2, au départ de l'ester obtenu selon l'exemple 102, on obtient le composé attendu sous forme d'un solide beige (rendement = 75 %).
F = 163°C.

### Exemple 104

### Acide 1-[(1-acétyl-2,3-dihydro-1H-indol-5-yl)sulfonyl]-5-chloro-1H-pyrrolo[3,2-b]pyridine-2-butanoïque, méthyl ester

En opérant de façon analogue à l'exemple 76, au départ du chlorure de 1-acétyl-2,3-dihydro-1*H*-indole-5-sulfonyle, on obtient le composé attendu sous forme d'un solide beige (rendement = 91 %).
F = 123°C.

### Exemple 105

### Acide 1-[(1-acétyl-2,3-dihydro-1H-indol-5-yl)sulfonyl]-5-chloro-1H-pyrrolo[3,2-b]pyridine-2-butanoïque

En opérant de façon analogue à l'exemple 2, au départ de l'ester obtenu selon l'exemple 104, on obtient le composé attendu sous forme d'un solide beige (rendement = 54 %).
F = 226°C.

### Exemple 106

### Acide 5-chloro-1-[(6-chloroimidazo[2,1-b]thiazol-5-yl)sulfonyl]-1H-pyrrolo[3,2-b]pyridine-2-butanoïque, méthyl ester

En opérant de façon analogue à l'exemple 76, au départ du chlorure de 6-chloroimidazo[2,1-*b*]thiazole-5-sulfonyle, on obtient le composé attendu sous forme d'une huile incolore (rendement = 70 %).
¹H RMN (250 MHz, DMSOd₆) δ : 8,55 (s, 1H) ; 8,43 (d, 1H) ; 7,95 (m, 5H) ; 7,39 (d, 1H) ; 6,78 (s, 1H) ; 3,58 (s, 3H) ; 3,07 (t, 2H) ; 2,44 (t, 2H) ; 1,97 (m, 2H).

### Exemple 107

### Acide 5-chloro-1-[(6-chloroimidazo[2,1-b]thiazol-5-yl)sulfonyl]-1H-pyrrolo-[3,2-b]pyridine-2-butanoïque

En opérant de façon analogue à l'exemple 2, au départ de l'ester obtenu selon l'exemple 106, on obtient le composé attendu sous forme d'un solide blanc (rendement = 99%).
F = 186°C.

### Exemple 108

### Acide 5-chloro-1-[[4-(1H-pyrazol-1-yl)phényl]sulfonyl]-1H-pyrrolo[3,2-b]pyridine-2-butanoïque, méthyl ester

En opérant de façon analogue à la préparation VI, au départ du composé obtenu selon la préparation V et du chlorure de 4-(1*H*-pyrazol-1-yl)benzènesulfonyle, on obtient le composé attendu sous forme d'un solide blanc (rendement = 10 %).
F = 122-124°C.

### Exemple 109

### Acide 5-chloro-1-[[4-(1H-pyrazol-1-yl)phényl]sulfonyl]-1H-pyrrolo[3,2-b]pyridine-2-butanoïque

En opérant de façon analogue à l'exemple 2, au départ de l'ester obtenu selon l'exemple 108, on obtient le composé attendu sous forme d'un solide blanc (rendement = 58%).
F = 196-207°C.

### Exemple 110

### Acide 5-chloro-1-[[3-(trifluorométhyl)phényl]sulfonyl]-1H-pyrrolo[3,2-b]pyridine-2-butanoïque, méthyl ester

En opérant de façon analogue à la préparation VI, au départ du composé obtenu selon la préparation V et du chlorure de 3-(trifluorométhyl)benzènesulfonyle, on obtient le composé attendu sous forme d'un solide jaune (rendement = 11 %).
F = 97°C.

### Exemple 111

### Acide 5-chloro-1-[[3-(trifluorométhyl)phényl]sulfonyl]-1H-pyrrolo[3,2-b]pyridine-2-butanoïque

En opérant de façon analogue à l'exemple 2, au départ de l'ester obtenu selon l'exemple 110, on obtient le composé attendu sous forme d'un solide jaune (rendement = 41 %).
F = 188°C.

### Exemple 112

### Acide 5-chloro-1-[[4-(trifluorométhyl)phényl]sulfonyl]-1H-pyrrolo[3,2-b]pyridine-2-butanoïque, méthyl ester

En opérant de façon analogue à la préparation VI, au départ du composé obtenu selon la préparation V et du chlorure de 4-(trifluorométhyl)benzènesulfonyle, on obtient le composé attendu sous forme d'un solide beige (rendement = 27 %).
F = 99°C.

### Exemple 113

### Acide 5-chloro-1-[[4-(trifluorométhyl)phényl]sulfonyl]-1H-pyrrolo[3,2-b]pyridine-2-butanoïque

En opérant de façon analogue à l'exemple 2, au départ de l'ester obtenu selon l'exemple 112, on obtient le composé attendu sous forme d'un solide blanc (rendement = 27 %).
F = 185°C.

### Exemple 114

### Acide 5-chloro-1-[[4-(5-oxazolyl)phényl]sulfonyl]-1H-pyrrolo[3,2-b]pyridine-2-butanoïque, méthyl ester

En opérant de façon analogue à l'exemple 76, au départ du chlorure de 4-(5-oxazolyl)benzènesulfonyle, on obtient le composé attendu sous forme d'une huile incolore (rendement = 50 %).
¹H RMN (250 MHz, DMSOd₆) δ : 8,55 (s, 1H) ; 8,43 (d, 1H) ; 7,95 (m, 5H) ; 7,39 (d, 1H) ; 6,78 (s, 1H) ; 3,58 (s, 3H) ; 3,07 (t, 2H) ; 2,44 (t, 2H) ; 1,97 (m, 2H).

### Exemple 115

### Acide 5-chloro-1-[[4-(5-oxazolyl)phényl]sulfonyl]-1H-pyrrolo[3,2-b]pyridine-2-butanoïque

En opérant de façon analogue à l'exemple 2, au départ de l'ester obtenu selon l'exemple 114, on obtient le composé attendu sous forme d'un solide blanc (rendement = 27 %).
F = 169-176°C.

### Exemple 116

### Acide 1-[[3,5-bis(trifluorométhyl)phényl]sulfonyl]-5-chloro-1H-pyrrolo[3,2-b]pyridine-2-butanoïque, méthyl ester

En opérant de façon analogue à l'exemple 76, au départ du chlorure de 3,5-bis(trifluorométhyl)benzènesulfonyle, on obtient le composé attendu sous forme d'un solide blanc (rendement = 62 %).
F = 134-144°C.

### Exemple 117

### Acide 1-[[3,5-bis(trifluorométhyl)phényl]sulfonyl]-5-chloro-1H-pyrrolo[3,2-b]pyridine-2-butanoïque

En opérant de façon analogue à l'exemple 2, au départ de l'ester obtenu selon l'exemple 116, on obtient le composé attendu sous forme d'un solide beige (rendement = 6 %).
F = 155-164°C.

### Exemple 118

### Acide 5-chloro-1-[(4-chloro-3-méthylphényl)sulfonyl]-1H-pyrrolo[3,2-b]pyridine-2-butanoïque, méthyl ester

En opérant de façon analogue à l'exemple 76, au départ du chlorure de 4-chloro-3-méthylbenzènesulfonyle, on obtient le composé attendu sous forme d'un solide blanc (rendement = 78 %).
F = 111-114°C

### Exemple 119

### Acide 5-chloro-1-[(4-chloro-3-méthylphényl)sulfonyl]-1H-pyrrolo[3,2-b]-pyridine-2-butanoïque

En opérant de façon analogue à l'exemple 2, au départ de l'ester obtenu selon l'exemple 118, on obtient le composé attendu sous forme d'un solide blanc (rendement = 42 %).
F = 175-183°C.

### Exemple 120

### Acide 1-([1,1'-biphényl]-4-ylsulfonyl)-5-chloro-1H-pyrrolo[3,2-b]pyridine-2-butanoïque, méthyl ester

En opérant de façon analogue à l'exemple 76, au départ du chlorure de [1,1'-biphényl]-4-sulfonyle, on obtient le composé attendu sous forme d'un solide blanc (rendement = 85 %).
F = 122-124°C.

### Exemple 121

### Acide 1-([1,1'-biphényl]-4-ylsulfonyl)-5-chloro-1H-pyrrolo[3,2-b]pyridine-2-butanoïque

En opérant de façon analogue à l'exemple 2, au départ de l'ester obtenu selon l'exemple 120, on obtient le composé attendu sous forme d'un solide blanc (rendement = 55 %).
F = 186-190°C.

### Exemple 122

### Acide 5-chloro-1-[(3,4-difluorophényl)sulfonyl]-1H-pyrrolo[3,2-b]pyridine-2-butanoïque, méthyl ester

En opérant de façon analogue à l'exemple 76, au départ du chlorure de 3,4-difluorobenzènesulfonyle, on obtient le composé attendu sous forme d'un solide blanc (rendement = 61 %).
F = 96-98 °C.

### Exemple 123

### Acide 5-chloro-1-[(3,4-difluorophényl)sulfonyl]-1H-pyrrolo[3,2-b]pyridine-2-butanoïque

En opérant de façon analogue à l'exemple 2, au départ de l'ester obtenu selon l'exemple 122, on obtient le composé attendu sous forme d'un solide beige (rendement = 59 %).
F = 180-190°C.

### Exemple 124

### Acide 5-chloro-1-[3-(trifluorométhoxy)phénylsulfonyl]-1H-pyrrolo[3,2-b]pyridine-2-butanoïque, méthyl ester

En opérant de façon analogue à l'exemple 76, au départ du chlorure de 3-(trifluorométhoxy)benzènesulfonyle, on obtient le composé attendu sous forme d'une huile brune (rendement = 84 %).
¹H RMN (300 MHz, DMSOd₆) δ : 8,44 (d, 1H) ; 7,89 (m, 2H) ; 7,77 (m, 2H) ; 7,39 (d, 1H) ; 6,80 (s, 1H) ; 3,58 (s, 3H) ; 3,06 (t, 2H) ; 2,44 (t, 2H) ; 1,96 (quin, 2H).

### Exemple 125

### Acide 5-chloro-1-[3-(trifluorométhoxy)phénylsulfonyl]-1H-pyrrolo[3,2-b]-pyridine-2-butanoïque

En opérant de façon analogue à l'exemple 2, au départ de l'ester obtenu selon l'exemple 124, on obtient le composé attendu sous forme d'un solide beige (rendement = 37 %).
F = 176°C.

### Exemple 126

### Acide 1-(1,2,3-benzothiadiazol-5-ylsulfonyl)-5-chloro-1H-pyrrolo[3,2-b]pyridine-2-butanoïque, méthyl ester

En opérant de façon analogue à la préparation VI, au départ du composé obtenu selon la préparation V et du chlorure de 1,2,3-benzothiadiazole-5-sulfonyle, on obtient le composé attendu sous forme d'un solide beige (rendement = 9 %).
F = 219°C.

### Exemple 127

### Acide 1-(1,2,3-benzothiadiazol-5-ylsulfonyl)-5-chloro-1H-pyrrolo[3,2-b]pyridine-2-butanoïque

En opérant de façon analogue à l'exemple 2, au départ de l'ester obtenu selon l'exemple 126, on obtient le composé attendu sous forme d'un solide blanc (rendement = 58 %).
F = 227°C.

### Exemple 128

### Acide 5-chloro-1-[4-(trifluorométhoxy)phénylsulfonyl]-1H-pyrrolo[3,2-b]pyridine-2-butanoïque, méthyl ester

En opérant de façon analogue à l'exemple 76, au départ du chlorure de 4-(trifluorométhoxy)benzènesulfonyle, on obtient le composé attendu sous forme d'un solide blanc (rendement = 52 %).
F = 109°C.

### Exemple 129

### Acide 5-chloro-1-[4-(trifluorométhoxy)phénylsulfonyl]-1H-pyrrolo[3,2-b]-pyridine-2-butanoïque

En opérant de façon analogue à l'exemple 2, au départ de l'ester obtenu selon l'exemple 128, on obtient le composé attendu sous forme d'un solide blanc (rendement = 88 %).
F = 168°C.

### Exemple 130

### Acide 5-chloro-1-[(3-chlorophényl)sulfonyl]-1H-pyrrolo[3,2-b]pyridine-2-butanoïque, méthyl ester

En opérant de façon analogue à l'exemple 76, au départ du chlorure de 3-chlorobenzènesulfonyle, on obtient le composé attendu sous forme d'un solide blanc (rendement = 14 %).
F = 107°C.

### Exemple 131

### Acide 5-chloro-1-[(3-chlorophényl)sulfonyl]-1H-pyrrolo[3,2-b]pyridine-2-butanoïque

En opérant de façon analogue à l'exemple 2, au départ de l'ester obtenu selon l'exemple 130, on obtient le composé attendu sous forme d'un solide beige (rendement = 83 %).
F = 174°C.

### Exemple 132

### Acide 5-chloro-1-[(4-chlorophényl)sulfonyl]-1H-pyrrolo[3,2-b]pyridine-2-butanoique, méthyl ester

En opérant de façon analogue à l'exemple 76, au départ du chlorure de 4-chlorobenzènesulfonyle, on obtient le composé attendu sous forme d'un solide beige (rendement = 35 %).
F = 108°C.

### Exemple 133

### Acide 5-chloro-1-[(4-chlorophényl)sulfonyl]-1H-pyrrolo[3,2-b]pyridine-2-butanoïque

En opérant de façon analogue à l'exemple 2, au départ de l'ester obtenu selon l'exemple 132, on obtient le composé attendu sous forme d'un solide beige (rendement = 81 %).
F = 174°C.

### Exemple 134

### Acide 5-chloro-1-[(3-méthoxyphényl)sulfonyl]-1H-pyrrolo[3,2-b]pyridine-2-butanoïque, méthyl ester

En opérant de façon analogue à l'exemple 76, au départ du chlorure de 3-méthoxybenzènesulfonyle, on obtient le composé attendu sous forme d'un solide jaune (rendement = 45 %).
F = 90°C.

### Exemple 135

### Acide 5-chloro-1-[(3-méthoxyphényl)sulfonyl]-1H-pyrrolo[3,2-b]pyridine-2-butanoïque

En opérant de façon analogue à l'exemple 2, au départ de l'ester obtenu selon l'exemple 134, on obtient le composé attendu sous forme d'un solide beige (rendement = 94 %).
F = 139°C.

### Exemple 136

### Acide 5-chloro-1-[(4-méthoxyphényl)sulfonyl]-1H-pyrrolo[3,2-b]pyridine-2-butanoïque, méthyl ester

En opérant de façon analogue à l'exemple 76, au départ du chlorure de 4-méthoxybenzènesulfonyle, on obtient le composé attendu sous forme d'un solide beige (rendement = 66 %).
F = 96°C.

### Exemple 137

### Acide 5-chloro-1-[(4-méthoxyphényl)sulfonyl]-1H-pyrrolo[3,2-b]pyridine-2-butanoïque

En opérant de façon analogue à l'exemple 2, au départ de l'ester obtenu selon l'exemple 136, on obtient le composé attendu sous forme d'un solide blanc (rendement = 78 %).
F = 189°C.

### Exemple 138

### Acide 5-chloro-1-[(4-méthylphényl)sulfonyl]-1H-pyrrolo[3,2-b]pyridine-2-butanoïque, méthyl ester

En opérant de façon analogue à l'exemple 76, au départ du chlorure de 4-méthylbenzènesulfonyle, on obtient le composé attendu sous forme d'un solide gris (rendement = 37 %).
F = 106°C.

### Exemple 139

### Acide 5-chloro-1-[(4-méthylphényl)sulfonyl]-1H-pyrrolo[3,2-b]pyridine-2-butanoïque

En opérant de façon analogue à l'exemple 2, au départ de l'ester obtenu selon l'exemple 138, on obtient le composé attendu sous forme d'un solide blanc (rendement = 87 %).
F = 172°C.

### Exemple 140

### Acide 5-chloro-1-[[4-(1-méthyléthyl)phényl]sulfonyl]-1H-pyrrolo[3,2-b]pyridine-2-butanoïque, méthyl ester

En opérant de façon analogue à l'exemple 76, au départ du chlorure de 4-(1-méthyléthyl)benzènesulfonyle, on obtient le composé attendu sous forme d'un solide blanc (rendement = 51 %).
F = 75°C.

### Exemple 141

### Acide 5-chloro-1-[[4-(1-méthyléthyl)phényl]sulfonyl]-1H-pyrrolo[3,2-b]pyridine-2-butanoïque

En opérant de façon analogue à l'exemple 2, au départ de l'ester obtenu selon l'exemple 140, on obtient le composé attendu sous forme d'un solide blanc (rendement = 88 %).
F = 155°C.

### Exemple 142

### Acide 5-chloro-1-[[3-(2-méthyl-4-pyrimidinyl)phényl]sulfonyl]-1H-pyrrolo[3,2-b]pyridine-2-butanoïque, méthyl ester

En opérant de façon analogue à l'exemple 76, au départ du chlorure de 3-(2-méthyl-4-pyrimidinyl)benzènesulfonyle, on obtient le composé attendu sous forme d'un solide jaune (rendement = 78 %).
F = 140-144°C.

### Exemple 143

### Acide 5-chloro-1-[[3-(2-méthyl-4-pyrimidinyl)phényl]sulfonyl]-1H-pyrrolo[3,2-b]pyridine-2-butanoïque

En opérant de façon analogue à l'exemple 2, au départ de l'ester obtenu selon l'exemple 142, on obtient le composé attendu sous forme d'un solide blanc (rendement = 38 %).
F = 178-188°C.

### Exemple 144

### Acide 1-(1,2-benzisoxazol-5-ylsulfonyl)-5-chloro-1H-pyrrolo[3,2-b]pyridine-2-butanoïque, méthyl ester

En opérant de façon analogue à la préparation VI, au départ du composé obtenu selon la préparation V et du chlorure de 1,2-benzisoxazole-5-sulfonyle, on obtient le composé attendu sous forme d'un solide beige (rendement = 7 %).
¹H RMN (250 MHz, DMSOd₆) δ : 8,42 (d, 1H) ; 8,32 (d, 1H) ; 7,98 (dd, 1H) ; 7,36 (d, 1H) ; 7,10 (d, 1H) ; 6,75 (s, 1H) ; 3,58 (s, 3H) ; 3,05 (t, 2H) ; 2,45 (t, 2H) ; 1,95 (quin, 2H).

### Exemple 145

### Acide 1-(1,2-benzisoxazol-5-ylsulfonyl)-5-chloro-1H-pyrrolo[3,2-b]pyridine-2-butanoïque

En opérant de façon analogue à l'exemple 2, au départ de l'ester obtenu selon l'exemple 144, on obtient le composé attendu sous forme d'un solide jaune (rendement = 33 %).
F = 226°C.

### Exemple 146

### Acide 5-chloro-1-[2-(trifluorométhoxy)phénylsulfonyl]-1H-pyrrolo[3,2-b]pyridine-2-butanoïque, méthyl ester

En opérant de façon analogue à la préparation VI, au départ du composé obtenu selon la préparation V et du chlorure de 2-(trifluorométhoxy)benzènesulfonyle, on obtient le composé attendu sous forme d'un solide blanc pâteux.

### Exemple 147

### Acide 5-chloro-1-[2-(trifluorométhoxy)phénylsulfonyl]-1H-pyrrolo[3,2-b]-pyridine-2-butanoïque

En opérant de façon analogue à l'exemple 2, au départ de l'ester obtenu selon l'exemple 146, on obtient le composé attendu sous forme d'un solide blanc (rendement = 50 %).
F = 138°C.

### Exemple 148

### Acide 5-chloro-1-[(3,5-difluorophényl)sulfonyl]-1H-pyrrolo[3,2-b]pyridine-2butanoïque, méthyl ester

En opérant de façon analogue à l'exemple 76, au départ du chlorure de 3,5-difluorobenzènesulfonyle, on obtient le composé attendu sous forme d'un solide beige (rendement = 83 %).
F = 90°C.

### Exemple 149

### Acide 5-chloro-1-[(3,4-difluorophényl)sulfonyl]-1H-pyrrolo[3,2-b]pyridine-2-butanoïque

En opérant de façon analogue à l'exemple 2, au départ de l'ester obtenu selon l'exemple 148, on obtient le composé attendu sous forme d'un solide gris (rendement = 24 %).
¹H RMN (300 MHz, DMSOd₆) δ : 12,11 (s, 1H) ; 8,44 (d, 1H) ; 7,75 (m, 3H) ; 7,38 (d, 1H) ; 6,80 (s, 1H) ; 3,08 (t, 2H) ; 2,37 (m, 2H) ; 1,94 (m, 2H).

### Exemple 150

### Acide 5-chloro-1-[(2,5-dichlorophényl)sulfonyl]-1H-pyrrolo[3,2-b]pyridine-2-butanoïque, méthyl ester

En opérant de façon analogue à l'exemple 76, au départ du chlorure de 2,5-dichlorobenzènesulfonyle, on obtient le composé attendu sous forme d'un solide beige (rendement = 65 %).
F = 95-105°C.

### Exemple 151

### Acide 5-chloro-1-[(2,5-dichlorophényl)sulfonyl]-1H-pyrrolo[3,2-b]pyridine-2-butanoïque

En opérant de façon analogue à l'exemple 2, au départ de l'ester obtenu selon l'exemple 150, on obtient le composé attendu sous forme d'un solide gris (rendement = 43 %).
F = 86-89°C.

### Exemple 152

### Acide 5-chloro-1-[(2-chlorophényl)sulfonyl]-1H-pyrrolo[3,2-b]pyridine-2-butanoïque, méthyl ester

En opérant de façon analogue à l'exemple 76, au départ du chlorure de 2-chlorobenzènesulfonyle, on obtient le composé attendu, mis en réaction directement pour obtenir l'acide.

### Exemple 153

### Acide 5-chloro-1-[(2-chlorophényl)sulfonyl]-1H-pyrrolo[3,2-b]pyridine-2-butanoïque

En opérant de façon analogue à l'exemple 2, au départ de l'ester obtenu selon l'exemple 152, on obtient le composé attendu sous forme d'un solide beige (rendement = 48 %).
F = 139°C.

### Exemple 154

### Acide 5-chloro-1-[(2-méthylphényl)sulfonyl]-1H-pyrrolo[3,2-b]pyridine-2-butanoïque, méthyl ester

En opérant de façon analogue à l'exemple 76, au départ du chlorure de 2-méthylbenzènesulfonyle, on obtient le composé attendu, mis en réaction directement pour obtenir l'acide correspondant.

### Exemple 155

### Acide 5-chloro-1-[(2-méthylphényl)sulfonyl]-1H-pyrrolo[3,2-b]pyridine-2-butanoïque

En opérant de façon analogue à l'exemple 2, au départ de l'ester obtenu selon l'exemple 154, on obtient le composé attendu sous forme d'un solide blanc (rendement = 66 %).
F = 161°C.

### Exemple 156

### Acide 5-chloro-1-[(3-méthylphényl)sulfonyl]-1H-pyrrolo[3,2-b]pyridine-2-butanoïque, méthyl ester

En opérant de façon analogue à l'exemple 76, au départ du chlorure de 3-méthylbenzènesulfonyle, on obtient le composé attendu, mis en réaction directement pour obtenir l'acide correspondant.

### Exemple 157

### Acide 5-chloro-1-[(3-méthylphényl)sulfonyl]-1H-pyrrolo[3,2-b]pyridine-2-butanoïque

En opérant de façon analogue à l'exemple 2, au départ de l'ester obtenu selon l'exemple 156, on obtient le composé attendu sous forme d'un solide blanc (rendement = 38 %).
F = 165°C.

### Exemple 158

### Acide 5-chloro-1-[(2,6-difluorophényl)sulfonyl]-1H-pyrrolo[3,2-b]pyridine-2-butanoïque, méthyl ester

En opérant de façon analogue à l'exemple 76, au départ du chlorure de 2,6-difluorobenzènesulfonyle, on obtient le composé attendu sous forme d'un solide blanc (rendement = 76 %).
F = 107-109°C.

### Exemple 159

### Acide 5-chloro-1-[(2,6-difluorophényl)sulfonyl]-1H-pyrrolo[3,2-b]pyridine-2-butanoïque

En opérant de façon analogue à l'exemple 2, au départ de l'ester obtenu selon l'exemple 158, on obtient le composé attendu sous forme d'un solide blanc (rendement = 38 %).
F = 192-195°C.

### Exemple 160

### Acide 5-chloro-1-[(2,4,6-trifluorophényl)sulfonyl]-1H-pyrrolo[3,2-b]pyridine-2-butanoïque, méthyl ester

En opérant de façon analogue à l'exemple 76, au départ du chlorure de 2,4,6-trifluorobenzènesulfonyle, on obtient le composé attendu sous forme d'un solide blanc (rendement = 71 %).
¹H RMN (250 MHz, DMSOd₆) δ : 8,20 (d, 1H) ; 7,56 (m, 2H) ; 7,41 (d, 1H) ; 6,84 (s, 1H) ; 3,57 (s, 3H) ; 2,94 (t, 2H) ; 2,41 (t, 2H) ; 1,94 (quin, 2H).

### Exemple 161

### Acide 5-chloro-1-[(2,4,6-trifluorophényl)sulfonyl]-1H-pyrrolo[3,2-b]pyridine-2-butanoïque

En opérant de façon analogue à l'exemple 2, au départ de l'ester obtenu selon l'exemple 160, on obtient le composé attendu sous forme d'un solide beige (rendement = 27 %).
F = 135-150°C.

### Exemple 162

### Acide 5-chloro-1-[(2,5-diméthylphényl)sulfonyl]-1H-pyrrolo[3,2-b]pyridine-2-butanoïque, méthyl ester

En opérant de façon analogue à l'exemple 76, au départ du chlorure de 2,5-diméthylbenzènesulfonyle, on obtient le composé attendu sous forme d'un solide blanc (rendement = 87 %).
F = 92-103 °C.

### Exemple 163

### Acide 5-chloro-1-[(2,5-diméthylphényl)sulfonyl]-1H-pyrrolo[3,2-b]pyridine-2-butanoïque

En opérant de façon analogue à l'exemple 2, au départ de l'ester obtenu selon l'exemple 162, on obtient le composé attendu sous forme d'un solide jaune (rendement = 72 %).
F = 106-117 °C.

### Exemple 164

### Acide 5-chloro-1-[(3,5-diméthoxyphényl)sulfonyl]-1H-pyrrolo[3,2-b]pyridine-2-butanoïque, méthyl ester

En opérant de façon analogue à l'exemple 76, au départ du chlorure de 3,5-diméthoxybenzènesulfonyle, on obtient le composé attendu sous forme d'un solide beige (rendement = 75 %).
F = 109-111°C.

### Exemple 165

### Acide 5-chloro-1-[(3,5-diméthoxyphényl)sulfonyl]-1H-pyrrolo[3,2-b]pyridine-2-butanoïque

En opérant de façon analogue à l'exemple 2, au départ de l'ester obtenu selon l'exemple 164, on obtient le composé attendu sous forme d'un solide blanc (rendement = 27 %).
F = 136-138°C.

### Exemple 166

### Acide 5-chloro-1-[4-(1-méthyléthoxy)phénylsulfonyl]-1H-pyrrolo[3,2-b]pyridine-2-butanoïque, méthyl ester

En opérant de façon analogue à l'exemple 76, au départ du chlorure de 4-(1-méthyléthoxy)benzènesulfonyle, on obtient le composé attendu sous forme d'une huile incolore (rendement = 88 %).
¹H RMN (250 MHz, DMSOd₆) δ : 8,41 (d, 1H) ; 7,77 (d, 2H) ; 7,38 (d, 1H) ; 7,05 (d, 2H) ; 6,73 (s, 1H) ; 4,70 (hep, 1H) ; 3,59 (s, 3H) ; 3,04 (t, 2H) ; 2,43 (t, 2H) ; 1,95 (quin, 2H) ; 1,24 (d, 6H).

### Exemple 167

### Acide 5-chloro-1-[4-(1-méthyléthoxy)phénylsulfonyl]-1H-pyrrolo[3,2-b]-pyridine-2-butanoïque

En opérant de façon analogue à l'exemple 2, au départ de l'ester obtenu selon l'exemple 166, on obtient le composé attendu sous forme d'un solide blanc (rendement = 97 %).
F = 171°C.

### Exemple 168

### Acide 5-chloro-1-[(2-méthoxyphényl)sulfonyl]-1H-pyrrolo[3,2-b]pyridine-2-butanoïque, méthyl ester

En opérant de façon analogue à la préparation VI, au départ du composé obtenu selon la préparation V et du chlorure de 2-méthoxybenzènesulfonyle, on obtient le composé attendu sous forme d'un solide beige (rendement = 41 %).
F = 115°C.

### Exemple 169

### Acide 5-chloro-1-[(2-méthoxyphényl)sulfonyl]-1H-pyrrolo[3,2-b]pyridine-2-butanoïque

En opérant de façon analogue à l'exemple 2, au départ de l'ester obtenu selon l'exemple 168, on obtient le composé attendu sous forme d'un solide blanc (rendement = 89 %).
F = 197°C.

### Exemple 170

### Acide 5-chloro-1-[(2-chloro-3-méthylphényl)sulfonyl]-1H-pyrrolo[3,2-b]pyridine-2-butanoïque, méthyl ester

En opérant de façon analogue à l'exemple 76, au départ du chlorure de 2-chloro-3-méthylbenzènesulfonyle, on obtient le composé attendu sous forme d'une huile verte (rendement = 57 %).
¹H RMN (250 MHz, DMSOd₆) δ : 8,18 (d, 1H) ; 7,87 (d, 1H) ; 7,79 (d, 1H) ; 7,55 (t, 1H) ; 7,34 (d, 1H) ; 6;81 (s, 1H) ; 3,55 (s, 3H) ; 2,86 (t, 2H) ; 2,35 (m, 5H) ; 1,85 (quin, 2H).

### Exemple 171

### Acide 5-chloro-1-[(2-chloro-3-méthylphényl)sulfonyl]-1H-pyrrolo[3,2-b]pyridine-2-butanoïque

En opérant de façon analogue à l'exemple 2, au départ de l'ester obtenu selon l'exemple 170, on obtient le composé attendu sous forme d'un solide blanc (rendement = 16 %).
F = 171-174°C.

### Exemple 172

### Acide 5-chloro-1-[(2,4-difluorophényl)sulfonyl]-1H-pyrrolo[3,2-b]pyridine-2-butanoïque, méthyl ester

En opérant de façon analogue à l'exemple 76, au départ du chlorure de 2,4-difluorobenzènesulfonyle, on obtient le composé attendu sous forme d'une huile jaune (rendement = 82 %).
¹H RMN (250 MHz, DMSOd₆) δ : 8,26 (d, 1H) ; 8,17 (m, 1H) ; 7,59 (m, 1H) ; 7,40 (m, 2H) ; 6,81 (s, 1H) ; 3,57 (s, 3H) ; 2,94 (t, 2H) ; 2,40 (t, 2H) ; 1,89 (quin, 2H).

### Exemple 173

### Acide 5-chloro-1-[(2,4-difluorophényl)sulfonyl]-1H-pyrrolo[3,2-b]pyridine-2-butanoïque

En opérant de façon analogue à l'exemple 2, au départ de l'ester obtenu selon l'exemple 172, on obtient le composé attendu sous forme d'un solide blanc (rendement = 8 %).
F = 172-177°C.

### Exemple 174

### Acide 5-chloro-1-[(2-chloro-4-méthoxyphényl)sulfonyl]-1H-pyrrolo[3,2-b]pyridine-2-butanoïque, méthyl ester

En opérant de façon analogue à l'exemple 76, au départ du chlorure de 2-chloro-4-méthoxybenzènesulfonyle, on obtient le composé attendu sous forme d'un solide blanc (rendement = 75 %).
F = 116-118°C.

### Exemple 175

### Acide 5-chloro-1-[(2-chloro-4-méthoxyphényl)sulfonyl]-1H-pyrrolo[3,2-b]pyridine-2-butanoïque

En opérant de façon analogue à l'exemple 2, au départ de l'ester obtenu selon l'exemple 174, on obtient le composé attendu sous forme d'un solide blanc (rendement = 78 %).
F = 154-156°C.

### Exemple 176

### Acide 5-chloro-1-[[4-(4-thiazolyl)phényl]sulfonyl]-1H-pyrrolo[3,2-b]pyridine-2-butanoïque, méthyl ester

En opérant de façon analogue à l'exemple 76, au départ du chlorure de 4-(4-thiazolyl)benzènesulfonyle, on obtient le composé attendu sous forme d'une pâte blanche (rendement = 78 %).
¹H RMN (300 MHz, DMSOd₆) δ : 9,23 (d, 1H) ; 8,43 (m, 2H) ; 8,18 (d, 2H) ; 7,95 (d, 2H) ; 7,40 (d, 1H) ; 6,77 (s, 1H) ; 3,58 (s, 3H) ; 3,09 (t, 2H) ; 2,45 (t, 2H) ; 1,98 (quin, 2H).

### Exemple 177

### Acide 5-chloro-1-[[4-(4-thiazolyl)phényl]sulfonyl]-1H-pyrrolo[3,2-b]pyridine-2-butanoïque

En opérant de façon analogue à l'exemple 2, au départ de l'ester obtenu selon l'exemple 176, on obtient le composé attendu sous forme d'un solide blanc (rendement = 62 %).
F = 211-217°C.

### Exemple 178

### Acide 5-chloro-1-[[4-(1,1-diméthyléthyl)phényl]sulfonyl]-1H-pyrrolo[3,2-b]pyridine-2-butanoïque, méthyl ester

En opérant de façon analogue à l'exemple 76, au départ du chlorure de 4-(1,1-diméthyléthyl)benzènesulfonyle, on obtient le composé attendu sous forme d'un solide blanc (rendement = 98%).
F = 107°C.

### Exemple 179

### Acide 5-chloro-1-[[4-(1,1-diméthyléthyl)phényl]sulfonyl]-1H-pyrrolo[3,2-b]pyridine-2-butanoïque

En opérant de façon analogue à l'exemple 2, au départ de l'ester obtenu selon l'exemple 178, on obtient le composé attendu sous forme d'un solide blanc (rendement = 89 %).
F = 168°C.

### Exemple 180

### Acide 5-chloro-1-[(2,3-dihydro-1,4-benzodioxin-6-yl)sulfonyl]-1H-pyrrolo[3,2-b]pyridine-2-butanoïque, méthyl ester

En opérant de façon analogue à la préparation VI, au départ du composé obtenu selon la préparation V et du chlorure de 2,3-dihydro-1,4-benzodioxine-6-sulfonyle, on obtient le composé attendu sous forme d'une huile brune (rendement = 23 %).
¹H RMN (300 MHz, DMSOd₆) δ : 8,41 (d, 1H) ; 7,35 (m, 3H) ; 7,03 (d, 1H) ; 6,74 (s, 1H) ; 4,28 (m, 4H) ; 3,59 (s, 3H) ; 3,05 (t, 2H) ; 2,44 (t, 2H) ; 1,94 (quin, 2H).

### Exemple 181

### Acide 5-chloro-1-[(2,3-dihydro-1,4-benzodioxin-6-yl)sulfonyl]-1H-pyrrolo[3,2-b]pyridine-2-butanoïque

En opérant de façon analogue à l'exemple 2, au départ de l'ester obtenu selon l'exemple 180, on obtient le composé attendu sous forme d'un solide blanc (rendement = 95%).
F = 166°C.

### Exemple 182

### Acide 5-chloro-1-[(2-pyridinyl)sulfonyl]-1H-pyrrolo[3,2-b]pyridine-2-butanoïque, méthyl ester

En opérant de façon analogue à l'exemple 76, au départ du chlorure de 2-pyridinesulfonyle, on obtient le composé attendu sous forme d'une huile incolore (rendement = 50 %).
¹H RMN (250 MHz, DMSOd₆) δ : 8,61 (d, 1H) ; 8,24 (m, 3H) ; 7,74 (m, 1H) ; 7,34 (d, 1H) ; 6,77 (s, 1H) ; 3,58 (s, 3H) ; 3,08 (t, 2H) ; 2,41 (t, 2H) ; 1,94 (quin, 2H).

### Exemple 183

### Acide 5-chloro-1-[(2-pyridinyl)sulfonyl]-1H-pyrrolo[3,2-b]pyridine-2-butanoïque

En opérant de façon analogue à l'exemple 2, au départ de l'ester obtenu selon l'exemple 182, on obtient le composé attendu sous forme d'un solide blanc (rendement = 30 %).
F = 187°C.

### Exemple 184

### Acide 5-chloro-1-[[3-(1-méthyléthyl)phényl]sulfonyl]-1H-pyrrolo[3,2-b]pyridine-2-butanoïque, méthyl ester

En opérant de façon analogue à l'exemple 76, au départ du chlorure de 3-(1-méthyléthyl)benzènesulfonyle, on obtient le composé attendu sous forme d'un solide brun (rendement = 73 %).
F = 92°C.

### Exemple 185

### Acide 5-chloro-1-[[3-(1-méthyléthyl)phényl]sulfonyl]-1H-pyrrolo[3,2-b]pyridine-2-butanoïque

En opérant de façon analogue à l'exemple 2, au départ de l'ester obtenu selon l'exemple 184, on obtient le composé attendu sous forme d'un solide blanc (rendement = 37 %).
F = 119°C.

### Exemple 186

### Acide 5-chloro-1-[(4-méthyl-1-naphthalènyl)sulfonyl]-1H-pyrrolo[3,2-b]pyridine-2-butanoïque, méthyl ester

En opérant de façon analogue à l'exemple 76, au départ du chlorure de 4-méthyl-1-naphthalènesulfonyle, on obtient le composé attendu sous forme d'un solide blanc (rendement = 85 %).
F = 118-120°C.

### Exemple 187

### Acide 5-chloro-1-[(4-méthyl-1-naphthalènyl)sulfonyl]-1H-pyrrolo[3,2-b]pyridine-2-butanoïque

En opérant de façon analogue à l'exemple 2, au départ de l'ester obtenu selon l'exemple 186, on obtient le composé attendu sous forme d'un solide beige (rendement = 13 %).
F = 199-204°C.

### Exemple 188

### Acide 1-[(3,5-diméthylphényl)sulfonyl]-5-(trifluorométhyl)-1H-pyrrolo[3,2-b]pyridine-2-butanoïque, méthyl ester

En opérant de façon analogue à l'exemple 52, au départ du composé obtenu selon la préparation XXII et du chlorure de 3,5-diméthylbenzènesulfonyle, on obtient le composé attendu sous forme d'un solide blanc (rendement = 51 %).
F = 156-160°C.

### Exemple 189

### Acide 1-[(3,5-diméthylphényl)sulfonyl]-5-(trifluorométhyl)-1H-pyrrolo[3,2-b]pyridine-2-butanoïque

En opérant de façon analogue à l'exemple 2, au départ de l'ester obtenu selon l'exemple 188, on obtient le composé attendu sous forme d'un solide blanc (rendement = 33 %).
F = 227-231°C.

### Exemple 190

### Acide 5-chloro-1-[(2,4-dichlorophényl)sulfonyl]-1H-pyrrolo[3,2-b]pyridine-2-butanoïque, méthyl ester

En opérant de façon analogue à l'exemple 76, au départ du chlorure de 2,4-dichlorobenzènesulfonyle, on obtient le composé attendu sous forme d'un solide blanc (rendement = 22 %).
F = 100-101°C.

### Exemple 191

### Acide 5-chloro-1-[(2,4-dichlorophényl)sulfonyl]-1H-pyrrolo[3,2-b]pyridine-2-butanoïque

En opérant de façon analogue à l'exemple 2, au départ de l'ester obtenu selon l'exemple 190, on obtient le composé attendu sous forme d'un solide blanc (rendement = 96 %).
F = 154-155°C.

### Exemple 192

### Acide 5-chloro-1-[(2,3-dichlorophényl)sulfonyl]-1H-pyrrolo[3,2-b]pyridine-2-butanoïque, méthyl ester

En opérant de façon analogue à l'exemple 76, au départ du chlorure de 2,3-dichlorobenzènesulfonyle, on obtient le composé attendu sous forme d'une huile rose (rendement = 40 %).
¹H RMN (250 MHz, CDCl₃) δ : 8,18 (d, 1H) ; 7,83 (d, 1H) ; 7,74 (d, 1H) ; 7,41 (t, 1H) ; 7,18 (d, 1H) ; 6,61 (s, 1H) ; 3,66 (s, 3H) ; 2,90 (t, 2H) ; 2,39 (t, 2H) ; 2,03 (quin, 2H).

### Exemple 193

### Acide 5-chloro-1-[(2,3-dichlorophényl)sulfonyl]-1H-pyrrolo[3,2-b]pyridine-2-butanoïque

En opérant de façon analogue à l'exemple 2, au départ de l'ester obtenu selon l'exemple 192, on obtient le composé attendu sous forme d'un solide blanc (rendement = 79 %).
F = 173-174°C.

### Exemple 194

### Acide 5-chloro-1-[(3-chloro-2-méthylphényl)sulfonyl]-1H-pyrrolo[3,2-b]pyridine-2-butanoïque, méthyl ester

En opérant de façon analogue à l'exemple 76, au départ du chlorure de 3-chloro-2-méthylbenzènesulfonyle, on obtient le composé attendu sous forme d'une pâte incolore (rendement = 84 %).
¹H RMN (250 MHz, CDCl3) δ : 8,21 (d, 1H) ; 7,61 (d, 1H) ; 6,98 (m, 2H) ; 6,95 (d, 1H) ; 6,65 (s, 1H) ; 3,66 (s, 3H) ; 2,87 (t, 2H) ; 2,55 (s, 3H) ; 2,38 (t, 2H) ; 2,02 (quin, 2H).

### Exemple 195

### Acide 5-chloro-1-[(3-chloro-2-méthylphényl)sulfonyl]-1H-pyrrolo[3,2-b]-pyridine-2-butanoïque

En opérant de façon analogue à l'exemple 2, au départ de l'ester obtenu selon l'exemple 194, on obtient le composé attendu sous forme d'un solide blanc (rendement = 93 %).
F = 127-128°C.

### Exemple 196

### Acide 5-chloro-1-[(4-méthoxy-2-méthylphényl)sulfonyl]-1H-pyrrolo[3,2-b]pyridine-2-butanoïque, méthyl ester

En opérant de façon analogue à l'exemple 76, au départ du chlorure de 4-méthoxy-2-méthylbenzènesulfonyle, on obtient le composé attendu sous forme d'une pâte blanche (rendement = 38 %).
¹H RMN (250 MHz, DMSOd₆) δ : 8,23 (d, 1H) ; 7,71 (d, 1H) ; 7,35 (d, 1H) ; 6,98 (m, 2H) ; 6,78 (s, 1H) ; 3,82 (s, 3H) ; 3,55 (s, 3H) ; 2,81 (t, 2H) ; 2,33 (t, 2H) ; 2,22 (s, 3H) ; 1,83 (quin, 2H)).

### Exemple 197

### Acide 5-chloro-1-[(4-méthoxy-2-méthylphényl)sulfonyl]-1H-pyrrolo[3,2-b]-pyridine-2-butanoïque

En opérant de façon analogue à l'exemple 2, au départ de l'ester obtenu selon l'exemple 196, on obtient le composé attendu sous forme d'un solide beige (rendement = 40 %).
F = 134-140°C.

### Exemple 198

### Acide 1-[(1-naphthalènyl)sulfonyl]-5-(trifluorométhyl)-1H-pyrrolo[3,2-b]-pyridine-2-butanoïque, méthyl ester

En opérant de façon analogue à l'exemple 188, au départ du chlorure de 1-naphthalènesulfonyle, on obtient le composé attendu sous forme d'une huile incolore (rendement = 49 %).
¹H RMN (250 MHz, DMSOd₆) δ : 8,57 (d, 1H) ; 8,36 (m, 2H) ; 8,15 (m, 1H) ; 7,82 (m, 2H) ; 7,69 (m, 3H) ; 7,00 (s, 1H) ; 3,52 (s, 3H) ; 2,92 (t, 2H) ; 2,35 (t, 2H) ; 1,88 (quin, 2H).

### Exemple 199

### Acide 1-[(1-naphthalènyl)sulfonyl]-5-(trifluorométhyl)-1H-pyrrolo[3,2-b]pyridine-2-butanoïque

En opérant de façon analogue à l'exemple 2, au départ de l'ester obtenu selon l'exemple 198, on obtient le composé attendu sous forme d'un solide beige (rendement = 97%).
F = 195°C.

### Exemple 200

### Acide 1-(1,3-benzodioxol-5-ylsulfonyl)-5-(trifluorométhyl)-1H-pyrrolo[3,2-b]pyridine-2-butanoïque, méthyl ester

En opérant de façon analogue à l'exemple 188, au départ du chlorure de 1,3-benzodioxole-5-sulfonyle, on obtient le composé attendu sous forme d'une huile incolore (rendement = 45 %).
¹H RMN (250 MHz, DMSOd₆) δ : 8,82 (d, 1H) ; 7,78 (d, 1H) ; 7,54 (dd, 1H) ; 7,42 (d, 1H) ; 7,08 (d, 1H) ; 6,91 (s, 1H) ; 6,15 (s, 2H) ; 3,59 (s, 3H) ; 3,11 (t, 2H) ; 2,47 (t, 2H) ; 1,96 (quin, 2H).

### Exemple 201

### Acide 1-(1,3-benzodioxol-5-ylsulfonyl)-5-(trifluorométhyl)-1H-pyrrolo[3,2-b]-pyridine-2-butanoïque

En opérant de façon analogue à l'exemple 2, au départ de l'ester obtenu selon l'exemple 200, on obtient le composé attendu sous forme d'un solide blanc (rendement = 44 %).
F = 161°C.

### Exemple 202

### Acide 1-[(3,4-dihydro-4-méthyl-2H-1,4-benzoxazin-7-yl)sulfonyl]-5-(trifluorométhyl)-1H-pyrrolo[3,2-b]pyridine-2-butanoïque, méthyl ester

En opérant de façon analogue à l'exemple 188, au départ du chlorure de 3,4-dihydro-4-méthyl-2*H*-1,4-benzoxazine-7-sulfonyle, on obtient le composé attendu sous forme d'un solide beige (rendement = 27 %).
F = 118°C.

### Exemple 203

### Acide 1-[(3,4-dihydro-4-méthyl-2H-1,4-benzoxazin-7-yl)sulfonyl]-5-(trifluorométhyl)-1H-pyrrolo[3,2-b]pyridine-2-butanoïque

En opérant de façon analogue à l'exemple 2, au départ de l'ester obtenu selon l'exemple 202, on obtient le composé attendu sous forme d'un solide rose (rendement = 39 %).
F = 134°C.

### Exemple 204

### Acide 1-[(2-méthyl-7-benzothiazolyl)sulfonyl]-5-(trifluorométhyl)-1H-pyrrolo[3,2-b]pyridine-2-butanoïque, méthyl ester

En opérant de façon analogue à l'exemple 188, au départ du chlorure de 2-méthyl-7-benzothiazolesulfonyle, on obtient le composé attendu sous forme d'une huile jaune (rendement = 9 %).
¹H RMN (250 MHz, DMSOd₆) δ : 8,60 (d, 1H) ; 8,30 (d, 1H) ; 7,91 (d, 1H) ; 7,83 (d, 1H) ; 7,70 (t, 1H) ; 6,98 (s, 1H) ; 3,54 (s, 3H) ; 2,99 (t, 2H) ; 2,83 (s, 3H) ; 2,38 (t, 2H) ; 1,90 (quin, 2H).

### Exemple 205

### Acide 1-[(2-méthyl-7-benzothiazolyl)sulfonyl]-5-(trifluorométhyl)-1H-pyrrolo[3,2-b]pyridine-2-butanoïque

En opérant de façon analogue à l'exemple 2, au départ de l'ester obtenu selon l'exemple 204, on obtient le composé attendu sous forme d'un solide beige (rendement = 48 %).
F = 187°C.

### Exemple 206

### Acide 5-(trifluorométhyl)-1-[(2,3-dihydro-1,4-benzodioxin-6-yl)sulfonyl]-1H-pyrrolo[3,2-b]pyridine-2-butanoïque, méthyl ester

En opérant de façon analogue à la préparation VI, au départ du composé obtenu selon la préparation XXII et du chlorure de 2,3-dihydro-1,4-benzodioxine-6-sulfonyle, on obtient le composé attendu sous forme d'une huile incolore (rendement = 13 %).
¹H RMN (300 MHz, DMSOd₆) δ : 8,63 (d, 1H) ; 7,79 (d, 1H) ; 7,39 (m, 2H) ; 7,04 (d, 1H) ; 6,92 (s, 1H) ; 4,28 (m, 4H) ; 3,60 (s, 3H) ; 3,10 (t, 2H) ; 2,47 (t, 2H) ; 1,99 (quin, 2H).

### Exemple 207

### Acide 5-(trifluorométhyl)-1-[(2,3-dihydro-1,4-benzodioxin-6-yl)sulfonyl]-1H-pyrrolo[3,2-b]pyridine-2-butanoïque

En opérant de façon analogue à l'exemple 2, au départ de l'ester obtenu selon l'exemple 206, on obtient le composé attendu sous forme d'un solide blanc (rendement = 88 %).
F = 158°C.

### Exemple 208

### Acide 1-(6-benzothiazolylsulfonyl)-5-chloro-1H-pyrrolo[3,2-b]pyridine-2-pentanoïque, méthyl ester

En opérant de façon analogue à la préparation VI, au départ du composé obtenu selon la préparation XXIII et du chlorure de benzothiazole-6-sulfonyle, on obtient le composé attendu sous forme d'une pâte incolore (rendement = 36 %).
¹H RMN (250 MHz, DMSOd₆) δ : 9,68 (s, 1H) ; 9,02 (d, 1H) ; 8,46 (d, 1H) ; 8,23 (d, 1H) ; 7,92 (dd, 1H) ; 7,39 (d, 1H) ; 6,75 (s, 1H) ; 3,58 (s, 3H) ; 3,06 (t, 2H) ; 2,34 (t, 2H) ; 1,66 (m, 4H).

### Exemple 209

### Acide 1-(6-benzothiazolylsulfonyl)-5-chloro-1H-pyrrolo[3,2-b]pyridine-2-pentanoïque

En opérant de façon analogue à l'exemple 2, au départ de l'ester obtenu selon l'exemple 208, on obtient le composé attendu sous forme d'un solide gris (rendement = 43 %).
F = 169°C.

### Exemple 210

### Acide 2-[[5-chloro-1-[(4-éthylphényl)sulfonyl]-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXIV, on obtient le composé attendu sous forme d'une huile jaune (rendement = 74 %).
¹H RMN (300 MHz, DMSOd₆) δ : 8,42 (d, 1H) ; 7,92 (d, 2H) ; 7,45 (d, 1H) ; 7,42 (d, 2H) ; 6,90 (s, 1H) ; 4,87 (s, 2H) ; 3,66 (s, 3H) : 2,65 (q, 2H) ; 1,45 (s, 6H) ; 1,13 (t, 3H).

### Exemple 211

### Acide 2-[[5-chloro-1-[(4-éthylphényl)sulfonyl]-1H-pyrrolo[3,2-b]pyridin-2-yl]méthoxy]-2-méthylpropanoïque

En opérant de façon analogue à l'exemple 43, au départ de l'ester obtenu selon l'exemple 210, on obtient le composé attendu sous forme d'un solide blanc (rendement = 14 %).
F = 172°C.

### Exemple 212

### Acide 2-[[5-chloro-1-[(4-méthoxyphényl)sulfonyl]-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXV, on obtient le composé attendu sous forme d'une pâte jaune (rendement = 27 %).
¹H RMN (250 MHz, DMSOd₆) δ : 8,41 (d, 1H) ; 7,96 (d, 2H) ; 7,40 (d, 1H) ; 7,10 (d, 2H) ; 6,88 (s, 1H) ; 4,87 (s, 2H) ; 3,81 (s, 3H) ; 3,67 (s, 3H) ; 1,47 (s, 6H).

### Exemple 213

### Acide 2-[[5-chloro-1-[(4-méthoxyphényl)sulfonyl]-1H-pyrrolo[3,2-b]pyridin-2-yl]méthoxy]-2-méthylpropanoïque

En opérant de façon analogue à l'exemple 43, au départ de l'ester obtenu selon l'exemple 212, on obtient le composé attendu sous forme d'un solide blanc (rendement = 57 %).
F = 199°C.

### Exemple 214

### Acide 2-[[5-chloro-1-[(2,3-dichlorophényl)sulfonyl]-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXVI, on obtient le composé attendu sous forme d'une pâte incolore. (rendement = 41 %).
¹H RMN (300 MHz, DMSOd₆) δ : 8,20 (d, 1H) ; 8,06 (dd, 1H) ; 7,72 (dd, 1H) ; 7,62 (t, 1H) ; 7,41 (d, 1H) ; 7,01 (s, 1H) ; 4,75 (s, 2H) ; 3,61 (s, 3H) ; 1,24 (s, 6H).

### Exemple 215

### Acide 2-[[5-chloro-1-[(2,3-dichlorophényl)sulfonyl]-1H-pyrrolo[3,2-b]pyridin-2-yl]méthoxy]-2-méthylpropanoïque

En opérant de façon analogue à l'exemple 43, au départ de l'ester obtenu selon l'exemple 214, on obtient le composé attendu sous forme d'un solide jaune (rendement = 16 %).
F = 174°C.

### Exemple 216

### Acide 2-[[5-chloro-1-[[4-(1-méthyléthyl)phényl]sulfonyl]-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXVII, on obtient le composé attendu sous forme d'un solide blanc (rendement = 87 %).
F = 120°C.

### Exemple 217

### Acide 2-[[5-chloro-1-[[4-(1-méthyléthyl)phényl]sulfonyl]-1H-pyrrolo[3,2-b]pyridin-2-yl]méthoxy]-2-méthylpropanoïque

En opérant de façon analogue à l'exemple 43, au départ de l'ester obtenu selon l'exemple 216, on obtient le composé attendu sous forme d'un solide jaune (rendement = 39 %).
F = 190°C.

### Exemple 218

### Acide 2-[[5-chloro-1-(1-naphthalènylsulfonyl)-1H-pyrrolo[3,2-b]pyridin-2yl]méthoxy]-2-méthylpropanoïque, méthyl ester

En opérant de façon analogue à l'exemple 42, au départ du composé obtenu selon la préparation XXVIII, on obtient le composé attendu sous forme d'un solide blanc (rendement = 54 %).
F = 108°C.

### Exemple 219

### Acide 2-[[5-chloro-1-(1-naphthalènylsulfonyl)-1H-pyrrolo[3,2-b]pyridin-2-yl]méthoxy]-2-méthylpropanoïque

En opérant de façon analogue à l'exemple 43, au départ de l'ester obtenu selon l'exemple 218, on obtient le composé attendu sous forme d'un solide bleuté (rendement = 36 %).
F = 209°C.

### Exemple 220

### Acide 2-[[5-chloro-1-(8-quinolinylsulfonyl)-1H-pyrrolo[3,2-b]pyridin-2-yl]-méthoxy]-2-méthylpropanoïque, méthyl ester

En opérant de façon analogue à l'exemple 42, au départ du composé obtenu selon la préparation XXIX, on obtient le composé attendu sous forme d'une mousse brune (rendement = 66 %).
¹H RMN (250 MHz, DMSOd₆) δ : 8,77 (dd, 1H) ; 8,67 (dd, 1H) ; 8,51 (dd, 1H) ; 8,42 (dd, 1H) ; 8,24 (d, 1H) ; 7,65 (t, 1H) ; 7,28 (m, 1H) ; 7,20 (d, 1H) ; 6,76 (s, 1H) ; 5,11 (s, 2H) ; 3,63 (s, 3H) ; 1,39 (s, 6H).

### Exemple 221

### Acide 2-[[5-chloro-1-(8-quinolinylsulfonyl)-1H-pyrrolo[3,2-b]pyridin-2-yl]méthoxy]-2-méthylpropanoïque

En opérant de façon analogue à l'exemple 43, au départ de l'ester obtenu selon l'exemple 220, on obtient le composé attendu sous forme d'un solide beige (rendement = 14 %).
F = 223°C.

### Exemple 222

### Acide 2-[[5-chloro-1-[(2,5-diméthoxyphényl)sulfonyl]-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXX, on obtient le composé attendu sous forme d'une huile brune (rendement = 37 %).
¹H RMN (250 MHz, DMSOd₆) δ : 8,20 (d, 1H) ; 7,52 (d, 1H) ; 7,36 (d, 1H) ; 7,32 (dd, 1H) ; 7,14 (d, 1H) ; 6,81 (s, 1H) ; 4,77 (s, 2H) ; 3,82 (s, 3H) ; 3,64 (s, 3H) ; 3,48 (s, 3H) ; 1,37 (s, 6H).

### Exemple 223

### Acide 2-[[5-chloro-1-[(2,5-diméthoxyphényl)sulfonyl]-1H-pyrrolo[3,2-b]pyridin-2-yl]méthoxy]-2-méthylpropanoïque

En opérant de façon analogue à l'exemple 43, au départ de l'ester obtenu selon l'exemple 222, on obtient le composé attendu sous forme d'un solide beige (rendement = 23 %).
F = 200°C.

### Exemple 224

### Acide 2-[[1-(1,3-benzodioxol-5-ylsulfonyl)-5-chloro-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXXI, on obtient le composé attendu sous forme d'un solide blanc (rendement = 99 %).
F = 131°C.

### Exemple 225

### Acide 2-[[1-(1,3-benzodioxol-5-ylsulfonyl)-5-chloro-1H-pyrrolo[3,2-b]pyridin-2-yl]méthoxy]-2-méthylpropanoïque

En opérant de façon analogue à l'exemple 43, au départ de l'ester obtenu selon l'exemple 224, on obtient le composé attendu sous forme d'un solide blanc (rendement = 39 %).
F = 172°C.

### Exemple 226

### Acide 2-[[5-chloro-1-[(2,3-dihydro-1,4-benzodioxin-6-yl)sulfonyl]-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXXII, on obtient le composé attendu sous forme d'une huile brune (rendement = 91 %).
¹H RMN (500 MHz, DMSOd₆) δ : 8,41 (d, 1H) ; 7,58 (m, 2H) ; 7,41 (d, 1H) ; 7,13 (d, 1H) ; 6,87 (s, 1H) ; 4,87 (s, 2H) ; 4,30 (m, 4H) ; 3,67 (s, 3H) ; 1,47 (s, 6H).

### Exemple 227

### Acide 2-[[5-chloro-1-[(2,3-dihydro-1,4-benzodioxin-6-yl)sulfonyl]-1H-pyrrolo[3,2-b]pyridin-2-yl]méthoxy]-2-méthylpropanoïque

En opérant de façon analogue à l'exemple 43, au départ de l'ester obtenu selon l'exemple 226, on obtient le composé attendu sous forme d'un solide beige (rendement = 59 %).
F = 213°C.

### Exemple 228

### Acide 2-[[5-chloro-1-(2,3-dihydro-5-benzofuranesulfonyl)-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXXIII, on obtient le composé attendu sous forme d'une huile jaune (rendement = 99 %).
¹H RMN (300 MHz, DMSOd₆) δ : 8,40 (d, 1H) ; 7,91 (d, 1H) ; 7,83 (dd, 1H) ; 7,40 (d, 1H) ; 6,93 (d, 1H) ; 6,87 (s, 1H) ; 4,89 (s, 2H) ; 4,62 (t, 2H) ; 3,67 (s, 3H) ; 3,20 (t, 2H) ; 1,47 (s, 6H).

### Exemple 229

### Acide 2-[[5-chloro-1-(2,3-dihydro-5-benzofuranesulfonyl)-1H-pyrrolo[3,2-b]pyridin-2-yl]méthoxy]-2-méthylpropanoïque

En opérant de façon analogue à l'exemple 43, au départ de l'ester obtenu selon l'exemple 228, on obtient le composé attendu sous forme d'un solide beige (rendement = 26 %).
F = 169°C.

### Exemple 230

### Acide 2-[[5-chloro-1-[(3,5-diméthylphényl)sulfonyl]-1H-pyrrolo[3,2-b]-pyridin-2-yl]méthoxy]-2-méthylpropanoïque, méthyl ester

En opérant de façon analogue à l'exemple 42, au départ du composé obtenu selon la préparation XXXIV, on obtient le composé attendu sous forme d'une huile brune (rendement = 99 %).
¹H RMN (300 MHz, DMSOd₆) δ : 8,40 (d, 1H) 7,64 (s, 2H) ; 7,40 (d, 1H) ; 7,38 (s, 1H) ; 6,89 (s, 1H) ; 4,90 (s, 2H) ; 3,66 (s, 3H) ; 2,31 (s, 6H) ; 1,45 (s, 6H).

### Exemple 231

### Acide 2-[[5-chloro-1-[(3,5-diméthylphényl)sulfonyl]-1H-pyrrolo[3,2-b]pyridin-2-yl]méthoxy]-2-méthylpropanoïque

En opérant de façon analogue à l'exemple 43, au départ de l'ester obtenu selon l'exemple 230, on obtient le composé attendu sous forme d'un solide beige (rendement = 40 %).
F = 154°C.

### Exemple 232

### Acide 2-[[5-chloro-1-[(3,4-dihydro-4-méthyl-2H-1,4-benzoxazin-7-yl)sulfonyl]-1H-pyrrolo[3,2-b]pyridin-2-yl]méthoxy]-2-méthylpropanoïque, méthyl ester

En opérant de façon analogue à l'exemple 42, au départ du composé obtenu selon la préparation XXXV, on obtient le composé attendu sous forme d'une huile brune (rendement = 99 %).
¹H RMN (300 MHz, DMSOd₆) δ : 8,44 (d, 1H) ; 7,39 (d, 1H) ; 7,15 (dd, 1H) ; 6,98 (d, 1H) ; 6,82 (m, 2H) ; 4,87 (s, 2H) ; 4,25 (t, 2H) ; 3,66 (s, 3H) ; 3,26 (t, 2H) ; 2,83 (s, 3H) ; 1,45 (s 6H).

### Exemple 233

### Acide 2-[[5-chloro-1-[(3,4-dihydro-4-méthyl-2H-1,4-benzoxazin-7-yl)sulfonyl]-1H-pyrrolo[3,2-b]pyridin-2-yl]méthoxy]-2-méthylpropanoïque

En opérant de façon analogue à l'exemple 43, au départ de l'ester obtenu selon l'exemple 232, on obtient le composé attendu sous forme d'un solide rose (rendement = 37 %).
F = 229°C.

### Exemple 234

### Acide (2S)-2-[[5-chloro-1-(phénylsulfonyl)-1H-pyrrolo[3,2-b]pyridin-2-yl]méthoxy]propanoïque, éthyl ester

a)- Ester éthylique de l'acide (2S)-2-(2-propynyloxy)propanoïque : ce composé est obtenu, avec un rendement de 24 %, par réaction du bromure de propargyle sur l'ester éthylique de l'acide (S)-(-)-lactique préalablement sodé par l'hydrure de sodium dans le THF (liquide incolore ; Eb = 70-73°C sous 13 hPa).
b)- En opérant de façon analogue à l'exemple 1, au départ de l'ester obtenu au a) ci-dessus, on obtient le composé attendu sous forme d'une huile jaune (rendement = 19 %).
¹H RMN (300 MHz, DMSOd₆) δ : 8,42 (d, 1H) ; 8,02 (d, 2H) ; 7,74 (m, 1H) ; 7,60 (m, 2H) ; 7,43 (d, 1H) ; 6,95 (s, 1H) ; 5,04 (d, 1H) ; 4,92 (d, 1H) ; 4,23 (q, 1H) ; 4,12 (q, 2H) ; 1,31 (d, 3H) ; 1,16 (t, 3H)

### Exemple 235

### Acide (2S)-2-[[5-chloro-1-(phénylsulfonyl)-1H-pyrrolo[3,2-b]pyridin-2-yl]-méthoxy]propanoïque

En opérant de façon analogue à l'exemple 2, au départ de l'ester obtenu selon l'exemple 234, on obtient le composé attendu sous forme d'un solide rose (rendement = 33 %).
F = 154°C.
[α]_{D} = -50 ° (c = 0,39 ; MeOH).

### Exemple 236

### Acide (2R)-2-[[5-chloro-1-(phénylsulfonyl)-1H-pyrrolo[3,2-b]pyridin-2-yl]méthoxy]propanoïque, méthyl ester

En opérant de façon analogue à l'exemple 234, au départ de l'ester méthylique de l'acide (R)-(+)-lactique, on obtient
a)- Ester méthylique de l'acide (2R)-2-(2-propynyloxy)propanoïque : (liquide incolore ; Eb = 81-88°C sous pression atmosphérique).
b)- Le composé attendu sous forme d'une huile jaune (rendement = 73 %).
¹H RMN (300 MHz, DMSOd₆) δ : 8,42 (d, 1H) ; 8,01 (dd, 2H) ; 7,73 (m, 1H) ; 7,63 (m, 2H) ; 7,43 (d, 1H) ; 6,96 (s, 1H) ; 5,05 (d, 1H) ; 4,92 (d, 1H) ; 4,26 (q, 1H) ; 3,66 (s, 3H) ; 1,31 (d, 3H).

### Exemple 237

### Acide (2R)-2-[[5-chloro-1-(phénylsulfonyl)-1H-pyrrolo[3,2-b]pyridin-2-yl]-méthoxy]propanoïque

En opérant de façon analogue à l'exemple 2, au départ de l'ester obtenu selon l'exemple 236, on obtient le composé attendu sous forme d'un solide blanc (rendement = 58 %).
F = 150°C.
[α]_{D} = +50 ° (c = 0,375 ; MeOH).

### Exemple 238

### Acide (2S)-2-[[1-(phénylsulfonyl)-5-(trifluorométhyl)-1H-pyrrolo[3,2-b]-pyridin-2-yl]méthoxy]propanoïque, éthyl ester

En opérant de façon analogue à l'exemple 234, au départ du composé obtenu selon la préparation VII, on obtient le composé attendu sous forme d'une huile jaune (rendement = 91 %).
¹H RMN (300 MHz, DMSOd₆) δ : 8,63 (d, 1H) ; 8,06 (dd, 2H) ; 7,84 (d, 1H) ; 7,74 (m, 1 H) ; 7,62 (m, 2H) ; 7,12 (s, 1H) ; 5,10 (d, 1H) ; 4,97 (d, 1H) ; 4,26 (q, 1H) ; 4,15 (q, 2H) ; 1,31 (d, 3H) ; 1,18 (t, 3H).

### Exemple 239

### Acide (2S)-2-[[1-(phénylsulfonyl)-5-(trifluorométhyl)-1H-pyrrolo[3,2-b]-pyridin-2-yl]méthoxy]propanoïque

En opérant de façon analogue à l'exemple 2, au départ de l'ester obtenu selon l'exemple 238, on obtient le composé attendu sous forme d'un solide beige (rendement = 60 %).
F = 142°C.
[α]_{D} = -46 ° (c = 0,52 ; MeOH).

### Exemple 240

### Acide (2R)-2-[[1-(phénylsulfonyl)-5-(trifluorométhyl)-1H-pyrrolo[3,2-b]-pyridin-2-yl]méthoxy]propanoïque, méthyl ester

En opérant de façon analogue à l'exemple 236, au départ du composé obtenu selon la préparation VII, on obtient le composé attendu sous forme d'une huile jaune (rendement = 88 %).
¹H RMN (300 MHz, DMSOd₆) δ : 8,63 (d, 1H) ; 8,06 (d, 2H) ; 7,83 (d, 1H) ; 7,75 (m, 1H) ; 7,62 (t, 2H) ; 7,13 (s, 1H) ; 5,10 (d, 1H) ; 4,96 (d, 1H) ; 4,28 (q, 1H) ; 3,66 (s, 3H) ; 1,31 (d, 3H).

### Exemple 241

### Acide (2R)-2-[[1-(phénylsulfonyl)-5-(trifluorométhyl)-1H-pyrrolo[3,2-b]-pyridin-2-yl]méthoxy]propanoïque

En opérant de façon analogue à l'exemple 2, au départ de l'ester obtenu selon l'exemple 240, on obtient le composé attendu sous forme d'un solide beige (rendement = 78 %).
F = 140°C.
[α]_{D} = +39 ° (c = 0,39 ; MeOH).

### Exemple 242

### Acide 5-méthyl-1-(phénylsulfonyl)-1H-pyrrolo[3,2-b]pyridine-2-propanoique, méthyl ester

En opérant de façon analogue à l'exemple 1, au départ du composé obtenu selon la préparation XXXVI, on obtient le composé attendu sous forme d'un solide brun (rendement = 56 %).
F = 98-115°C.

### Exemple 243

### Acide 5-méthyl-1-(phénylsulfonyl)-1H-pyrrolo[3,2-b]pyridine-2-propanoïque

En opérant de façon analogue à l'exemple 2, au départ de l'ester obtenu selon l'exemple 242, on obtient le composé attendu sous forme d'un solide jaune (rendement = 21 %).
F = 88-92°C.

### Exemple 244

### Acide 5-méthoxy-1-(phénylsulfonyl)-1H-pyrrolo[3,2-b]pyridine-2-propanoïque, méthyl ester

En opérant de façon analogue à l'exemple 1, au départ du composé obtenu selon la préparation XXXVIII, on obtient le composé attendu sous forme d'un solide blanc (rendement = 63 %).
F = 145-150°C.

### Exemple 245

### Acide 5-méthoxy-1-(phénylsulfonyl)-1H-pyrrolo[3,2-b]pyridine-2-propanoïque

En opérant de façon analogue à l'exemple 2, au départ de l'ester obtenu selon l'exemple 244, on obtient le composé attendu sous forme d'un solide beige (rendement = 75 %).
F = 136-139°C.

### Exemple 246

### Acide 5-méthoxy-1-(phénylsulfonyl)-1H-pyrrolo[3,2-b]pyridine-2-butanoïque, méthyl ester

En opérant de façon analogue à l'exemple 1, au départ du composé obtenu selon la préparationXL, on obtient le composé attendu sous forme d'un solide jaune (rendement = 86 %).
F = 113-114°C.

### Exemple 247

### Acide 5-méthoxy-1-(phénylsulfonyl)-1H-pyrrolo[3,2-b]pyridine-2-butanoïque

En opérant de façon analogue à l'exemple 2, au départ de l'ester obtenu selon l'exemple 246, on obtient le composé attendu sous forme d'un solide beige (rendement = 34 %).
F = 190-198°C.

### Exemple 248

### Acide 5-méthyl-1-(phénylsulfonyl)-1H-pyrrolo[3,2-b]pyridine-2-butanoïque, méthyl ester

En opérant de façon analogue à l'exemple 242, au départ de l'ester méthylique de l'acide 5-hexynoïque, on obtient le composé attendu sous forme d'un solide jaune (rendement = 92 %).
F = 91-94°C.

### Exemple 249

### Acide 5-méthyl-1-(phénylsulfonyl)-1H-pyrrolo[3,2-b]pyridine-2-butanoïque

En opérant de façon analogue à l'exemple 2, au départ de l'ester obtenu selon l'exemple 248, on obtient le composé attendu sous forme d'un solide jaune (rendement = 38 %).
F = 172-180°C.

### Exemple 250

### Acide 1-(6-benzothiazolylsulfonyl)-5-méthyl-1H-pyrrolo[3,2-b]pyridine-2-butanoïque, méthyl ester

En opérant de façon analogue à l'exemple 248, au départ du composé obtenu selon la préparation XLI, on obtient le composé attendu sous forme d'un solide jaune (rendement = 76 %).
¹H RMN (300 MHz, DMSOd₆) δ : 9,65 (s, 1H) ; 8,97 (d, 1H) ; 8,29 (d, 1H) ; 8,20 (d, 1H) ; 7,85 (dd, 1H) ; 7,17 (d, 1H) ; 6,66 (s, 1H) ; 3,58 (s, 3H) ; 3,10 (t, 2H) ; 2,49 (s, 3H) ; 2,41 (t, 2H) ; 1,99 (quin, 2H).

### Exemple 251

### Acide 1-(6-benzothiazolylsulfonyl)-5-méthyl-1H-pyrrolo[3,2-b]pyridine-2-butanoïque

En opérant de façon analogue à l'exemple 2, au départ de l'ester obtenu selon l'exemple 250, on obtient le composé attendu sous forme d'un solide violet (rendement = 14 %).
F = 67-70°C.

### Exemple 252

### Acide 5-chloro-1-(3-pyridinylsulfonyl)-1H-pyrrolo[3,2-b]pyridine-2-butanoïque, méthyl ester

En opérant de façon analogue à l'exemple 248, au départ du composé obtenu selon la préparation XLII, on obtient le composé attendu sous forme d'un solide blanc (rendement = 57 %).
F = 119°C.

### Exemple 253

### Acide 5-chloro-1-(3-pyridinylsulfonyl)-1H-pyrrolo[3,2-b]pyridine-2-butanoïque

En opérant de façon analogue à l'exemple 2, au départ de l'ester obtenu selon l'exemple 252, on obtient le composé attendu sous forme d'un solide blanc (rendement = 73 %).
F = 181°C.

### Exemple 254

### Acide 5-chloro-1-(6-quinolinylsulfonyl)-1H-pyrrolo[3,2-b]pyridine-2-butanoïque, méthyl ester

En opérant de façon analogue à l'exemple 248, au départ du composé obtenu selon la préparation XLIII, on obtient le composé attendu sous forme d'un solide marron (rendement = 72 %).
F = 133-141°C.

### Exemple 255

### Acide 5-chloro-1-(6-quinolinylsulfonyl)-1H-pyrrolo[3,2-b]pyridine-2-butanoïque

En opérant de façon analogue à l'exemple 2, au départ de l'ester obtenu selon l'exemple 254, on obtient le composé attendu sous forme d'un solide beige (rendement = 40 %).
F = 153-162°C.

### Exemple 256

### Acide 5-chloro-1-(phénylsulfonyl)-1H-pyrrolo[3,2-b]pyridine-2-pentanoïque, méthyl ester

En opérant de façon analogue à l'exemple 1, au départ de l'ester méthylique de l'acide heptynoïque, on obtient le composé attendu sous forme d'un solide blanc (rendement = 37 %).
F = 73°C.

### Exemple 257

### Acide 5-chloro-1-(phénylsulfonyl)-1H-pyrrolo[3,2-b]pyridine-2-pentanoïque

En opérant de façon analogue à l'exemple 2, au départ de l'ester obtenu selon l'exemple 256, on obtient le composé attendu sous forme d'un solide beige (rendement = 69 %).
F = 113°C.

### Exemple 258

### Acide 5-chloro-1-[(3,5-diméthylphényl)sulfonyl]-1H-pyrrolo[3,2-b]pyridine-2-pentanoïque, méthyl ester

En opérant de façon analogue à l'exemple 256, au départ du composé obtenu selon la préparation XXXIV, on obtient le composé attendu sous forme d'un solide beige (rendement = 84 %).
F = 126°C.

### Exemple 259

### Acide 5-chloro-1-[(3,5-diméthylphényl)sulfonyl]-1H-pyrrolo[3,2-b]pyridine-2-pentanoïque

En opérant de façon analogue à l'exemple 2, au départ de l'ester obtenu selon l'exemple 258, on obtient le composé attendu sous forme d'un solide blanc (rendement = 84 %).
F = 160°C.

### Exemple 260

### Acide 5-chloro-α,α-diméthyl-1-(phénylsulfonyl)-1H-pyrrolo[3,2-b]pyridine-2-butanoïque,

En opérant de façon analogue à l'exemple 1, au départ de l'acide 2,2-diméthylhexynoïque, on obtient le composé attendu sous forme d'un solide beige (rendement = 14 %).
F = 197°C.

### Exemple 261

### Acide 1-(6-benzothiazolylsulfonyl)-5-chloro-α,α-diméthyl-1H-pyrrolo[3,2-b]pyridine-2-butanoïque

En opérant de façon analogue à l'exemple 1, au départ de l'acide 2,2-diméthylhexynoïque et du composé obtenu selon la préparation XLIV, on obtient le composé attendu sous forme d'un solide beige (rendement = 8 %).
F = 210°C.

### Exemple 262

### Acide 1-[[4-(1-méthyléthyl)phényl]sulfonyl]-5-(trifluorométhyl)-1H-pyrrolo-[3,2-b]pyridine-2-butanoïque, méthyl ester

On prépare une solution de 0,263 g (0,92 mM) du composé obtenu selon la préparation XLV dans 10 ml de DMF et on ajoute à 0°C, 73 mg (1,8 mM) d'hydrure de sodium en suspension à 60 % dans l'huile. Le mélange est agité pendant 15 mn à 0°C, puis on ajoute 0,302 g (1,4 mM) de chlorure de 4-(1-méthyléthyl)benzènesulfonamide. Le milieu réactionnel est agité pendant 60 heures à température ambiante puis versé sur un mélange de glace pilée et de chlorure d'ammonium, et extrait par de l'acétate d'éthyle. La phase organique obtenue est séchée sur sulfate de magnésium et concentrée sous pression réduite. On obtient ainsi le composé attendu sous forme d'un solide brun (rendement = 93 %).
F = 78°C.

### Exemple 263

### Acide 1-[[4-(1-méthyléthyl)phényl]sulfonyl]-5-(trifluorométhyl)-1H-pyrrolo-[3,2-b]pyridine-2-butanoïque

En opérant de façon analogue à l'exemple 2, au départ de l'ester obtenu selon l'exemple 262, on obtient le composé attendu sous forme d'un solide vert clair (rendement = 50 %).
F = 144°C.

### Exemple 264

### Acide 1-[(4-chlorophényl)sulfonyl]-5-(trifluorométhyl)-1H-pyrrolo[3,2-b]-pyridine-2-butanoïque, méthyl ester

En opérant de façon analogue à l'exemple 262, au départ de chlorure de 4-chlorobenzènesulfonyle, on obtient le composé attendu sous forme d'un solide blanc (rendement = 47 %).
F = 97°C.

### Exemple 265

### Acide 1-[(4-chlorophényl)sulfonyl]-5-(trifluorométhyl)-1H-pyrrolo[3,2-b]-pyridine-2-butanoïque

En opérant de façon analogue à l'exemple 2, au départ de l'ester obtenu selon l'exemple 264, on obtient le composé attendu sous forme d'un solide vert clair (rendement = 37 %).
F = 176°C.

### Exemple 266

### Acide 1-[(2-chlorophényl)sulfonyl]-5-(trifluorométhyl)-1H-pyrrolo[3,2-b]-pyridine-2-butanoïque, méthyl ester

En opérant de façon analogue à l'exemple 262, au départ de chlorure de 2-chlorobenzènesulfonyle, on obtient le composé attendu sous forme d'un solide beige (rendement = 71 %).
F = 88°C.

### Exemple 267

### Acide 1-[(2-chlorophényl)sulfonyl]-5-(trifluorométhyl)-1H-pyrrolo[3,2-b]-pyridine-2-butanoïque

En opérant de façon analogue à l'exemple 2, au départ de l'ester obtenu selon l'exemple 266, on obtient le composé attendu sous forme d'un solide vert clair (rendement = 50 %).
F = 171°C.

### Exemple 268

### Acide 1-[[(3-(trifluorométhoxy)phényl]sulfonyl]-5-(trifluorométhyl)-1H-pyrrolo[3,2-b]pyridine-2-butanoïque, méthyl ester

En opérant de façon analogue à l'exemple 262, au départ de chlorure de 3-(trifluorométhoxy)benzènesulfonyle, on obtient le composé attendu sous forme d'un solide ocre (rendement = 88 %).
F = 72°C.

### Exemple 269

### Acide 1-[[(3-(trifluorométhoxy)phényl]sulfonyl]-5-(trifluorométhyl)-1H-pyrrolo[3,2-b]pyridine-2-butanoïque

En opérant de façon analogue à l'exemple 2, au départ de l'ester obtenu selon l'exemple 268, on obtient le composé attendu sous forme d'un solide beige (rendement = 27 %).
F = 164°C.

### Exemple 270

### Acide 1-[(4-bromophényl)sulfonyl]-5-chloro-1H-pyrrolo[3,2-b]pyridine-2-butanoïque, méthyl ester

En opérant de façon analogue à l'exemple 52, au départ de l'ester obtenu selon la préparation V et du chlorure de 4-bromobenzènesulfonamide, on obtient le composé attendu sous forme d'un solide blanc (rendement = 97%).
F = 102 °C.

### Exemple 271

### Acide 5-chloro-1-[[4-(4-morpholinyl)phényl]sulfonyl]-1H-pyrrolo[3,2-b]-pyridine-2-butanoïque, méthyl ester

On place dans un tube réacteur adapté au chauffage par micro-ondes, en maintenant sous atmosphère d'argon, 1,5 mg de tris(benzylidèneacétone)-dipalladium, 2 mg de di(*t*-butyl)([1,1'-biphényl]2-yl)phosphine, 48 mg (0,22 mM) de phosphate de potassium, 75 mg ( 0,159 mM) du composé obtenu selon l'exemple 270 et 0,16 ml de DME. Ces composés sont mélangés et on ajoute 17 mg (0,19 mM) de morpholine et 0,32 ml de DME. Ce mélange réactionnel est chauffé sous micro ondes à 110 °C pendant 2 heures puis concentré sous pression réduite. Le résidu est purifié par chromatographie sur gel de silice en éluant à l'aide d'un mélange toluène/acétate d'éthyle (en gradient de 9/1 à 8/2 ; v/v). On obtient ainsi le composé attendu sous forme d'un solide blanc (rendement = 80%).
F = 110°C.

### Exemple 272

### Acide 5-chloro-1-[[4-(4-morpholinyl)phényl]sulfonyl]-1H-pyrrolo[3,2-b]-pyridine-2-butanoïque

En opérant de façon analogue à l'exemple 2, au départ de l'ester obtenu selon l'exemple 271, on obtient le composé attendu sous forme d'un solide blanc (rendement = 55 %).
F = 176°C.

### Exemple 273

### Acide 5-chloro-1-[[4-(1-pyrrolidinyl)phényl]sulfonyl]-1H-pyrrolo[3,2-b]-pyridine-2-butanoïque, méthyl ester

En opérant de façon analogue à l'exemple 271, au départ de pyrrolidine, on obtient le composé attendu sous forme d'un solide blanc (rendement = 63 %).
F= 121°C.

### Exemple 274

### Acide 5-chloro-1-[[4-(1-pyrrolidinyl)phényl]sulfonyl]-1H-pyrrolo[3,2-b]-pyridine-2-butanoïque

En opérant de façon analogue à l'exemple 2, au départ de l'ester obtenu selon l'exemple 273, on obtient le composé attendu sous forme d'un solide blanc (rendement = 99 %).
F = 88 °C.

### Exemple 275

### Acide 5-chloro-1-[[4-(diméthylamino)phényl]sulfonyl]-1H-pyrrolo[3,2-b]-pyridine-2-butanoïque, méthyl ester

En opérant de façon analogue à l'exemple 271, au départ de diméthylamine en solution 2N dans le THF, on obtient le composé attendu sous forme d'un solide blanc (rendement = 35 %).
F = 188°C.

### Exemple 276

### Acide 5-chloro-1-[[4-(diméthylamino)phényl]sulfonyl]-1H-pyrrolo[3,2-b]-pyridine-2-butanoïque

En opérant de façon analogue à l'exemple 2, au départ de l'ester obtenu selon l'exemple 275, on obtient le composé attendu sous forme d'un solide blanc (rendement = 99 %).
F = 76°C.

### Exemple 277

### Acide 5-chloro-1-[(3-bromophényl)sulfonyl]-1H-pyrrolo[3,2-b]pyridine-2-butanoïque, méthyl ester

En opérant de façon analogue à l'exemple 52, au départ de l'ester obtenu selon la préparation V et du chlorure de 3-bromobenzènesulfonamide, on obtient le composé attendu sous forme d'un solide blanc (rendement = 97%).
F = 109°C.

### Exemple 278

### Acide 5-chloro-1-[[3-(diméthylamino)phényl]sulfonyl]-1H-pyrrolo[3,2-b]pyridine-2-butanoïque, méthyl ester

En opérant de façon analogue à l'exemple 275, au départ de diméthylamine en solution 2N dans le THF et du dérivé bromé obtenu selon l'exemple 277, on obtient le composé attendu sous forme d'un solide beige (rendement = 6 %).
F = 96-97°C.

### Exemple 279

### Acide 5-chloro-1-[(4-iodophényl)sulfonyl]-1H-pyrrolo[3,2-b]pyridine-2-butanoïque, méthyl ester

En opérant de façon analogue à l'exemple 52, au départ de l'ester obtenu selon la préparation V et du chlorure de 4-iodobenzènesulfonamide, on obtient le composé attendu sous forme d'un solide beige (rendement = 70 %).
F = 117-118°C.

### Exemple 280

### Acide 5-chloro-1-[[4-(2-thiazolyl)phényl]sulfonyl]-1H-pyrrolo[3,2-b]pyridine-2-butanoïque, méthyl ester

Dans un tube-réacteur adapté pour chauffage sous micro-ondes, on prépare une solution de 249 mg (0,48 mM) du composé obtenu selon l'exemple 279 dans 2,5 ml de toluène et on ajoute 110 mg (0,096 mM) de tetrakis-(triphényl)phosphine, puis 0,15 ml de 2-(tributylstannyl)thiazole et 0,4 ml de N-méthylpyrrolidinone. Le mélange est ensuite chauffé à 100 °C par micro-ondes pendant 1 heure et 30 mn. Après refroidissement, on ajoute de l'eau et extrait par de l'éther diéthylique. La phase organique obtenue est lavée à l'eau, puis séchée sur sulfate de magnésium et concentrée sous pression réduite. Le produit brut obtenu est purifié par chromatographie sur gel de silice en éluant à l'aide d'un mélange éther de pétrole/éther diéthylique (6/4 ; v/v). Le composé purifié est cristallisé dans un mélange éther diéthylique et éther de pétrole. On obtient le composé attendu sous forme d'un solide jaune (rendement = 57 %).
F = 101 °C.

### Exemple 281

### Acide 5-chloro-1-[[4-(2-thiazolyl)phényl]sulfonyl]-1H-pyrrolo[3,2-b]pyridine-2-butanoïque

En opérant de façon analogue à l'exemple 2, au départ de l'ester obtenu selon l'exemple 280, on obtient le composé attendu sous forme d'un solide blanc (rendement = 44 %).
F = 214-215°C.

### Exemple 282

### Acide 5-chloro-1-[[3-(2-thiazolyl)phényl]sulfonyl]-1H-pyrrolo[3,2-b]pyridine-2-butanoïque, méthyl ester

En opérant de façon analogue à l'exemple 280, au départ du dérivé bromé obtenu selon l'exemple 277, on obtient le composé attendu sous forme d'un solide blanc (rendement = 32 %).
F = 82-86°C.

### Exemple 283

### Acide 5-chloro-1-[[3-(2-thiazolyl)phényl]sulfonyl]-1H-pyrrolo[3,2-b]pyridine-2-butanoïque

En opérant de façon analogue à l'exemple 2, au départ de l'ester obtenu selon l'exemple 282, on obtient le composé attendu sous forme d'un solide blanc (rendement = 68 %).
F = 187-188°C.

### Exemple 284

### Acide 5-chloro-1-[[3-(5-thiazolyl)phényl]sulfonyl]-1H-pyrrolo[3,2-b]pyridine-2-butanoïque, méthyl ester

En opérant de façon analogue à l'exemple 282, au départ du dérivé bromé obtenu selon l'exemple 277 et du 5-(tributylstannyl)thiazole, on obtient le composé attendu sous forme d'un solide blanc (rendement = 71 %).
F = 147-148°C.

### Exemple 285

### Acide 5-chloro-1-[[3-(5-thiazolyl)phényl]sulfonyl]-1H-pyrrolo[3,2-b]pyridine-2-butanoïque

En opérant de façon analogue à l'exemple 2, au départ de l'ester obtenu selon l'exemple 284; on obtient le composé attendu sous forme d'un solide blanc (rendement = 45 %).
F = 157-160°C.

### Exemple 286

### Acide 5-chloro-1-[[3-(4-thiazolyl)phényl]sulfonyl]-1H-pyrrolo[3,2-b]pyridine-2-butanoïque, méthyl ester

En opérant de façon analogue à l'exemple 282, au départ du dérivé bromé obtenu selon l'exemple 277 et du 4-(tributylstannyl)thiazole, on obtient le composé attendu sous forme d'un solide blanc (rendement = 38 %).
F = 147-148°C.

### Exemple 287

### Acide 5-chloro-1-[[3-(4-thiazolyl)phényl]sulfonyl]-1H-pyrrolo[3,2-b]pyridine-2-butanoïque

En opérant de façon analogue à l'exemple 2, au départ de l'ester obtenu selon l'exemple 286, on obtient le composé attendu sous forme d'un solide blanc (rendement = 4 %).
F = 185-186 °C.

### Exemple 288

### Acide 5-chloro-1-[[4-(2-pyridinyl)phényl]sulfonyl]-1H-pyrrolo[3,2-b]-pyridine-2-butanoïque, méthyl ester

En opérant de façon analogue à l'exemple 280, au départ du dérivé bromé obtenu selon l'exemple 270 et de la 2-(tributylstannyl)pyridine, on obtient le composé attendu sous forme d'un solide orange (rendement = 61 %).
F = 123-124°C.

### Exemple 289

### Acide 5-chloro-1-[[4-(2-pyridinyl)phényl]sulfonyl]-1H-pyrrolo[3,2-b]-pyridine-2-butanoïque

En opérant de façon analogue à l'exemple 2, au départ de l'ester obtenu selon l'exemple 288, on obtient le composé attendu sous forme d'un solide beige (rendement = 40 %).
F = 214-215°C.

### Exemple 290

### Acide 5-chloro-1-[[4-(3-pyridinyl)phényl]sulfonyl]-1H-pyrrolo[3,2-b]pyridine-2-butanoïque, méthyl ester

Dans un tube-réacteur adapté pour chauffage sous micro-ondes, on prépare un mélange de 300 mg (0,63 mM) du composé obtenu selon l'exemple 270, 98 mg (0,80 mM) d'acide 3-pyridineboronique, 0,9 ml d'une solution 2M de carbonate de potassium dans 5 ml de DME et on ajoute 6 mg de PdCl₂dppf. Le mélange est ensuite chauffé à 120°C par micro-ondes pendant 1 heure. Après refroidissement, on ajoute de l'eau et extrait par de l'acétate d'éthyle. La phase organique obtenue est lavée à l'eau, puis séchée sur sulfate de magnésium et concentrée sous pression réduite. Le produit brut obtenu est purifié par chromatographie sur gel de silice en éluant à l'aide d'un mélange cyclohexane/acétate d'éthyle (80/20 puis 70/30 ; v/v). On obtient ainsi le composé attendu sous forme d'une huile incolore (rendement = 37 %).
¹H RMN (300 MHz, DMSOd₆) δ : 8,90 (d, 1H) ; 8,63 (dd, 1H) ; 8,47 (d, 1H) ; 8,11 (m, 1H) ; 7,97 (m, 4H) ; 7,51 (m, 1H) ; 7,41 (d, 1H) ; 6,79 (s, 1H) ; 3,58 (s, 3H) ; 3,10 (t, 2H) ; 2,49 (s, 3H) ; 2,46 (t, 2H) ; 1,99 (quin, 2H).

### Exemple 291

### Acide 5-chloro-1-[[4-(3-pyridinyl)phényl]sulfonyl]-1H-pyrrolo[3,2-b]-pyridine-2-butanoïque

En opérant de façon analogue à l'exemple 2, au départ de l'ester obtenu selon l'exemple 290, on obtient le composé attendu sous forme d'un solide blanc (rendement = 92 %).
F = 141 °C.

### Exemple 292

### Acide 5-chloro-1-[[4-(4-pyridinyl)phényl]sulfonyl]-1H-pyrrolo[3,2-b]-pyridine-2-butanoïque, méthyl ester

En opérant de façon analogue à l'exemple 290, au départ de l'acide 4-pyridineboronique, on obtient le composé attendu sous forme d'un solide blanc (rendement = 70 %).
F = 215°C.

### Exemple 293

### Acide 5-chloro-1-[[4-(4-pyridinyl)phényl]sulfonyl]-1H-pyrrolo[3,2-b]-pyridine-2-butanoïque

En opérant de façon analogue à l'exemple 2, au départ de l'ester obtenu selon l'exemple 292, on obtient le composé attendu sous forme d'un solide blanc (rendement = 77 %).
F = 174°C.

### Exemple 294

### Acide 5-chloro-1-[(4-fluoro-3-nitrophényl)sulfonyl]-1H-pyrrolo[3,2-b]-pyridine-2-butanoïque, méthyl ester

En opérant de façon analogue à l'exemple 3, au départ de l'ester obtenu selon la préparation XLVI, on obtient le produit attendu sous forme d'un solide jaune (rendement = 88 %).
F = 129-131°C.

### Exemple 295

### Acide 1-[(5-benzothiazolyl)sulfonyl]-5-chloro-1H-pyrrolo[3,2-b]pyridine-2-butanoïque, méthyl ester

On prépare une solution de 0,21 g (0,46 mM) du composé obtenu selon l'exemple 294 dans 2 ml de pyridine et on ajoute, à température ambiante, 0,323 g (4,37 mM) d'hydrogénosulfure de sodium monohydraté en suspension dans 2 ml d'éthylèneglycol, puis 1 ml de pyridine. Le mélange est agité pendant 30 mn à température ambiante, puis versé sur un mélange d'eau et de glace. Le mélange est amené à pH 2 par ajout d'acide chlorhydrique dilué et extrait par de l'acétate d'éthyle. La phase organique obtenue est lavée par une solution d'acide chlorhydrique N, puis à l'eau, séchée sur sulfate de magnésium et concentrée sous pression réduite. Le produit brut obtenu est purifié par chromatographie sur gel de silice en éluant à l'aide d'un mélange toluène/acétate d'éthyle (90/10 puis 75/25 ; v/v). On obtient ainsi 160 mg de l'ester méthylique de l'acide 5-chloro-1-[(4-mercapto-3-nitrophényl)sulfonyl]-1*H*-pyrrolo[3,2-*b*]pyridine-2-butanoïque (huile jaune). Ce composé est remis en solution dans 4 ml d'acide acétique et on ajoute, sous agitation et à température ambiante, 99 mg (1,7 mM) de fer en poudre. Le mélange réactionnel est agité à 70 °C pendant 2 heures puis concentré sous pression réduite. Le résidu d'évaporation est repris dans de l'eau et de l'acétate d'éthyle. La phase aqueuse est séparée et à nouveau extraite par de l'acétate d'éthyle. Les phases organiques rassemblées sont lavées à l'eau, par une solution de carbonate de sodium, puis par de la saumure, séchées sur sulfate de magnésium et concentrées sous pression réduite. On obtient ainsi 150 mg de l'ester méthylique de l'acide 1-[(3-amino-4-mercaptophényl)sulfonyl]-5-chloro-1*H-*pyrrolo[3,2-*b*]pyridine-2-butanoïque (mousse orange, non purifié). Ce produit est repris dans 3 ml d'acide formique et on ajoute sous agitation, 80 mg de poudre de zinc. Le mélange réactionnel est agité pendant 3 heures à 100°C, puis refroidi et versé sur de l'eau. Cette phase aqueuse est amenée à pH 4 par ajout d'une solution de soude N et extraite par de l'acétate d'éthyle. La phase organique obtenue est lavée par une solution de bicarbonate de sodium, par une solution de soude N, puis par de l'eau, séchée sur sulfate de magnésium et concentrée sous pression réduite. Le produit brut obtenu est purifié par chromatographie sur gel de silice en éluant à l'aide d'un mélange toluène/acétate d'éthyle (95/5 puis 80/20 ; v/v). On obtient ainsi le composé attendu sous forme d'une huile incolore (rendement = 47 %).
¹H RMN (500 MHz, DMSOd₆) δ : 9,62 (s, 1H) ; 8,61 (s, 1H) ; 8,53 (d, 1H) ; 8,44 (d, 1H) ; 7,90 (dd, 1H) ; 7,40 (d, 1H) ; 6,78 (s, 1H) ; 3,59 (s, 3H) ; 3,13 (t, 2H) ; 2,46 (t, 2H) ; 1,99 (quin, 2H).

### Exemple 296

### Acide 5-chloro-1-[(5-benzothiazolyl)sulfonyl]-1H-pyrrolo[3,2-b]pyridine-2-butanoïque

En opérant de façon analogue à l'exemple 2, au départ de l'ester obtenu selon l'exemple 295, on obtient le composé attendu sous forme d'un solide blanc (rendement = 24 %).
F = 202-206°C.

### Exemple 297

### Acide 1-[(4-amino-3-nitrophényl)sulfonyl]-5-chloro-1H-pyrrolo[3,2-b]-pyridine-2-butanoïque, méthyl ester

On prépare un mélange de 400 mg (0,874 mM) du composé obtenu selon l'exemple 294 dans 4 ml de dioxanne et on ajoute doucement, sous agitation, 0,31 ml d'ammoniaque à 32 %. Le mélange réactionnel est maintenu sous agitation pendant une heure, à température ambiante, puis on ajoute 20 ml d'acétate d'éthyle. Cette phase organique est lavée à l'eau puis avec de la saumure, séchée sur sulfate de magnésium et concentrée sous pression réduite. On obtient 375 mg du composé attendu sous forme d'un solide jaune (rendement = 95 %).
F = 154-156 °C.

### Exemple 298

### Acide 5-chloro-1-[(3,4-diaminophényl)sulfonyl]-1H-pyrrolo[3,2-b]pyridine-2-butanoïque, méthyl ester

On prépare une solution de 365 mg (0,806 mM) du composé obtenu selon l'exemple 297 dans 5 ml d'acide acétique et on ajoute, sous agitation et à température ambiante, 225 mg (4,03 mM) de fer en poudre. Le mélange réactionnel est agité à 70°C pendant 3 heures puis concentré sous pression réduite. Le résidu d'évaporation est repris dans de l'eau et de l'acétate d'éthyle. La phase aqueuse est séparée et à nouveau extraite par de l'acétate d'éthyle. Les phases organiques rassemblées sont lavées à l'eau, par une solution de carbonate de sodium, puis par de la saumure, séchées sur sulfate de magnésium et concentrées sous pression réduite. On obtient ainsi 313 mg du composé attendu sous forme d'un solide jaune (rendement = 92 %).
F = 162-164°C.

### Exemple 299

### Acide 1-[(6-benzopyrazinyl)sulfonyl]-5-chloro-1H-pyrrolo[3,2-b]pyridine-2-butanoïque, méthyl ester

On prépare un mélange de 150 mg (0,355 mM) du composé obtenu selon l'exemple 298 dans 1,25 ml d'acétonitrile et on ajoute, sous agitation, 0,11 ml (0,95 mM) de glyoxal. Le mélange réactionnel est maintenu sous agitation pendant 14 heures, à 50°C, puis concentré sous pression réduite et repris dans 10 ml d'acétate d'éthyle. Cette phase organique est lavée à l'eau puis avec de la saumure, séchée sur sulfate de magnésium et concentrée sous pression réduite. Le produit brut obtenu est purifié par chromatographie sur gel de silice en éluant à l'aide d'un mélange toluène/acétate d'éthyle (95/5 puis 80/20 ; v/v). On obtient ainsi le composé attendu sous forme d'un solide jaune (rendement = 58 %).
¹H RMN (250 MHz, DMSO) δ : 9,12 (m, 2H) ; 8,65 (d, 1H) ; 8,53 (d, 1H) ; 8,28 (d, 1H) ; 8,09 (dd, 1H) ; 7,41 (d, 1H) ; 6,81 (s, 1H) ; 3,56 (s, 3H) ; 3,12 (t, 2H) ; 2,45 (t, 2H) ; 1,98 (quin, 2H).

### Exemple 300

### Acide 1-[(6-benzopyrazinyl)sulfonyl]-5-chloro-1H-pyrrolo[3,2-b]pyridine-2-butanoïque

En opérant de façon analogue à l'exemple 2, au départ de l'ester obtenu selon l'exemple 299, on obtient le composé attendu sous forme d'un solide beige (rendement = 31 %).
F = 103-106°C.

### Exemple 301

### Acide 5-chloro-1-[(2,3-dihydro-1H-indol-5-yl)sulfonyl]-1H-pyrrolo[3,2-b]pyridine-2-butanoïque

En opérant de façon analogue à l'exemple 2, au départ de l'ester obtenu selon l'exemple 104, on obtient le composé attendu sous forme d'un solide beige (rendement = 37 %).
F = 190°C.

### Exemple 302

### Acide 5-chloro-1-[(2,3-dihydro-1H-indol-5-yl)sulfonyl]-1H-pyrrolo[3,2-b]-pyridine-2-propanoïque

En opérant de façon analogue à l'exemple 2, au départ de l'ester obtenu selon l'exemple 66, on obtient le composé attendu sous forme d'un solide vert (rendement = 19 %).
F = 79°C.

### Exemple 303

### Acide 1-[(2,1,3-benzothiadiazol-4-yl)sulfonyl]-5-chloro-1H-pyrrolo[3,2-b]-pyridine-2-butanoïque, méthyl ester

En opérant de façon analogue à l'exemple 3, au départ de l'ester obtenu selon la préparation XLVII, on obtient le composé attendu sous forme d'un solide marron (rendement = 70 %).
F = 112°C.

### Exemple 304

### Acide 1-[(5-benzopyrazinyl)sulfonyl]-5-chloro-1H-pyrrolo[3,2-b]pyridine-2-butanoïque, méthyl ester

On prépare une solution de 253 mg (0,56 mM) du composé obtenu selon l'exemple 303 dans 17 ml d'acide acétique et on ajoute, sous agitation et à température ambiante, 364 mg (5,6 mM) de zinc en poudre. Le mélange réactionnel est agité à doux reflux pendant 7 heures puis refroidi et filtré sur filtre Whatman. Le filtrat est concentré sous pression réduite. Le solide jaune obtenu (ester méthylique de l'acide 5-chloro-1-[(2,3-diaminophényl)sulfonyl]-1*H-*pyrrolo[3,2-*b*]pyridine-2-butanoïque) est repris dans 25 ml de méthanol et on ajoute 242 mg (1,7 mM) de glyoxal, 0,025 ml d'acide acétique et 46 mg (0,56 mM) d'acétate de sodium. Le mélange réactionnel est chauffé à reflux pendant 3 heures et 30 mn, puis refroidi et dilué avec de l'acétate d'éthyle. La phase organique est lavée à l'eau, puis par de la saumure, séchée sur sulfate de magnésium et concentrée sous pression réduite. Le produit brut obtenu est purifié par chromatographie sur gel de silice en éluant à l'aide d'un mélange cyclohexane/acétate d'éthyle (50/50 ; v/v). On obtient ainsi le composé attendu sous forme d'un solide jaune (rendement = 73 %).
F = 71 °C.

### Exemple 305

### Acide 1-[(5-benzopyrazinyl)sulfonyl]-5-chloro-1H-pyrrolo[3,2-b]pyridine-2-butanoïque

En opérant de façon analogue à l'exemple 2, au départ de l'ester obtenu selon l'exemple 304, on obtient le composé attendu sous forme d'un solide jaune (rendement = 88 %).
F = 74°C.

### Exemple 306

### Acide 1-[(4-bromo-2-chlorophényl)sulfonyl]-5-chloro-1H-pyrrolo[3,2-b]-pyridine-2-butanoïque, méthyl ester

En opérant de façon analogue à l'exemple 277, au départ du chlorure de 4-bromo-2-chlorobenzènesulfonamide, on obtient le composé attendu sous forme d'un solide jaune pâle (rendement = 87 %).
¹H RMN (300 MHz, CDCl₃) δ : 8,17 (d, 1H) ; 7,85 (d, 1H) ; 7,66 (d, 1H) ; 7,60 (dd, 1H) ; 7, 18 (d, 1H) ; 6,59 (s, 1H) ; 3,67 (s, 3H) ; 2,89 (t, 2H) ; 2,39 (t, 2H) ; 2,02 (quin, 2H).

### Exemple 307

### Acide 1-[[2-chloro-4-(diméthylamino)phényl]sulfonyl]-5-chloro-1H-pyrrolo-[3,2-b]pyridine-2-butanoïque, méthyl ester

En opérant de façon analogue à l'exemple 275, au départ du composé obtenu selon l'exemple 306, on obtient le composé attendu sous forme d'un solide blanc (rendement = 36 %).
F = 123-124°C.

### Exemple 308

### Acide 1-[[2-chloro-4-(diméthylamino)phényl]sulfonyl]-5-chloro-1H-pyrrolo-[3,2-b]pyridine-2-butanoïque

En opérant de façon analogue à l'exemple 2, au départ de l'ester obtenu selon l'exemple 307, on obtient le composé attendu sous forme d'un solide blanc (rendement = 67 %).
F = 208°C.

### Exemple 309

### Acide 1-[(2-chloro-4-méthylphényl)sulfonyl]-5-chloro-1H-pyrrolo[3,2-b]-pyridine-2-butanoïque, méthyl ester

En opérant de façon analogue à l'exemple 277, au départ du chlorure de 2-chloro-4-méthylbenzènesulfonamide, on obtient le composé attendu sous forme d'un solide blanc (rendement = 74 %).
F = 88-89°C.

### Exemple 310

### Acide 1-[(2-chloro-4-méthylphényl)sulfonyl]-5-chloro-1H-pyrrolo[3,2-b]-pyridine-2-butanoïque

En opérant de façon analogue à l'exemple 2, au départ de l'ester obtenu selon l'exemple 309, on obtient le composé attendu sous forme d'un solide blanc (rendement = 82 %).
F = 168-169°C.

Les composés selon l'invention décrits ci-dessus ont été reportés dans le tableau suivant:

**TABLEAU I**

| | | | | | | | |
|---|---|---|---|---|---|---|---|
| 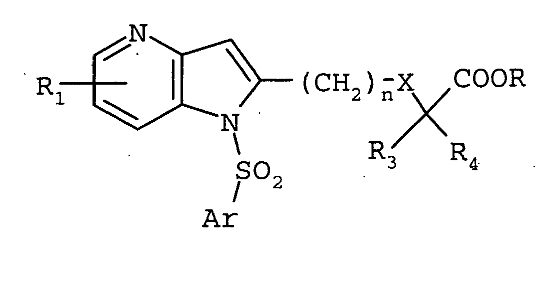 | | | | | | | |

| Ex | R₁ | X^{(*)} ls | R₃ | R₄ | n | Ar | R |
|---|---|---|---|---|---|---|---|
| 1 | 5-Cl | | H | H | 1 | 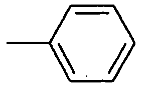 | CH₃ |
| 2 | 5-Cl | ls | H | H | 1 | 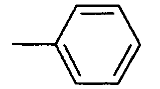 | H |
| 3 | 5-Cl | ls | H | H | 2 | 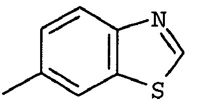 | CH₃ |
| 4 | 5-Cl | ls | H | H | 2 | 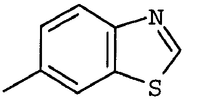 | H |
| 5 | 5-Cl | O | CH₃ | H | 1 | 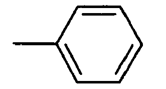 | C₂H₅ |
| 6 | 5-Cl | O | CH₃ | H | 1 | 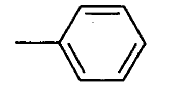 | H |
| 7 | 5-Cl | O | CH₃ | CH₃ | 1 | 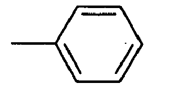 | CH₃ |
| 8 | 5-Cl | O | CH₃ | CH₃ | 1 | 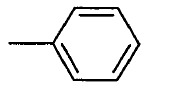 | H |
| 9 | 5-Cl | ls | H | H | 2 | 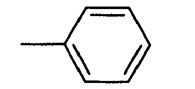 | CH₃ |
| 10 | 5-Cl | ls | H | H | 2 | 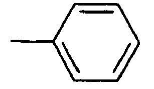 | H |
| 11 | 5-Cl | ls | CH₃ | CH₃ | 1 | 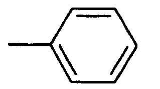 | CH₃ |
| 12 | 5-Cl | ls | CH₃ | CH₃ | 1 | 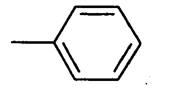 | H |
| 13 | 5-CF₃ | O | CH₃ | H | 1 | 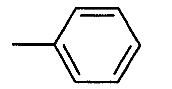 | C₂H₅ |
| 14 | 5-CF₃ | O | CH₃ | H | 1 | 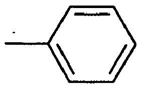 | H |
| 15 | 5-CF₃ | O | CH₃ | CH₃ | 1 | 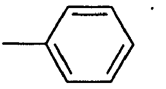 | CH₃ |
| 16 | 5-CF₃ | O | CH₃ | CH₃ | 1 | 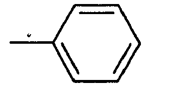 | H |
| 17 | 5-CF₃ | ls | H | H | 1 | 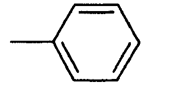 | CH₃ |
| 18 | 5-CF₃ | ls | H | H | 1 | 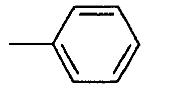 | H |
| 19 | 5-CF₃ | ls | H | H | 2 | 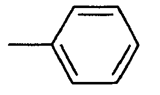 | CH₃ |
| 20 | 5-CF₃ | ls | H | H | 2 | 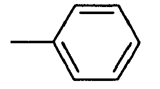 | H |
| 21 | 5-Cl | ls | H | H | 2 | 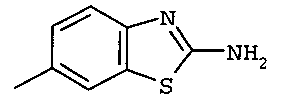 | CH₃ |
| 22 | 5-Cl | ls | H | H | 2 | 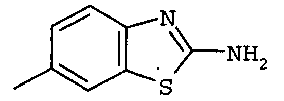 | H |
| 23 | 5-Cl | ls | H | H | 2 | 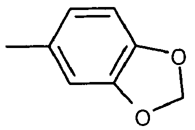 | CH₃ |
| 24 | 5-Cl | ls | H | H | 2 | 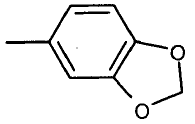 | H |
| 25 | 5-Cl | ls | H | H | 2 | 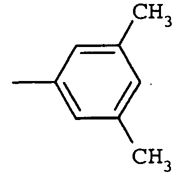 | CH₃ |
| 26 | 5-Cl | ls | H | H | 2 | 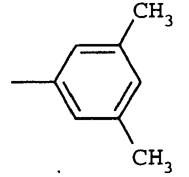 | H |
| 27 | 5-Cl | ls | H | H | 2 | 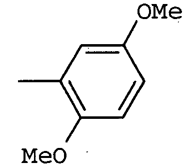 | CH₃ |
| 28 | 5-Cl | ls | H | H | 2 | 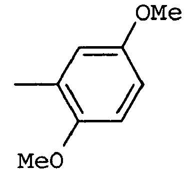 | H |
| 29 | 5-Cl | ls | H | H | 2 | 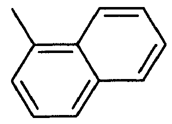 | CH₃ |
| 30 | 5-Cl | ls | H | H | 2 | 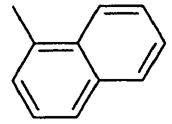 | H |
| 31 | 5-Cl | ls | H | H | 2 | 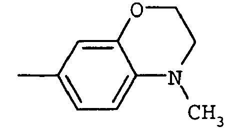 | CH₃ |
| 32 | 5-Cl | ls | H | H | 2 | 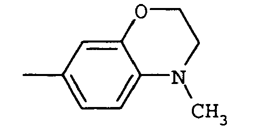 | H |
| 33 | 5-Cl | ls | H | H | 1 | 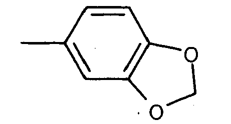 | CH₃ |
| 34 | 5-Cl | ls | H | H | 1 | 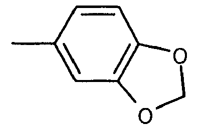 | H |
| 35 | 5-Cl | ls | H | H | 1 | 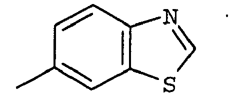 | CH₃ |
| 36 | 5-Cl | ls | H | H | 1 | 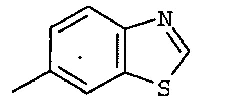 | H |
| 37 | 5-Cl | ls | H | H | 1 | 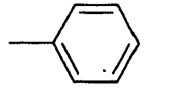 | Na |
| 38 | 5-Cl | O | CH₃ | H | 1 | 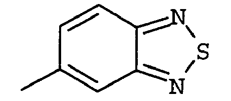 | C₂H₅ |
| 39 | 5-Cl | O | CH₃ | H | 1 | 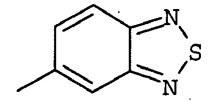 | H |
| 40 | 5-Cl | O | CH₃ | H | 1 | 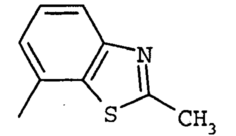 | C₂H₅ |
| 41 | 5-Cl | O | CH₃ | H | 1 | 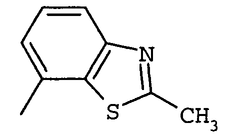 | H |
| 42 | 5-Cl | O | CH₃ | CH₃ | 1 | 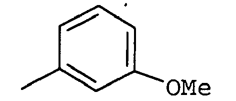 | CH₃ |
| 43 | 5-Cl | O | CH₃ | CH₃ | 1 | 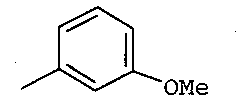 | H |
| 44 | 5-Cl | O | CH₃ | H | 1 | 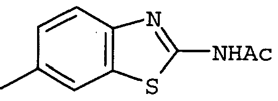 | C₂H₅ |
| 45 | 5-Cl | O | CH₃ | H | 1 | 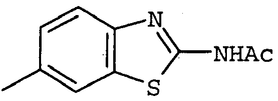 | H |
| 46 | 5-Cl | O | CH₃ | H | 1 | 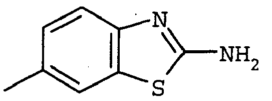 | C₂H₅ |
| 47 | 5-Cl | O | CH₃ | H | 1 | 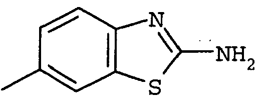 | H |
| 48 | 5-CF₃ | O | CH₃ | CH₃ | 1 | 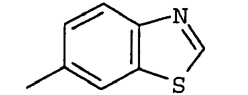 | CH₃ |
| 49 | 5-CF₃ | O | CH₃ | CH₃ | 1 | 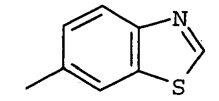 | H |
| 50 | 5-Cl | O | CH₃ | H | 1 | 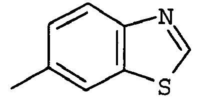 | C₂H₅ |
| 51 | 5-Cl | O | CH₃ | H | 1 | 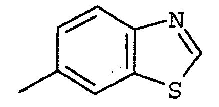 | H |
| 52 | 5-CF₃ | O | CH₃ | H | 1 | 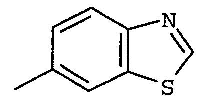 | C₂H₅ |
| 53 | 5-CF₃ | O | CH₃ | H | 1 | 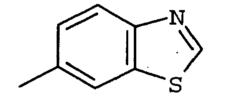 | H |
| 54 | 5-Cl | ls | H | H | 1 | 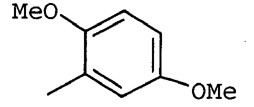 | CH₃ |
| 55 | 5-Cl | ls | H | H | 1 | 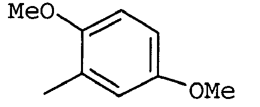 | H |
| 56 | 5-Cl | ls | H | H | 1 | 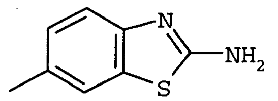 | CH₃ |
| 57 | 5-Cl | ls | H | H | 1 | 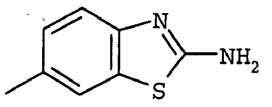 | H |
| 58 | 5-Cl | ls | H | H | 1 | 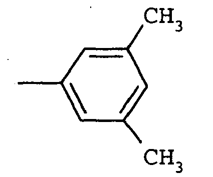 | CH₃ |
| 59 | 5-Cl | ls | H | H | 1 | 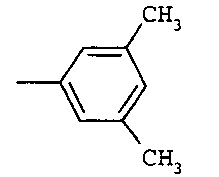 | H |
| 60 | 5-Cl | ls | H | H | 1 | 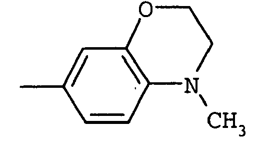 | CH₃ |
| 61 | 5-Cl | ls | H | H | 1 | 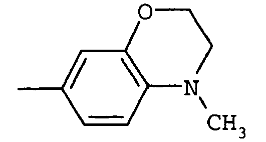 | H |
| 62 | 5-Cl | ls | H | H | 1 | 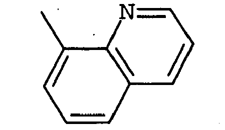 | CH₃ |
| 63 | 5-Cl | ls | H | H | 1 | 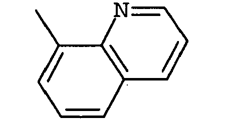 | H |
| 64 | 5-Cl | ls | H | H | 1 | 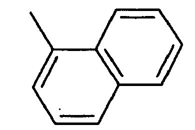 | CH₃ |
| 65 | 5-Cl | ls | H | H | 1 | 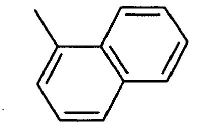 | H |
| 66 | 5-Cl | ls | H | H | 1 | 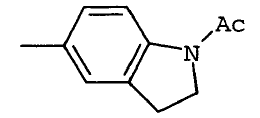 | CH₃ |
| 67 | 5-Cl | ls | H | H | 1 | 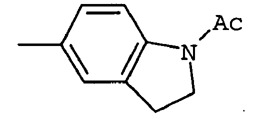 | H |
| 68 | 5-Cl | ls | H | H | 1 | 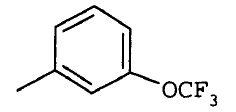 | CH₃ |
| 69 | 5-Cl | ls | H | H | 1 | 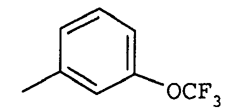 | H |
| 70 | 5-CF₃ | ls | H | H | 1 | 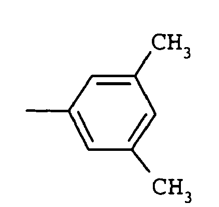 | CH₃ |
| 71 | 5-CF₃ | ls | H | H | 1 | 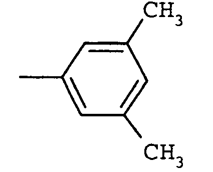 | H |
| 72 | 5-CF₃ | ls | H | H | 1 | 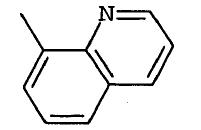 | CH₃ |
| 73 | 5-CF₃ | ls | H | H | 1 | 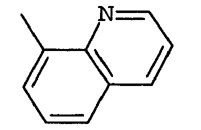 | H |
| 74 | 5-CF₃ | ls | H | H | 1 | 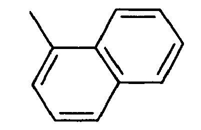 | CH₃ |
| 75 | 5-CF₃ | ls | H | H | 1 | 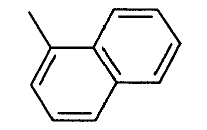 | H |
| 76 | 5-Cl | ls | H | H | 2 | 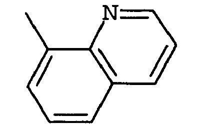 | CH₃ |
| 77 | 5-Cl | ls | H | H | 2 | 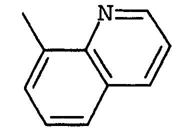 | H |
| 78 | 5-Cl | ls | H | H | 2 | 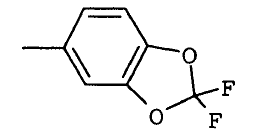 | CH₃ |
| 79 | 5-Cl | ls | H | H | 2 | 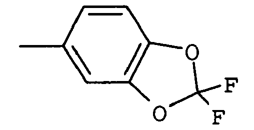 | H |
| 80 | 5-Cl | ls | H | H | 2 | 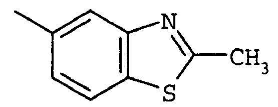 | CH₃ |
| 81 | 5-Cl | ls | H | H | 2 | 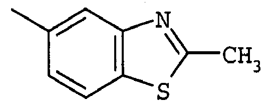 | H |
| 82 | 5-Cl | ls | H | H | 2 | 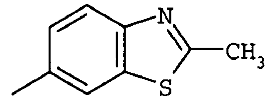 | CH₃ |
| 83 | 5-Cl | ls | H | H | 2 | 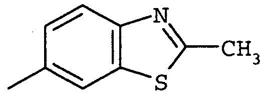 | H |
| 84 | 5-Cl | ls | H | H | 2 | 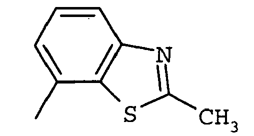 | CH₃ |
| 85 | 5-Cl | ls | H | H | 2 | 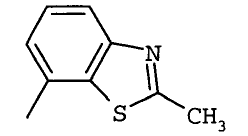 | H |
| 86 | 5-Cl | ls | H | H | 2 | 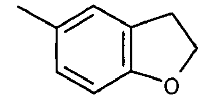 | CH₃ |
| 87 | 5-Cl | ls | H | H | 2 | 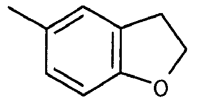 | H |
| 88 | 5-Cl | ls | H | H | 2 | 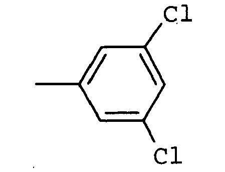 | CH₃ |
| 89 | 5-Cl | ls | H | H | 2 | 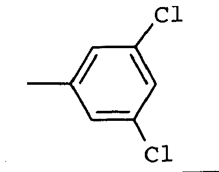 | H |
| 90 | 5-Cl | ls | H | H | 2 | 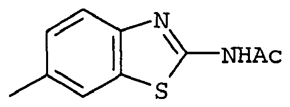 | CH₃ |
| 91 | 5-Cl | ls | H | H | 2 | 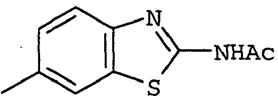 | H |
| 92 | 5-Cl | ls | H | H | 2 | 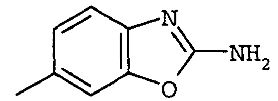 | CH₃ |
| 93 | 5-Cl | ls | H | H | 2 | 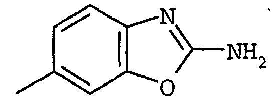 | H |
| 94 | 5-Cl | ls | H | H | 2 | 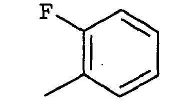 | CH₃ |
| 95 | 5-Cl | ls | H | H | 2 | 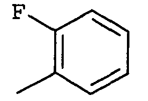 | H |
| 96 | 5-Cl | ls | H | H | 2 | 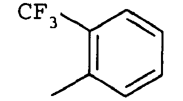 | CH₃ |
| 97 | 5-Cl | ls | H | H | 2 | 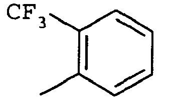 | H |
| 98 | 5-Cl | ls | H | H | 2 | 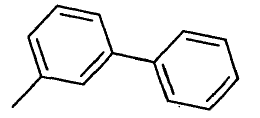 | CH₃ |
| 99 | 5-Cl | ls | H | H | 2 | 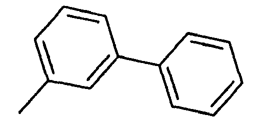 | H |
| 100 | 5-Cl | ls | H | H | 2 | 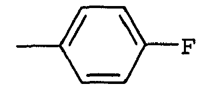 | CH₃ |
| 101 | 5-Cl | ls | H | H | 2 | 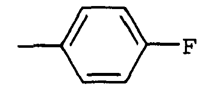 | H |
| 102 | 5-Cl | ls | H | H | 2 | 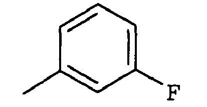 | CH₃ |
| 103 | 5-Cl | ls | H | H | 2 | 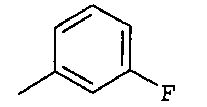 | H |
| 104 | 5-Cl | ls | H | H | 2 | 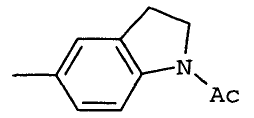 | CH₃ |
| 105 | 5-Cl | ls | H | H | 2 | 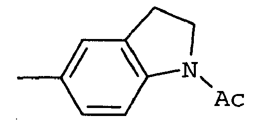 | H |
| 106 | 5-Cl | ls | H | H | 2 | 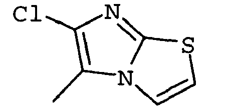 | CH₃ |
| 107 | 5-Cl | ls | H | H | 2 | 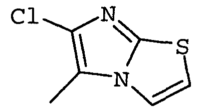 | H |
| 108 | 5-Cl | ls | H | H | 2 | 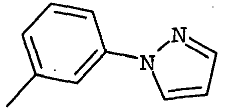 | CH₃ |
| 109 | 5-Cl | ls | H | H | 2 | 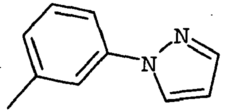 | H |
| 110 | 5-Cl | ls | H | H | 2 | 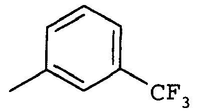 | CH₃ |
| 111 | 5-Cl | ls | H | H | 2 | 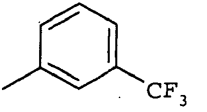 | H |
| 112 | 5-Cl | ls | H | H | 2 | 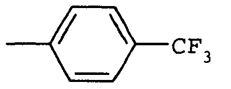 | CH₃ |
| 113 | 5-Cl | ls | H | H | 2 | 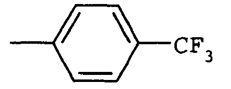 | H |
| 114 | 5-Cl | ls | H | H | 2 | 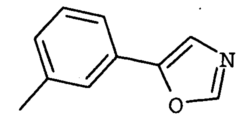 | CH₃ |
| 115 | 5-Cl | ls | H | H | 2 | 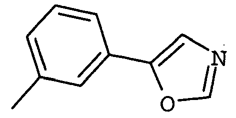 | H |
| 116 | 5-Cl | ls | H | H | 2 | 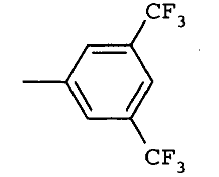 | CH₃ |
| 117 | 5-Cl | ls | H | H | 2 | 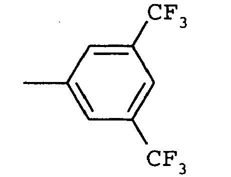 | H |
| 118 | 5-Cl | ls | H | H | 2 | 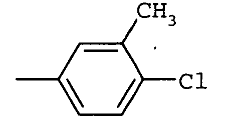 | CH₃ |
| 119 | 5-Cl | ls | H | H | 2 | 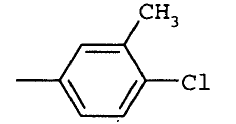 | H |
| 120 | 5-Cl | ls | H | H | 2 | 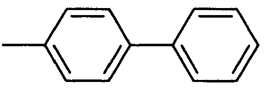 | CH₃ |
| 121 | 5-Cl | ls | H | H | 2 | 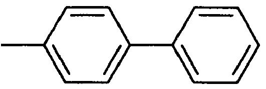 | H |
| 122 | 5-Cl | ls | H | H | 2 | 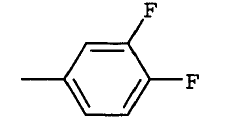 | CH₃ |
| 123 | 5-Cl | ls | H | H | 2 | 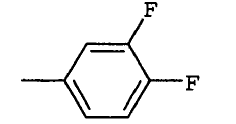 | H |
| 124 | 5-Cl | ls | H | H | 2 | 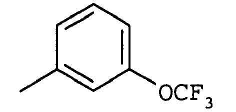 | CH₃ |
| 125 | 5-Cl | ls | H | H | 2 | 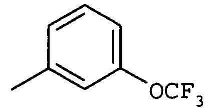 | H |
| 126 | 5-Cl | ls | H | H | 2 | 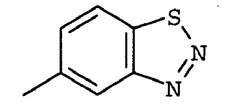 | CH₃ |
| 127 | 5-Cl | ls | H | H | 2 | 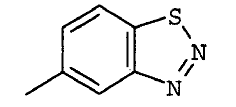 | H |
| 128 | 5-Cl | ls | H | H | 2 | 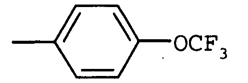 | CH₃ |
| 129 | 5-Cl | ls | H | H | 2 | 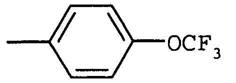 | H |
| 130 | 5-Cl | ls | H | H | 2 | 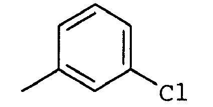 | CH₃ |
| 131 | 5-Cl | ls | H | H | 2 | 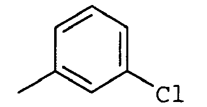 | H |
| 132 | 5-Cl | ls | H | H | 2 | 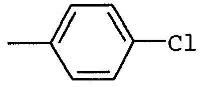 | CH₃ |
| 133 | 5-Cl | ls | H | H | 2 | 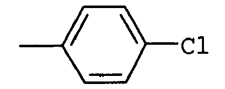 | H |
| 134 | 5-Cl | ls | H | H | 2 | 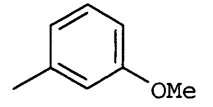 | CH₃ |
| 135 | 5-Cl | ls | H | H | 2 | 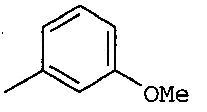 | H |
| 136 | 5-Cl | ls | H | H | 2 | 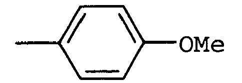 | CH₃ |
| 137 | 5-Cl | ls | H | H | 2 | 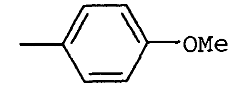 | H |
| 138 | 5-Cl | ls | H | H | 2 | 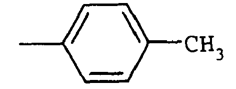 | CH₃ |
| 139 | 5-Cl | ls | H | H | 2 | 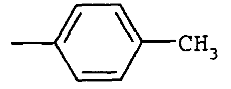 | H |
| 140 | 5-Cl | ls | H | H | 2 | 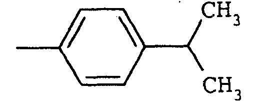 | CH₃ |
| 141 | 5-Cl | ls | H | H | 2 | 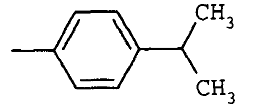 | H |
| 142 | 5-Cl | ls | H | H | 2 | 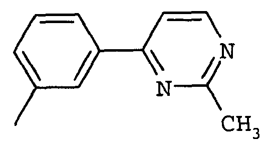 | CH₃ |
| 143 | 5-Cl | ls | H | H | 2 | 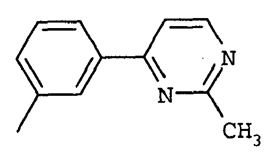 | H |
| 144 | 5-Cl | ls | H | H | 2 | 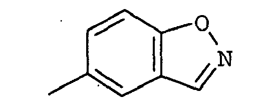 | CH₃ |
| 145 | 5-Cl | ls | H | H | 2 | 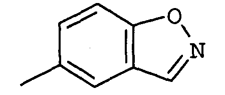 | H |
| 146 | 5-Cl | ls | H | H | 2 | 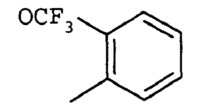 | CH₃ |
| 147 | 5-Cl | ls | H | H | 2 | 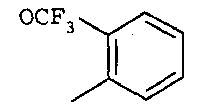 | H |
| 148 | 5-Cl | ls | H | H | 2 | 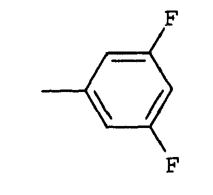 | CH₃ |
| 149 | 5-Cl | ls | H | H | 2 | 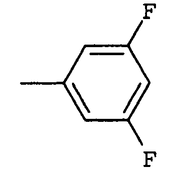 | H |
| 150 | 5-Cl | ls | H | H | 2 | 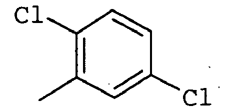 | CH₃ |
| 151 | 5-Cl | ls | H | H | 2 | 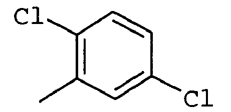 | H |
| 152 | 5-Cl | ls | H | H | 2 | 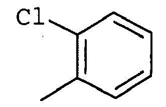 | CH₃ |
| 153 | 5-Cl | ls | H | H | 2 | 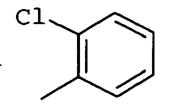 | H |
| 154 | 5-Cl | ls | H | H | 2 | 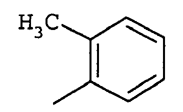 | CH₃ |
| 155 | 5-Cl | ls | H | H | 2 | 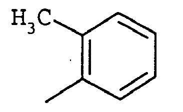 | H |
| 156 | 5-Cl | ls | H | H | 2 | 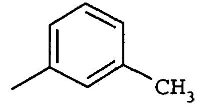 | CH₃ |
| 157 | 5-Cl | ls | H | H | 2 | 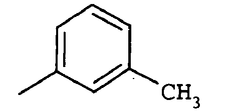 | H |
| 158 | 5-Cl | ls | H | H | 2 | 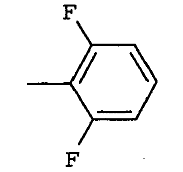 | CH₃ |
| 159 | 5-Cl | ls | H | H | 2 | 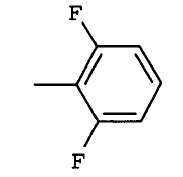 | H |
| 160 | 5-Cl | ls | H | H | 2 | 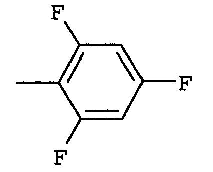 | CH₃ |
| 161 | 5-Cl | ls | H | H | 2 | 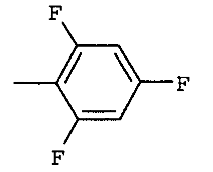 | H |
| 162 | 5-Cl | ls | H | H | 2 | 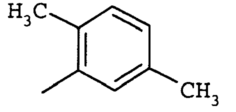 | CH₃ |
| 163 | 5-Cl | ls | H | H | 2 | 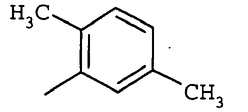 | H |
| 164 | 5-Cl | ls | H | H | 2 | 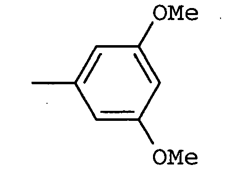 | CH₃ |
| 165 | 5-Cl | ls | H | H | 2 | 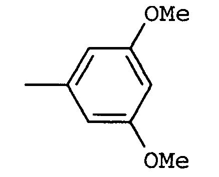 | H |
| 166 | 5-Cl | ls | H | H | 2 | 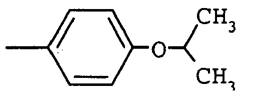 | CH₃ |
| 167 | 5-Cl | ls | H | H | 2 | 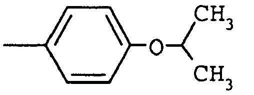 | H |
| 168 | 5-Cl | ls | H | H | 2 | 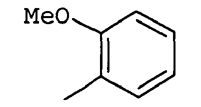 | CH₃ |
| 169 | 5-Cl | ls | H | H | 2 | 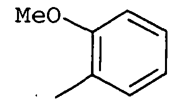 | H |
| 170 | 5-Cl | ls | H | H | 2 | 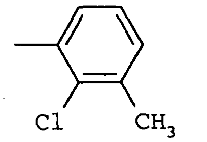 | CH₃ |
| 171 | 5-Cl | ls | H | H | 2 | 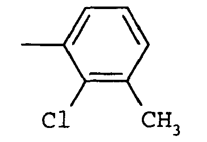 | H |
| 172 | 5-Cl | ls | H | H | 2 | 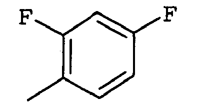 | CH₃ |
| 173 | 5-Cl | ls | H | H | 2 | 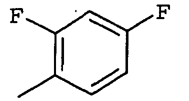 | H |
| 174 | 5-Cl | ls | H | H | 2 | 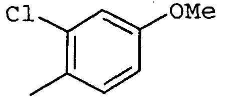 | CH₃ |
| 175 | 5-Cl | ls | H | H | 2 | 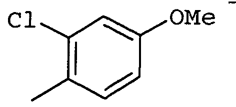 | H |
| 176 | 5-Cl | ls | H | H | 2 | 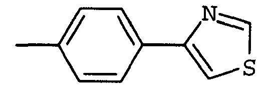 | CH₃ |
| 177 | 5-Cl | ls | H | H | 2 | 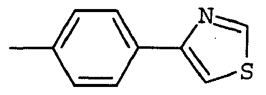 | H |
| 178 | 5-Cl | ls | H | H | 2 | 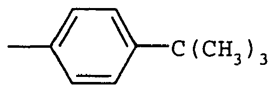 | CH₃ |
| 179 | 5-Cl | ls | H | H | 2 | 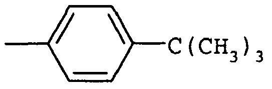 | H |
| 180 | 5-Cl | ls | H | H | 2 | 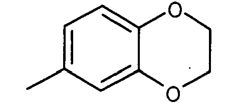 | CH₃ |
| 181 | 5-Cl | ls | H | H | 2 | 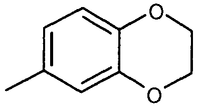 | H |
| 182 | 5-Cl | ls | H | H | 2 | 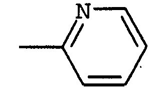 | CH₃ |
| 183 | 5-Cl | ls | H | H | 2 | 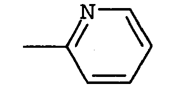 | H |
| 184 | 5-Cl | ls | H | H | 2 | 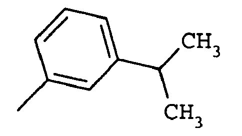 | CH₃ |
| 185 | 5-Cl | ls | H | H | 2 | 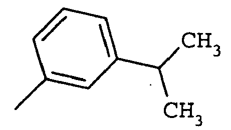 | H |
| 186 | 5-Cl | ls | H | H | 2 | 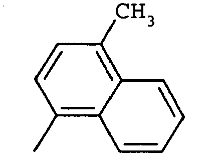 | CH₃ |
| 187 | 5-Cl | ls | H | H | 2 | 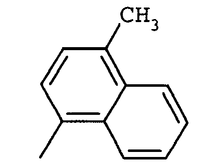 | H |
| 188 | 5-CF₃ | ls | H | H | 2 | 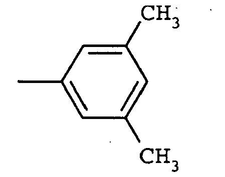 | CH₃ |
| 189 | 5-CF₃ | ls | H | H | 2 | 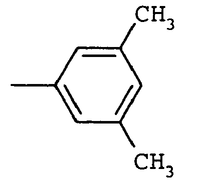 | H |
| 190 | 5-Cl | ls | H | H | 2 | 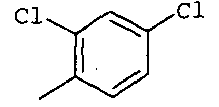 | CH₃ |
| 191 | 5-Cl | ls | H | H | 2 | 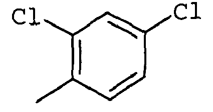 | H |
| 192 | 5-Cl | ls | H | H | 2 | 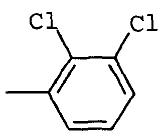 | CH₃ |
| 193 | 5-Cl | ls | H | H | 2 | 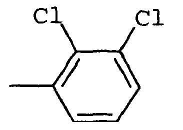 | H |
| 194 | 5-Cl | ls | H | H | 2 | 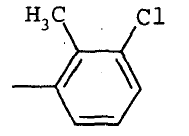 | CH₃ |
| 195 | 5-Cl | ls | H | H | 2 | 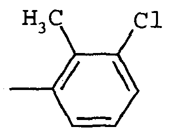 | H |
| 196 | 5-Cl | ls | H | H | 2 | 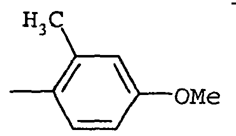 | CH₃ |
| 197 | 5-Cl | ls | H | H | 2 | 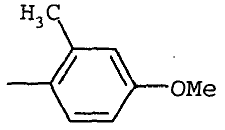 | H |
| 198 | 5-CF₃ | ls | H | H | 2 | 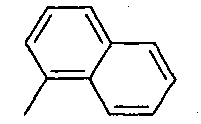 | CH₃ |
| 199 | 5-CF₃ | ls | H | H | 2 | 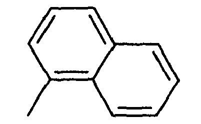 | H |
| 200 | 5-CF₃ | ls | H | H | 2 | 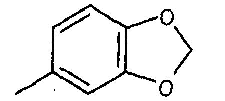 | CH₃ |
| 201 | 5-CF₃ | ls | H | H | 2 | 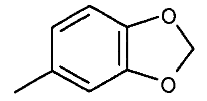 | H |
| 202 | 5-CF₃ | ls | H | H | 2 | 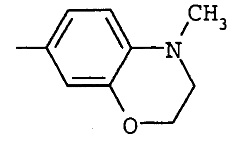 | CH₃ |
| 203 | 5-CF₃ | ls | H | H | 2 | 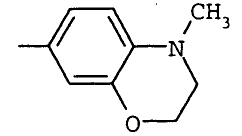 | H |
| 204 | 5-CF₃ | ls | H | H | 2 | 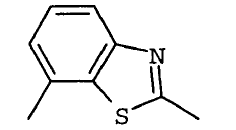 | CH₃ |
| 205 | 5-CF₃ | ls | H | H | 2 | 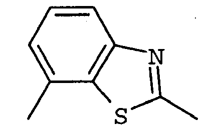 | H |
| 206 | 5-CF₃ | ls | H | H | 2 | 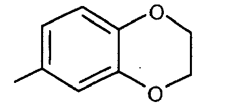 | CH₃ |
| 207 | 5-CF₃ | ls | H | H | 2 | 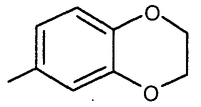 | H |
| 208 | 5-Cl | ls | H | H | 3 | 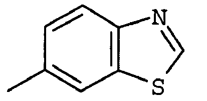 | CH₃ |
| 209 | 5-Cl | ls | H | H | 3 | 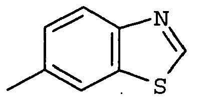 | H |
| 210 | 5-Cl | O | CH₃ | CH₃ | 1 | 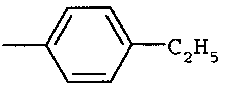 | CH₃ |
| 211 | 5-Cl | O | CH₃ | CH₃ | 1 | 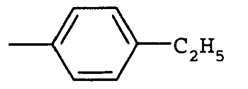 | H |
| 212 | 5-Cl | O | CH₃ | CH₃ | 1 | 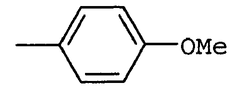 | CH₃ |
| 213 | 5-Cl | O | CH₃ | CH₃ | 1 | 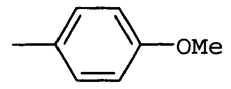 | H |
| 214 | 5-Cl | O | CH₃ | CH₃ | 1 | 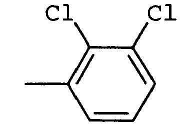 | CH₃ |
| 215 | 5-Cl | O | CH₃ | CH₃ | 1 | 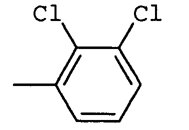 | H |
| 216 | 5-Cl | O | CH₃ | CH₃ | 1 | 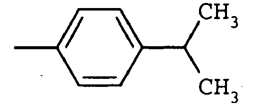 | CH₃ |
| 217 | 5-Cl | O | CH₃ | CH₃ | 1 | 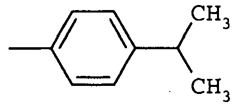 | H |
| 218 | 5-Cl | O | CH₃ | CH₃ | 1 | 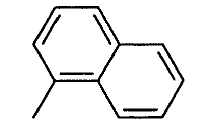 | CH₃ |
| 219 | 5-Cl | O | CH₃ | CH₃ | 1 | 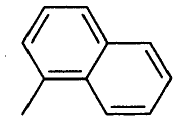 | H |
| 220 | 5-Cl | O | CH₃ | CH₃ | 1 | 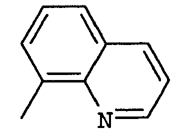 | CH₃ |
| 221 | 5-Cl | O | CH₃ | CH₃ | 1 | 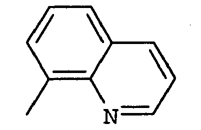 | H |
| 222 | 5-Cl | O | CH₃ | CH₃ | 1 | 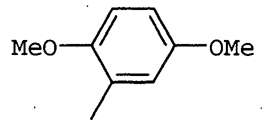 | CH₃ |
| 223 | 5-Cl | O | CH₃ | CH₃ | 1 | 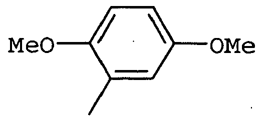 | H |
| 224 | 5-Cl | O | CH₃ | CH₃ | 1 | 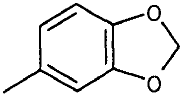 | CH₃ |
| 225 | 5-Cl | O | CH₃ | CH₃ | 1 | 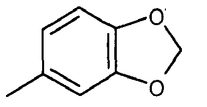 | H |
| 226 | 5-Cl | O | CH₃ | CH₃ | 1 | 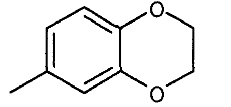 | CH₃ |
| 227 | 5-Cl | O | CH₃ | CH₃ | 1 | 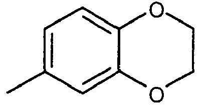 | H |
| 228 | 5-Cl | O | CH₃ | CH₃ | 1 | 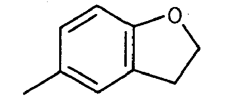 | CH₃ |
| 229 | 5-Cl | O | CH₃ | CH₃ | 1 | 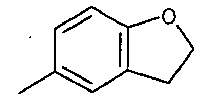 | H |
| 230 | 5-Cl | O | CH₃ | CH₃ | 1 | 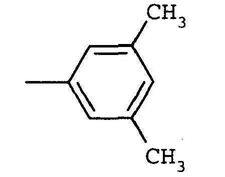 | CH₃ |
| 231 | 5-Cl | O | CH₃ | CH₃ | 1 | 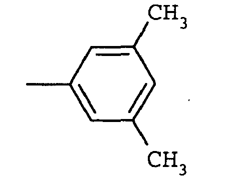 | H |
| 232 | 5-Cl | O | CH₃ | CH₃ | 1 | 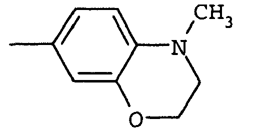 | CH₃ |
| 233 | 5-Cl | O | CH₃ | CH₃ | 1 | 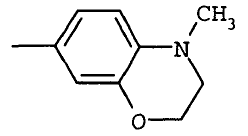 | H |
| 234 | 5-Cl | O | CH₃ (S) | H | 1 | 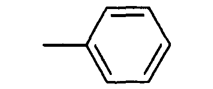 | C₂H₅ |
| 235 | 5-Cl | O | CH₃ (S) | H | 1 | 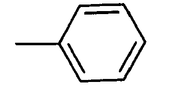 | H |
| 236 | 5-Cl | O | CH₃ (R) | H | 1 | 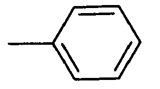 | CH₃ |
| 237 | 5-Cl | O | CH₃ (R) | H | 1 | 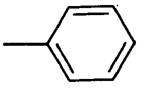 | H |
| 238 | 5-CF₃ | O | CH₃ (S) | H | 1 | 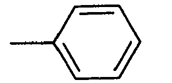 | C₂H₅ |
| 239 | 5-CF₃ | O | CH₃ (S) | H | 1 | 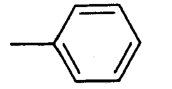 | H |
| 240 | 5-CF₃ | O | CH₃ (R) | H | 1 | 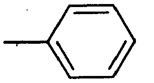 | CH₃ |
| 241 | 5-CF₃ | O | CH₃ (R) | H | 1 | 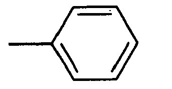 | H |
| 242 | 5-CH₃ | ls | H | H | 1 | 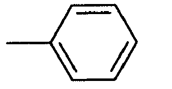 | CH₃ |
| 243 | 5-CH₃ | ls | H | H | 1 | 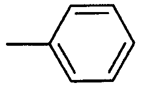 | H |
| 244 | 5-OMe | ls | H | H | 1 | 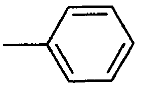 | CH₃ |
| 245 | 5-OMe | ls | H | H | 1 | 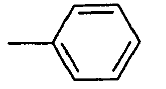 | H |
| 246 | 5-OMe | ls | H | H | 2 | 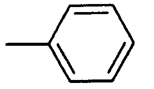 | CH₃ |
| 247 | 5-OMe | ls | H | H | 2 | 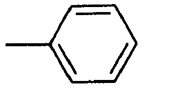 | H |
| 248 | 5-CH₃ | ls | H | H | 2 | 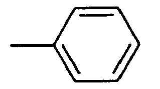 | CH₃ |
| 249 | 5-CH₃ | ls | H | H | 2 | 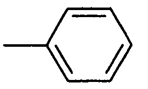 | H |
| 250 | 5-CH₃ | ls | H | H | 2 | 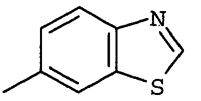 | CH₃ |
| 251 | 5-CH₃ | ls | H | H | 2 | 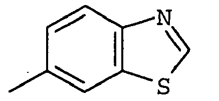 | H |
| 252 | 5-Cl | ls | H | H | 2 | 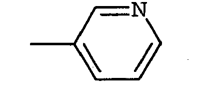 | CH₃ |
| 253 | 5-Cl | ls | H | H | 2 | 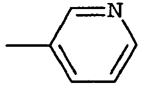 | H |
| 254 | 5-Cl | ls | H | H | 2 | 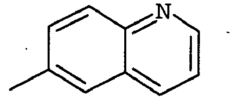 | CH₃ H |
| 255 | 5-Cl | ls | H | H | 2 | 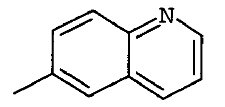 | |
| 256 | 5-Cl | ls | H | H | 3 | 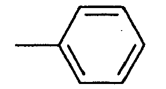 | CH₃ |
| 257 | 5-Cl | ls | H | H | 3 | 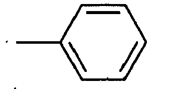 | H |
| 258 | 5-Cl | ls | H | H | 3 | 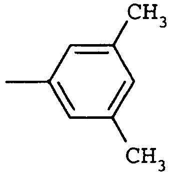 | CH₃ |
| 259 | 5-Cl | ls | H | H | 3 | 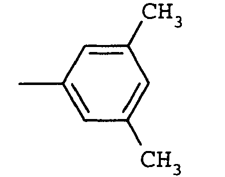 | H |
| 260 | 5-Cl | ls | CH₃ | CH₃ | 2 | 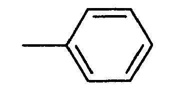 | H |
| 261 | 5-Cl | ls | CH₃ | CH₃ | 2 | 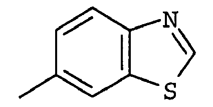 | H |
| 262 | 5-CF₃ | ls | H | H | 2 | 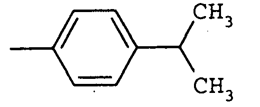 | CH₃ |
| 263 | 5-CF₃ | ls | H | H | 2 | 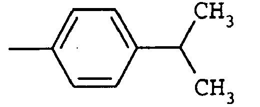 | H |
| 264 | 5-CF₃ | ls | H | H | 2 | 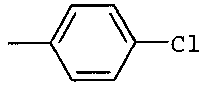 | CH₃ |
| 265 | 5-CF₃ | ls | H | H | 2 | 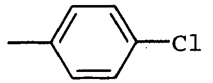 | H |
| 266 | 5-CF₃ | ls | H | H | 2 | 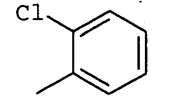 | CH₃ |
| 267 | 5-CF₃ | ls | H | H | 2 | 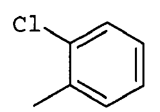 | H |
| 268 | 5-CF₃ | ls | H | H | 2 | 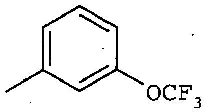 | CH₃ |
| 269 | 5-CF₃ | ls | H | H | 2 | 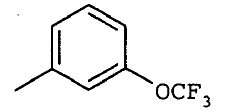 | H |
| 270 | 5-Cl | ls | H | H | 2 | 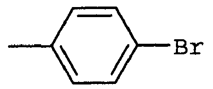 | CH₃ |
| 271 | 5-Cl | ls | H | H | 2 | 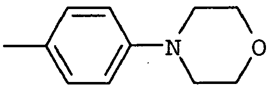 | CH₃ |
| 272 | 5-Cl | ls | H | H | 2 | 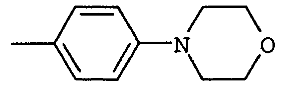 | H |
| 273 | 5-Cl | ls | H | H | 2 | 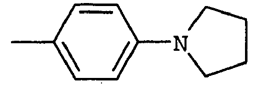 | CH₃ |
| 274 | 5-Cl | ls | H | H | 2 | 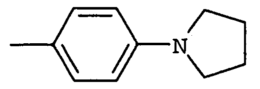 | H |
| 275 | 5-Cl | ls | H | H | 2 | 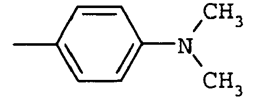 | CH₃ |
| 276 | 5-Cl | ls | H | H | 2 | 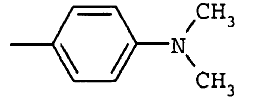 | H |
| 277 | 5-Cl | ls | H | H | 2 | 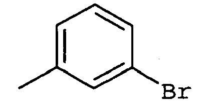 | CH₃ |
| 278 | 5-Cl | ls | H | H | 2 | 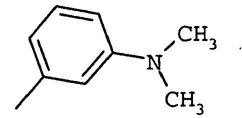 | CH₃ |
| 279 | 5-Cl | ls | H | H | 2 | 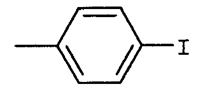 | CH₃ |
| 280 | 5-Cl | ls | H | H | 2 | 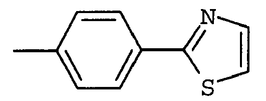 | CH₃ |
| 281 | 5-Cl | ls | H | H | 2 | 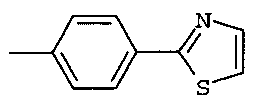 | H |
| 282 | 5-Cl | ls | H | H | 2 | 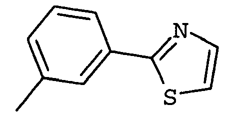 | CH₃ |
| 283 | 5-Cl | ls | H | H | 2 | 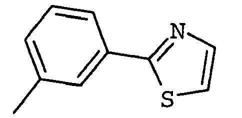 | H |
| 284 | 5-Cl | ls | H | H | 2 | 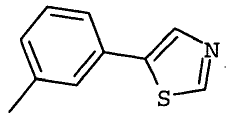 | CH₃ |
| 285 | 5-Cl | ls | H | H | 2 | 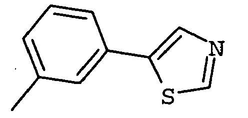 | H |
| 286 | 5-Cl | ls | H | H | 2 | 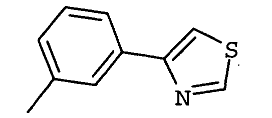 | CH₃ |
| 287 | 5-Cl | ls | H | H | 2 | 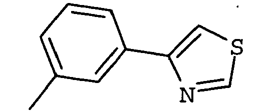 | H |
| 288 | 5-Cl | ls | H | H | 2 | 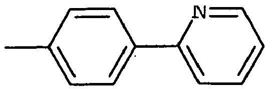 | CH₃ |
| 289 | 5-Cl | ls | H | H | 2 | 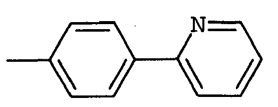 | H |
| 290 | 5-Cl | ls | H | H | 2 | 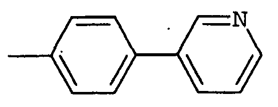 | CH₃ |
| 291 | 5-Cl | ls | H | H | 2 | 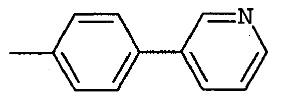 | H |
| 292 | 5-Cl | ls | H | H | 2 | 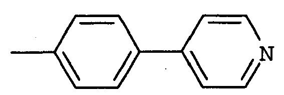 | CH₃ |
| 293 | 5-Cl | ls | H | H | 2 | 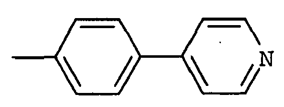 | H |
| 294 | 5-Cl | ls | H | H | 2 | 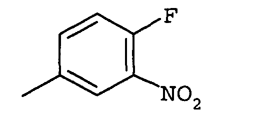 | CH₃ |
| 295 | 5-Cl | ls | H | H | 2 | 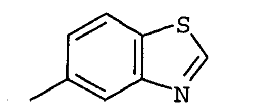 | CH₃ |
| 296 | 5-Cl | ls | H | H | 2 | 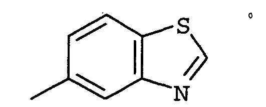 | H |
| 297 | 5-Cl | ls | H | H | 2 | 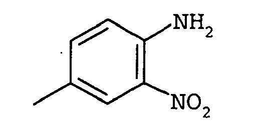 | CH₃ |
| 298 | 5-Cl | ls | H | H | 2 | 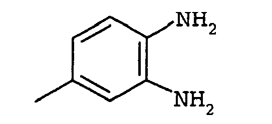 | CH₃ |
| 299 | 5-Cl | ls | H | H | 2 | 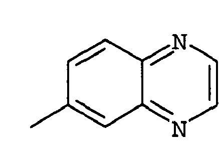 | CH₃ |
| 300 | 5-Cl | ls | H | H | 2 | 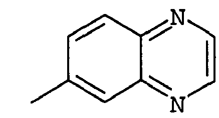 | H |
| 301 | 5-Cl | ls | H | H | 2 | 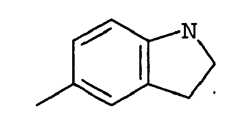 | H |
| 302 | 5-Cl | ls | H | H | 1 | 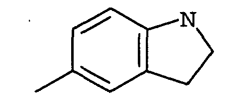 | H |
| 303 | 5-Cl | ls | H | H | 2 | 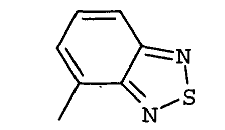 | CH₃ |
| 304 | 5-Cl | ls | H | H | 2 | 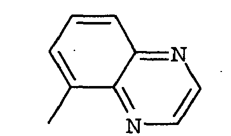 | CH₃ |
| 305 | 5-Cl | ls | H | H | 2 | 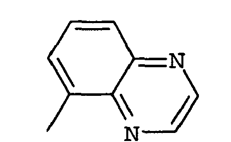 | H |
| 306 | 5-Cl | ls | H | H | 2 | 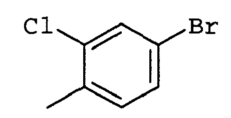 | CH₃ |
| 307 | 5-Cl | ls | H | H | 2 | 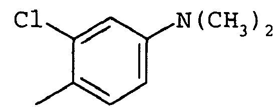 | CH₃ |
| 308 | 5-Cl | ls | H | H | 2 | 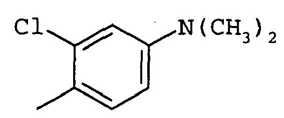 | H |
| 309 | 5-Cl | ls | H | H | 2 | 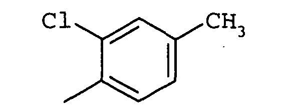 | CH₃ |
| 310 | 5-Cl | ls | H | H | 2 | 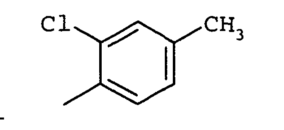 | H |

| | | | | | | | |
|---|---|---|---|---|---|---|---|
| * « ls » signifie : liaison simple et Ac représente le groupe acétyle | | | | | | | |

### Activité pharmacologique

Les composés de l'invention ont été soumis à des tests biologiques de façon à évaluer leur potentiel à traiter ou prévenir certaines pathologies. Dans un premier temps, on a mesuré l'aptitude des composés à se comporter en activateur des récepteurs nucléaires PPAR.

Un test de transactivation est utilisé comme test de screening primaire. Des cellules Cos-7 sont transfectées avec un plasmide exprimant une chimère d'un récepteur murin ou humain PPAR-Gal4 (récepteur PPARα-Gal4 ou PPARδ-Gal4 ou PPARγ-Gal4) et d'un plasmide rapporteur 5Gal4pGL3 TK Luc. Les transfections sont réalisées à l'aide d'un agent chimique (Jet PEI).

Les cellules transfectées sont distribuées dans des plaques 384 puits et laissées au repos pendant 24 heures.

Au temps 24 heures le milieu de culture est changé. Les produits à tester sont ajoutés (concentration finale comprise entre 10⁻⁴ et 3.10⁻¹⁰ M) dans le milieu de culture. Après une nuit d'incubation, l'expression de luciférase est mesurée après addition de « SteadyGlo » selon les instructions du fabricant (Promega). L'acide fénofibrique à 10⁻⁵ M (PPARα agoniste), le GW501516 à 10⁻⁸ M (PPARδ agoniste) et la rosiglitazone à 10⁻⁶ M (PPARγ agoniste) sont utilisés comme références.

Les résultats sont exprimés en taux d'induction (nombre de fois) comparativement au niveau basal en pourcentage d'activité de la référence adéquate (référence = 100 %). Les courbes effet-concentration et les EC₅₀ sont calculées à l'aide du logiciel Assay Explorer (MDL).

Les composés selon l'invention présentent un taux d'induction allant jusqu'à 319 % (PPARα), 151 % (PPARδ) et 114 % (PPARγ). Les composés selon l'invention présentent une EC 50 comprise entre 4 nM et 1500 nM.

Une seconde série de tests a été pratiquée avec les composés selon l'invention, dans le but de confirmer l'activité déduite de leur affinité pour les récepteurs précédemment cités. Ce test consiste en une mesure de la β-oxydation sur cellules d'origine hépatique humaine HuH7 et cellules d'origine musculaire murine C2C12 après différenciation en myotubes.

Les cellules sont ensemencées dans des boîtes de Pétri comportant un puits central. Les produits sont ajoutés dans le milieu de culture et incubés pendant 48 heures à différentes concentrations. Après 22 heures d'incubation, de l'oléate radiomarqué au C14 (oléate 1-C14) est ajouté dans le milieu de culture. La réaction de β-oxydation est arrêtée 2 heures plus tard par addition d'acide perchlorique à 40 %.

Le CO₂ dégagé au cours de l'oxydation de l'oléate est piégé par une solution de KOH puis compté.

Chaque essai est réalisé trois fois.

Les résultats sont exprimés en % de variation par rapport aux boîtes contrôles (boîtes sans composés).

Suivant cet essai, les composés selon l'invention augmentent la β-oxydation jusqu'à + 145 % à une concentration de 10 µM sur cellules HuH7. La β-oxydation est également augmentée de 70 % en présence, par exemple, du composé selon l'exemple 10 utilisé à une concentration de 10 µM lors d'un essai sur cellules C2C12.

Certains composés selon l'invention ont été testés sur un modèle de souris db/db afin de confirmer leur potentiel de principe actif. Le protocole de l'essai est le suivant :

Des souris mâles C57BL/Ks-db homozygotes (souris db/db), âgées de 11-13 semaines à l'initiation des études, sont réparties par groupe de 9-10 animaux. Les produits sont administrés par voie orale, 1 fois par jour pendant 5 jours. Un groupe de souris reçoit le véhicule seul (solution de méthylcellulose à 0,5 ou 1 %). Un prélèvement sanguin est réalisé au sinus rétro-orbitaire avant traitement et 4 heures après le dernier gavage.

Après centrifugation, le sérum est collecté et les taux de cholestérol, triglycérides et glucose sont mesurés à l'aide d'un analyseur multiparamétrique avec des kits commerciaux.

Les résultats sont exprimés en % de variation au jour final par rapport au groupe témoin. A titre d'exemple, quelques résultats obtenus avec les composés selon l'invention sont reportés dans le tableau suivant, en comparaison avec le fénofibrate ou la rosiglitazone :

### Activité pharmacologique

| Composé | Dose (mg/kg) | Glucose | Triglycérides | Cholestérol |
|---|---|---|---|---|
| Fénofibrate | 100 | -9 | -7 | +32 |
| Rosiglitazone | 3 | -41 | -52 | -30 |
| Ex 2 | 10 | -36 | -32 | +41 |
| Ex 20 | 10 | -72 | -65 | +13 |
| Ex 4 | 10 | -57 | -45 | +4 |
| Ex 293 | 10 | -24 | -15 | +16 |
| Ex 259 | 10 | -51 | -16 | +32 |

Ces résultats, qui sont en accord avec les modifications attendues d'activateurs des récepteurs nucléaires PPAR, confirment l'intérêt des composés selon l'invention pour leur utilisation en tant que principes actifs de médicaments à usage humain destinés à la prévention ou au traitement des hypertriglycéridémies, des hypercholestérolémies, de l'obésité et, d'une façon plus générale, au rétablissement de paramètres normaux lors d'une perturbation du métabolisme lipidique et glucidique. Les composés selon l'invention trouvent encore leur utilité dans le cas du traitement de la dysfonction endothéliale, de maladies inflammatoires ou de neurodégénérescences.

L'invention concerne également les compositions pharmaceutiques destinées à la prévention ou au traitement des maladies précédemment citées lorsqu'elles contiennent en tant que principe actif au moins l'un des composés de formule I selon l'invention.

Ces compositions pharmaceutiques peuvent être préparées de façon classique, à l'aide d'excipients pharmaceutiquement acceptables afin d'obtenir des formes administrables de préférence par voie orale, par exemple des comprimés ou des gélules.

De façon pratique, en cas d'administration du composé par voie orale, la posologie quotidienne chez l'homme sera de préférence comprise entre 5 et 500 mg.

## Revendications

1. Nouveau dérivé de pyrrolopyridine **caractérisé en ce qu'**il est choisi parmi :
i) les composés de formule : 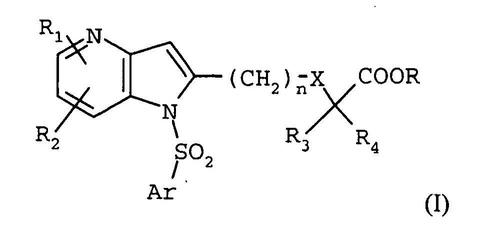 dans laquelle :
R₁ et R₂ représentent chacun indépendamment un atome d'hydrogène, un atome d'halogène, un groupe alkyle en C₁-C₃, alcoxy en C₁-C₄ ou un groupe CF₃,
R₃ et R₄ représentent chacun indépendamment un atome d'hydrogène ou un groupe alkyle en C₁-C₄,
R représente un atome d'hydrogène ou un groupe alkyle en C₁-C₃,
n= 1, 2 ou 3,
X représente une liaison simple ou un atome d'oxygène,
Ar représente un noyau aromatique ou hétéroaromatique choisi parmi les groupes phényle, pyrazolyle, imidazolyle, thiazolyle, oxazolyle, isoxazolyle, furanyle, thiényle, pyrrolyle, pyridinyle, biphényle, naphtyle, 1,2,3,4-tétrahydronaphtalènyle, quinolinyle, isoquinolinyle, 1,2,3,4-tetrahydroquinolinyle, benzimidazolyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle, ou benzoxazolyle, éventuellement substitué par un ou plusieurs (par exemple 2 ou 3) substituants choisis parmi les atomes d'halogène, les groupes alkyle en C₁-C₆, alcoxy en C₁-C₄, trifluorométhyle, trifluorométhoxy, nitro, acétyle, acétylamino, dialkylamino ou amino, ou les hétérocycles oxazolyle, thiazolyle, pyrazolyle, pyrrolidinyle, pyridinyle, pyrimidinyle, méthyl-pyrimidinyle ou morpholinyle,
ii) leurs sels pharmaceutiquement acceptables.

2. Composé selon la revendication 1, **caractérisé en ce que** Ar représente un noyau aromatique ou hétéroaromatique choisi parmi les groupes phényle, pyridinyle, biphényle, naphtyle, quinolinyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle, ou benzoxazolyle, éventuellement substitué par un ou plusieurs (par exemple 2 ou 3) substituants choisis parmi les atomes d'halogène, les groupes alkyle en C₁-C₆, alcoxy en C₁-C₄, trifluorométhyle, trifluorométhoxy, nitro, acétyle, acétylamino, dialkylamino ou amino, ou les hétérocycles oxazolyle, thiazolyle, pyrazolyle, pyrrolidinyle, pyridinyle, pyrimidinyle, méthyl-pyrimidinyle ou morpholinyle.

3. Composé selon l'une des revendications 1 ou 2, **caractérisé en ce que** R₁ représente un atome de chlore ou un groupe trifluorométhyle.

4. Composé selon l'une des revendications 1 à 3, pour son utilisation en tant que substance pharmacologiquement active.

5. Utilisation d'un composé selon l'une des revendications 1 à 3, pour la fabrication d'un médicament destiné à traiter les hypertriglycéridémies, les hyperlipidémies, les hypercholestérolémies, les dyslipidémies, l'insulino-résistance, le diabète et l'obésité.

6. Utilisation d'un composé selon l'une des revendications 1 à 3, pour la fabrication d'un médicament destiné à traiter la dysfonction endothéliale.

7. Utilisation d'un composé selon l'une des revendications 1 à 3, pour la fabrication d'un médicament destiné au traitement des maladies cardiovasculaires, des maladies inflammatoires et des neurodégénérescences comme notamment la maladie d'Alzheimer ou la maladie de Parkinson.

8. Composition pharmaceutique **caractérisée en ce qu'**elle contient au moins un composé selon l'une des revendications 1 à 3 en tant que substance active.

9. Procédé de préparation d'un composé selon la revendication 1, **caractérisé en ce qu'**il comprend les étapes consistant à :
a) effectuer une réaction d'halogénation, préférentiellement une iodation, d'une aminopyridine de formule 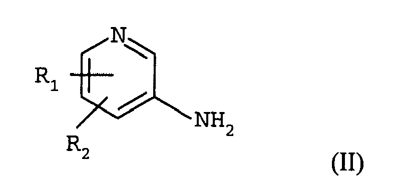 dans laquelle :
R₁ et R₂ représentent chacun indépendamment un atome d'hydrogène, un atome de fluor de brome ou de chlore ou un groupe alkyle en C₁-C₄, alcoxy en C₁-C₄ ou trifluorométhyle, à l'aide d'un agent halogénant, tel que par exemple l'iode en présence de sulfate d'argent ou le dichloroiodate de benzyltriméthylammonium, dans un solvant, à température ambiante, pendant 5 à 24 heures pour obtenir le composé de formule 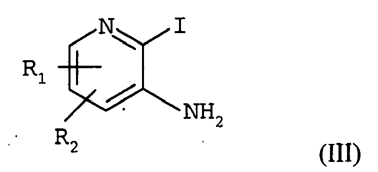
dans laquelle :
R₁ et R₂ conservent la même signification que dans les composés de départ ;
b) faire réagir selon la réaction dite de SONOGASHIRA, le composé de formule III avec un dérivé acétylénique de formule 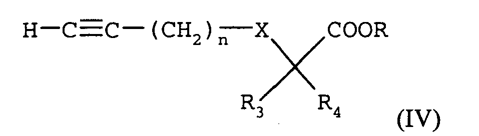 dans laquelle :
n = 1, 2 ou 3 ;
R₃ et R₄ représentent chacun indépendamment un atome d'hydrogène ou un groupe alkyle en C₁-C₄ ;
R représente un groupe alkyle en C₁-C₃ ;
X représente une liaison simple ou un atome d'oxygène ;
en présence d'iodure cuivreux, d'un catalyseur à base de palladium, tel que par exemple le tetrakis(triphénylphosphine)palladium ou le dichloro-bis(triphénylphosphine)palladium, et d'une base organique, dans un solvant, à une température comprise entre 0 et 60°C pendant 2 à 24 heures, pour obtenir le composé de formule 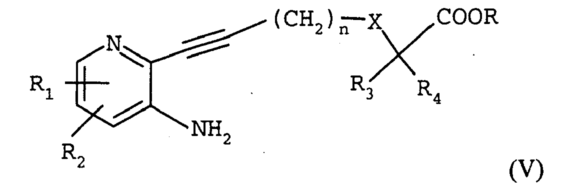
dans laquelle :
R₁, R₂, n, X, R₃, R₄ et R conservent la même signification que dans les composés de départ ;
c) faire réagir le composé de formule V avec un chlorure d'arylsulfonyle de formule 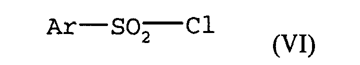 dans laquelle :
Ar représente un noyau aromatique ou hétéroaromatique choisi parmi les groupes phényle, pyrazolyle, imidazolyle, thiazolyle, oxazolyle, isoxazolyle, furanyle, thiényle, pyrrolyle, pyridinyle, biphényle, naphtyle, 1,2,3,4-tétrahydronaphtalènyle, quinolinyle, isoquinolinyle, 1,2,3,4-tetrahydroquinolinyle, benzimidazolyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle, ou benzoxazolyle, éventuellement substitué par un ou plusieurs (par exemple 2 ou 3) substituants choisis parmi les atomes d'halogène, les groupes alkyle en C₁-C₆, alcoxy en C₁-C₄, trifluorométhyle, trifluorométhoxy, nitro, acétyle, acétylamino, dialkylamino ou amino, ou les hétérocycles oxazolyle, thiazolyle, pyrazolyle, pyrrolidinyle, pyridinyle, pyrimidinyle, méthyl-pyrimidinyle ou morpholinyle,
en présence de pyridine, éventuellement dans un solvant, à température ambiante, pendant 10 à 120 mn, pour obtenir le composé de formule 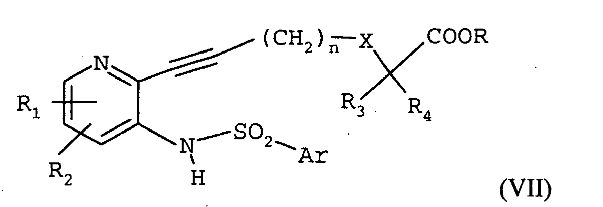
dans laquelle :
R₁, R₂, n, X, R₃, R₄, R et Ar conservent la même signification que dans les composés de départ ;
d) effectuer une cyclisation du composé de formule VII, par exemple par action de l'acétate de cuivre II, dans un solvant, à une température proche de la température de reflux du solvant, pendant 4 à 24 heures, pour obtenir le composé de formule 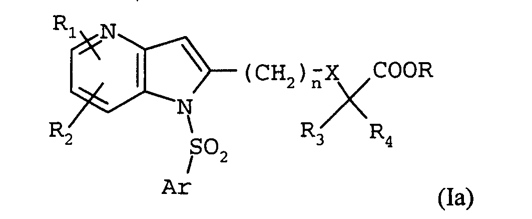 dans laquelle :
R₁, R₂, n, X, R₃, R₄, R et Ar conservent la même signification que dans les composés de départ ;
e) si nécessaire, hydrolyser la fonction ester du composé de formule Ia, par exemple par action d'une base minérale, pour obtenir, après traitement acide, le composé de formule I sous sa forme d'acide libre :

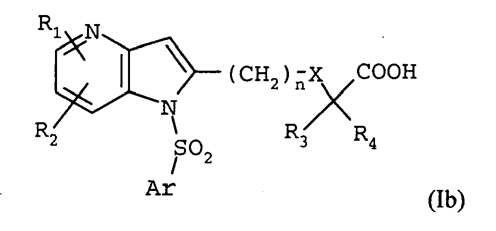

10. Procédé de préparation d'un composé selon la revendication 1, **caractérisé en ce qu'**il comprend les étapes consistant à :
a) faire réagir le composé de formule (III) 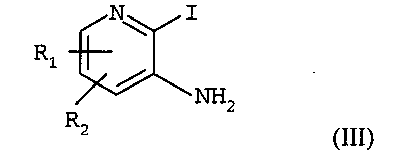 dans laquelle R₁ et R₂ représentent chacun indépendamment un atome d'hydrogène, de chlore ou de fluor ou un groupe alkyle en C₁-C₄, alcoxy en C₁-C₄ ou trifluorométhyle,
avec un chlorure d'arylsulfonyle de formule 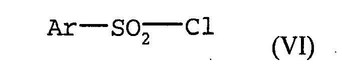 dans laquelle :
Ar représente un noyau aromatique ou hétéroaromatique choisi parmi les groupes phényle, pyrazolyle, imidazolyle, thiazolyle, oxazolyle, isoxazolyle, furanyle, thiényle, pyrrolyle, pyridinyle, biphényle, naphtyle, 1,2,3,4-tétrahydronaphtalènyle, quinolinyle, isoquinolinyle, 1,2,3,4-tetrahydroquinolinyle, benzimidazolyle, benzopyrazinyle, indolyle, 2,3-dihydroindolyle, benzofuranyle, 2,3-dihydrobenzofuranyle, benzothiazolyle, benzothiadiazolyle, benzisoxazolyle, 3,4-dihydro-1,4-benzoxazinyle, 1,3-benzodioxolyle, 2,3-dihydrobenzodioxinyle, imidazothiazolyle, ou benzoxazolyle, éventuellement substitué par un ou plusieurs (par exemple 2 ou 3) substituants choisis parmi les atomes d'halogène, les groupes alkyle en C₁-C₆, alcoxy en C₁-C₄, trifluorométhyle, trifluorométhoxy, nitro, acétyle, acétylamino, dialkylamino ou amino, ou les hétérocycles oxazolyle, thiazolyle, pyrazolyle, pyrrolidinyle, pyridinyle, pyrimidinyle, méthyl-pyrimidinyle ou morpholinyle,
dans un solvant, à température ambiante et pendant 1 à 12 heures, pour obtenir le composé de formule (VIII) 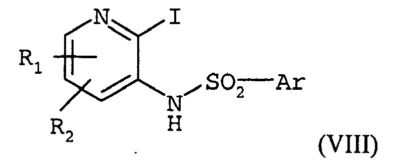
dans laquelle :
R₁, R₂ et Ar conservent la même signification que dans les composés de départ ;
b) faire réagir le composé de formule VIII avec un dérivé acétylénique de formule 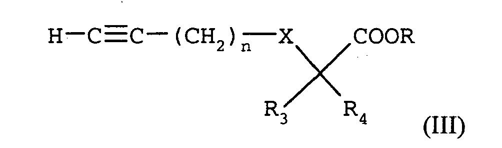 dans laquelle :
n= 1, 2 ou 3 ;
R₃ et R₄ représentent chacun indépendamment un atome d'hydrogène ou un groupe alkyle en C₁-C₄ ;
R représente un groupe alkyle en C₁-C₃ ;
X représente une liaison simple ou un atome d'oxygène ;
dans des conditions analogues à celles décrites pour l'étape b) du procédé selon la revendication 9,
pour obtenir le composé de formule 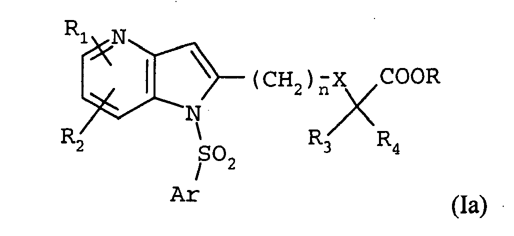 dans laquelle R₁, R₂, n, X, R₃, R₄, R et Ar conservent la même signification que dans les composés de départ ;
c) si nécessaire, hydrolyser la fonction ester du composé de formule Ia, par exemple par action d'une base minérale, pour obtenir, après traitement acide, le composé de formule I sous sa forme d'acide libre :

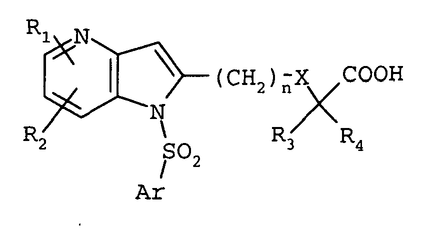

## Claims

1. A novel pyrrolopyridine derivative, **characterized in that** it is chosen from:
i) the compounds of formula: 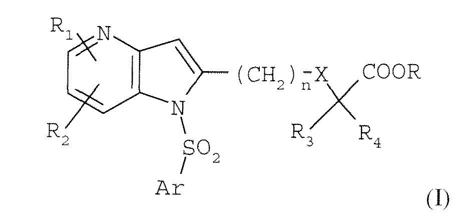 in which:
R₁ and R₂ each independently represent a hydrogen atom, a halogen atom, a C₁-C₃ alkyl or C₁-C₄ alkoxy group or a CF₃ group,
R₃ and R₄ each independently represent a hydrogen atom or a C₁-C₄ alkyl group,
R represents a hydrogen atom or a C₁-C₃ alkyl group,
n = 1, 2 or 3,
X represents a single bond or an oxygen atom,
Ar represents an aromatic or heteroaromatic ring chosen from phenyl, pyrazolyl, imidazolyl, thiazolyl, oxazolyl, isoxazolyl, furyl, thienyl, pyrrolyl, pyridyl, biphenyl, naphthyl, 1,2,3,4-tetrahydronaphthyl, quinolyl, isoquinolyl, 1,2,3,4-tetrahydroquinolyl, benzimidazolyl, benzopyrazinyl, indolyl, 2,3-dihydroindolyl, benzofuryl, 2,3-dihydrobenzofuryl, benzothiazolyl, benzothiadiazolyl, benzisoxazolyl, 3,4-dihydro-1,4-benzoxazinyl, 1,3-benzodioxolyl, 2,3-dihydrobenzodioxinyl, imidazothiazolyl and benzoxazolyl groups, optionally substituted with one or more (for example 2 or 3) substituents chosen from halogen atoms and C₁-C₆ alkyl, C₁-C₄ alkoxy, trifluoromethyl, trifluoromethoxy, nitro, acetyl, acetylamino, dialkylamino and amino groups, or oxazolyl, thiazolyl, pyrazolyl, pyrrolidinyl, pyridyl, pyrimidinyl, methylpyrimidinyl or morpholinyl heteracycles,
ii) pharmaceutically acceptable salts thereof.

2. The compound as claimed in claim 1, **characterized in that** Ar represents an aromatic or heteroaromatic ring chosen from phenyl, pyridyl, biphenyl, naphthyl, quinolyl, benzopyrazinyl, indolyl, 2,3-dihydroindolyl, benzofuryl, 2,3-dihydrobenzofuryl, benzothiazolyl, benzothiadiazolyl, benzisoxazolyl, 3,4-dihydro-1,4-benzoxazinyl, 1,3-benzodioxolyl, 2,3-dihydrobenzodioxinyl, imidazothiazolyl and benzoxazolyl groups, optionally substituted with one or more (for example 2 or 3) substituents chosen from halogen atoms and C₁-C₆ alkyl, C₁-C₄ alkoxy, trifluoromethyl, trifluoromethoxy, nitro, acetyl, acetylamino, dialkylamino and amino groups, or oxazolyl, thiazolyl, pyrazolyl, pyrrolidinyl, pyridyl, pyrimidinyl, methylpyrimidinyl or morpholinyl heterocycles.

3. The compound as claimed in either of claims 1 and 2, **characterized in that** R₁ represents a chlorine atom or a trifluoromethyl group.

4. The compound as claimed in one of claims 1 to 3, for its use as a pharmacologically active substance.

5. The use of a compound as claimed in one of claims 1 to 3 for the manufacture of a medicament for treating hypertriglyceridemia, hyperlipidemia, hypercholesterolemia, dyslipidemia, insulin resistance, diabetes and obesity.

6. The use of a compound as claimed in one of claims 1 to 3 for the manufacture of a medicament for treating endothelial dysfunction.

7. The use of a compound as claimed in one of claims 1 to 3 for the manufacture of a medicament for treating cardiovascular diseases, inflammatory diseases and neurodegenerative diseases especially such as Alzheimer's disease or Parkinson's disease.

8. A pharmaceutical composition, **characterized in that** it contains at least one compound as claimed in one of claims 1 to 3 as active substance.

9. A process for preparing a compound as claimed in claim 1, **characterized in that** it comprises the steps consisting in:
a) performing a halogenation reaction, preferably an iodination reaction, of an aminopyridine of formula 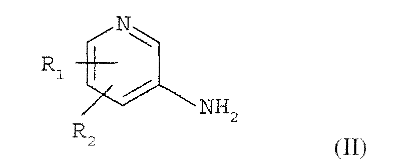 in which:
R₁ and R₂ each independently represent a hydrogen atom, a fluorine, bromine or chlorine atom or a C₁-C₄ alkyl, C₁-C₄ alkoxy or trifluoromethyl group,
using a halogenating agent, such as for instance iodine in the presence of silver sulfate or benzyltrimethylammonium dichloroiodate, in a solvent, at room temperature, for 5 to 24 hours, to obtain the compound of formula 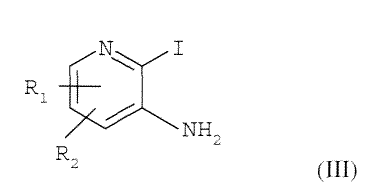
in which:
R₁ and R₂ conserve the same meaning as in the starting compounds;
b) reacting, according to the Sonogashira reaction, the compound of formula III with an acetylenic derivative of formula 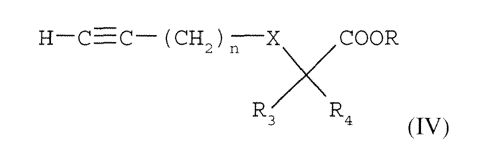 in which:
n = 1, 2 or 3;
R₃ and R₄ each independently represent a hydrogen atom or a C₁-C₄ alkyl group;
R represents a C₁-C₃ alkyl group;
X represents a single bond or an oxygen atom;
in the presence of cuprous iodide, a palladium-based catalyst, for instance tetrakis(triphenylphosphine)palladium or dichlorobis(triphenylphosphine)-palladium, and an organic base, in a solvent, at a temperature of between 0 and 60°C, for 2 to 24 hours, to obtain the compound of formula 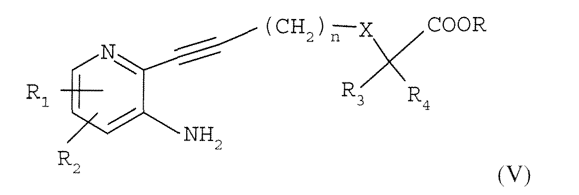
in which:
R₁, R₂, n, X, R₃, R₄ and R conserve the same meaning as in the starting compounds;
c) reacting the compound of formula V with an arylsulfonyl chloride of formula 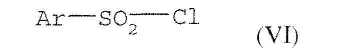 in which:
Ar represents an aromatic or heteroaromatic ring chosen from phenyl, pyrazolyl, imidazolyl, thiazolyl, oxazolyl, isoxazolyl, furyl, thienyl, pyrrolyl, pyridyl, biphenyl, naphthyl, 1,2,3.4-tetrahydronaphthyl, quinolyl, isoquinolyl 1,2,3,4-tetrahydroquinolyl, benzimidazolyl, benzopyrazinyl, indolyl, 2,3-dihydroindolyl, benzofuryl, 2,3-dihydrobenzofuryl, benzothiazolyl, benzothiadiazolyl, benzisoxazolyl, 3,4-dihydro-1,4-benzoxazinyl, 1,3-benzodioxolyl, 2,3-dihydrobenzodioxinyl, imidazothiazolyl and benzoxazolyl groups, optionally substituted with one or more (for example 2 or 3) substituents chosen from halogen atoms and C₁-C₆ alkyl, C₁-C₄ alkoxy, trifluoromethyl, trifluoromethoxy, nitro, acetyl, acetylamino, dialkylamino and amino groups, or oxazolyl, thiazolyl, pyrazolyl, pyrrolidinyl, pyridyl, pyrimidinyl, methylpyrimidinyl or morpholinyl heterocycles,
in the presence of pyridine, optionally in a solvent, at room temperature, for 10 to 120 minutes, to obtain the compound of formula 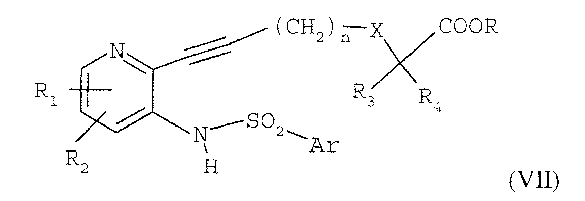
in which:
R₁, R₂, n, X, R₃, R₄, R and Ar conserve the same meaning as in the starting compounds;
d) performing a cyclization of the compound of formula VII, for example via the action of copper II acetate, in a solvent, at a temperature close to the reflux temperature of the solvent, for 4 to 24 hours, to obtain the compound of formula 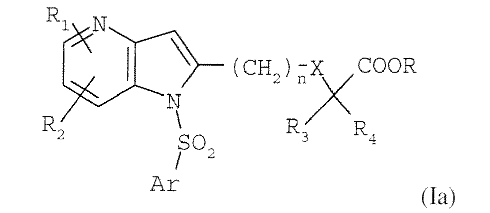 in which:
R₁, R₂, n, X, R₃, R₄, R and Ar conserve the same meaning as in the starting compounds;
e) if necessary, hydrolyzing the ester function of the compound of formula Ia, for example via the action of a mineral base, to obtain, after acid treatment, the compound of formula I in the form of the free acid:

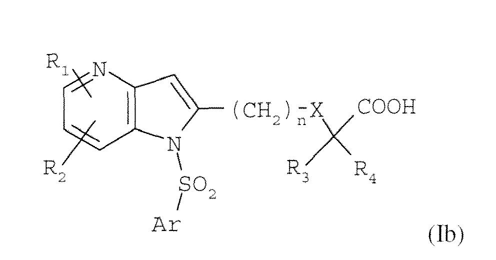

10. A process for preparing a compound as claimed in claim 1, **characterized in that** it comprises the steps consisting in:
a) reacting the compound of formula (III) 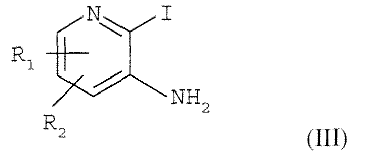 in which R₁ and R₂ each independently represent a hydrogen, chlorine or fluorine atom or a C₁-C₄ alkyl, C₁-C₄ alkoxy or trifluoromethyl group,
with an arylsulfonyl chloride of formula 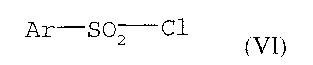 in which:
Ar represents an aromatic or heteroaromatic ring chosen from phenyl, pyrazolyl, imidazolyl, thiazolyl, oxazolyl, isoxazolyl, furyl, thienyl, pyrrolyl, pyridyl, biphenyl, naphthyl, 1,2,3,4-tetrahydronaphthyl, quinolyl, isoquinolyl, 1,2,3,4-tetrahydroquinolyl, benzimidazolyl, benzopyrazinyl, indolyl, 2,3-dihydroindolyl, benzofuryl, 2,3-dihydrobenzofuryl, benzothiazolyl, benzothiadiazolyl, benzisoxazolyl, 3,4-dihydro-1,4-benzoxazinyl, 1,3-benzodioxolyl, 2,3-dihydrobenzodioxinyl, imidazothiazolyl and benzoxazolyl groups, optionally substituted with one or more (for example 2 or 3) substituents chosen from halogen atoms and C₁-C₆ alkyl, C₁-C₄ alkoxy, trifluoromethyl, trifluoromethoxy, nitro, acetyl, acetylamino, dialkylamino and amino groups, or oxazolyl, thiazolyl, pyrazolyl, pyrrolidinyl, pyridyl, pyrimidinyl, methylpyrimidinyl or morpholinyl heterocycles,
in a solvent, at room temperature and for 1 to 12 hours, to obtain the compound of formula (VIII) 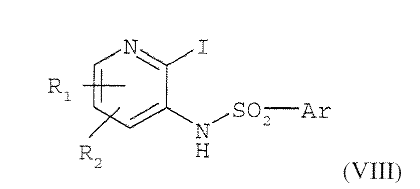
in which:
R₁, R₂ and Ar conserve the same meaning as in the starting compounds;
b) reacting the compound of formula VIII with an acetylenic derivative of formula 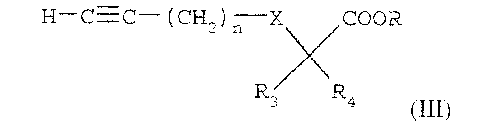 in which:
n = 1, 2 or 3;
R₃ and R₄ each independently represent a hydrogen atom or a C₁-C₄ alkyl group;
R represents a C₁-C₃ alkyl group;
X represents a single bond or an oxygen atom;
under conditions similar to those described for step b) of the process as claimed in claim 9,
to obtain the compound of formula 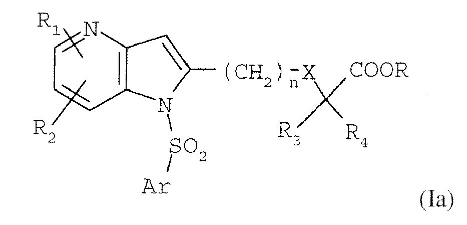 in which R₁, R₂, n, X, R₃, R₄, R and Ar conserve the same meaning as in the starting compounds;
c) if necessary, hydrolyzing the ester function of the compound of formule Ia, for example via the action of a mineral base, to obtain, after acid treatment, the compound of formula I in the form of the free acid:

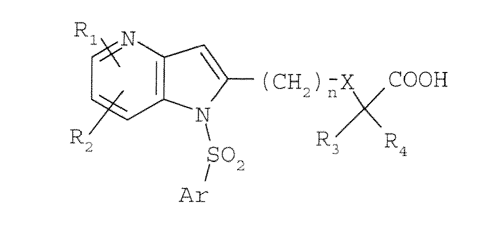

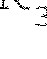

## Patentansprüche

1. Neues Pyrrolopyridinderivat, **dadurch gekennzeichnet, daß** es aus folgendem ausgewählt ist:
i) den Verbindungen der Formel: 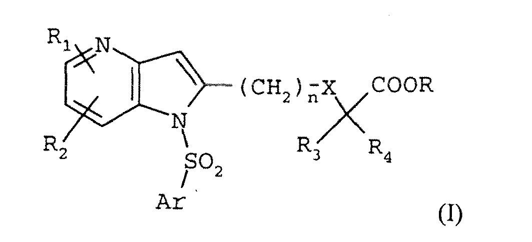 worin:
R₁ und R₂ jeweils unabhängig ein Wasserstoffatom, ein Halogenatom, eine C₁-C₃-Alkyl-, C₁-C₄-Alkoxygruppe oder eine CF₃-Gruppe darstellen,
R₃ und R₄ jeweils unabhängig ein Wasserstoffatom oder eine C₁-C₄-Alkylgruppe darstellen,
R ein Wasserstoffatom oder eine C₁-C₃-Alkylgruppe darstellt,
n = 1, 2 oder 3,
X eine einfache Bindung oder ein Sauerstoffatom darstellt,
Ar einen aromatischen oder heteroaromatischen Kern darstellt, ausgewählt aus den Phenyl-, Pyrazolyl-, Imidazolyl-, Thiazolyl-, Oxazolyl-, Isoxazolyl-, Furanyl-, Thienyl-, Pyrrolyl-, Pyridinyl-, Biphenyl-, Naphtyl-, 1,2,3,4-Tetrahydronaphthalenyl-, Quinolinyl-, Isoquinolinyl-, 1,2,3,4-Tetrahydroquinolinyl-, Benzimidazolyl-, Benzopyrazinyl-, Indolyl-, 2,3-Dihydroindolyl-, Benzofuranyl-, 2,3-Dihydrobenzofuranyl-, Benzothiazolyl-, Benzothiadiazolyl-, Benzisoxazolyl-, 3,4-Dihydro-1,4-benzoxazinyl-, 1,3-Benzodioxolyl-, 2,3-Dihydrobenzodioxinyl-, Imidazothiazolyl- oder Benzoxazolyl-Gruppen, eventuell substituiert durch einen oder mehrere (beispielsweise 2 oder 3) Substituenten, ausgewählt aus den Halogenatomen, den C₁-C₆-Alkyl-, C₁-C₄-Alkoxy-, Trifluormethyl-,
Trifluormethoxy-, Nitro-, Acetyl-, Acetylamino-, Dialkylamino- oder Amino-Gruppen oder den Heterozyklen Oxazolyl, Thiazolyl, Pyrazolyl, Pyrrolidinyl, Pyridinyl, Pyrimidinyl, Methyl-Pyrimidinyl oder Morpholinyl,
ii) ihren pharmazeutisch akzeptablen Salzen.

2. Verbindung nach Anspruch 1, **dadurch gekennzeichnet, daß** Ar einen aromatischen oder heteroaromatischen Kern darstellt, ausgewählt aus den Phenyl-, Pyridinyl-, Biphenyl-, Naphtyl-, Quinolinyl-, Benzopyrazinyl-, Indolyl-, 2,3-Dihydroindolyl-, Benzofuranyl-, 2,3-Dihydrobenzofuranyl-, Benzothiazolyl-, Benzothiadiazolyl-, Benzisoxazolyl-, 3,4-Dihydro-1,4-benzoxazinyl-, 1,3-Benzodioxolyl-, 2,3-Dihydrobenzodioxinyl-, Imidazothiazolyl- oder Benzoxazolyl-Gruppen, eventuell substituiert durch einen oder mehrere (beispielsweise 2 oder 3) Substituenten, ausgewählt aus den Halogenatomen, den C₁-C₆-Alkyl-, C₁-C₄-Alkoxy-, Trifluormethyl-, Trifluormethoxy-, Nitro-, Acetyl-, Acetylamino-, Dialkylamino- oder Amino-Gruppen oder den Heterozyklen Oxazolyl, Thiazolyl, Pyrazolyl, Pyrrolidinyl, Pyridinyl, Pyrimidinyl, Methyl-Pyrimidinyl oder Morpholinyl.

3. Verbindung nach einem der Ansprüche 1 oder 2, **dadurch gekennzeichnet, daß** R₁ ein Chloratom oder eine Trifluormethyl-Gruppe darstellt.

4. Verbindung nach einem der Ansprüche 1 bis 3, für ihre Verwendung als pharmakologisch aktive Substanz.

5. Verwendung einer Verbindung nach einem der Ansprüche 1 bis 3, für die Herstellung eines Medikaments, welches für die Behandlung von Hypertriglyzeridämien, Hyperlipidämien, Hypercholesterolämien, Dyslipidämien, Insulinresistenz, Diabetes und Fettleibigkeit bestimmt ist.

6. Verwendung einer Verbindung nach einem der Ansprüche 1 bis 3, für die Herstellung eines Medikaments, welches für die Behandlung von endothelialer Funktionsstörung bestimmt ist.

7. Verwendung einer Verbindung nach einem der Ansprüche 1 bis 3, für die Herstellung eines Medikaments, welches für die Behandlung von Herz-Kreislauf-Erkrankungen, Entzündungserkrankungen und neurodegenerativen Erkrankungen, wie insbesondere der Alzheimer-Krankheit oder der Parkinson-Krankheit bestimmt ist.

8. Pharmazeutische Zusammensetzung, **dadurch gekennzeichnet, daß** sie wenigstens eine Verbindung nach einem der Ansprüche 1 bis 3 als Wirkstoff enthält.

9. Verfahren zur Herstellung einer Verbindung nach Anspruch 1, **dadurch gekennzeichnet, daß** es die Schritte umfaßt, die darin bestehen:
a) eine Halogenierungs-Reaktion, vorzugsweise eine Jodierung, eines Aminopyridin folgender Formel durchzuführen 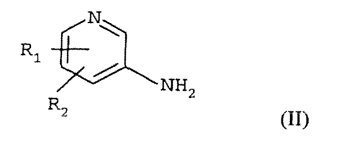 worin:
R₁ und R₂ jeweils unabhängig ein Wasserstoffatom, ein Fluor-, Brom- oder Chloratom oder eine C₁-C₄-Alkyl-, C₁-C₄-Alkoxy- oder Trifluormethyl-Gruppe darstellen,
und zwar mit Hilfe eines Halogenierungsmittels, wie zum Beispiel Jod in Anwesenheit von Silbersulfat oder Benzyltrimethylammonium-dichloriodat, in einem Lösungsmittel, bei Umgebungstemperatur, für 5 bis 24 Stunden, um die Verbindung folgender Formel zu erhalten 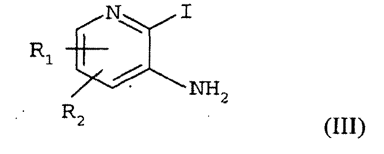
worin:
R₁ und R₂ die gleiche Bedeutung wie bei den Ausgangsverbindungen behalten;
b) die Verbindung der Formel III nach der sogenannten SONOGASHIRA-Reaktion mit einem Acetylenderivat folgender Formel reagieren zu lassen 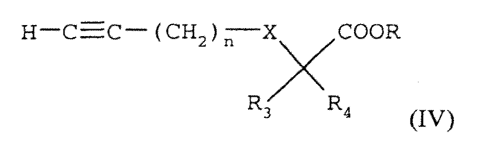 worin:
n = 1, 2 oder 3,
R₃ und R₄ jeweils unabhängig ein Wasserstoffatom oder eine C₁-C₄-Alkylgruppe darstellen,
R eine C₁-C₃-Alkylgruppe darstellt,
X eine einfache Bindung oder ein Sauerstoffatom darstellt,
und zwar in Anwesenheit von Kupfer(I)-iodid, einem Katalysator auf Palladiumbasis, wie zum Beispiel Tetrakis(triphenylphosphin)palladium oder Dichlor-bis(triphenylphosphin)palladium, und einer organischen Base, in einem Lösungsmittel, bei einer Temperatur zwischen 0 und 60°C für 2 bis 24 Stunden, um die Verbindung folgender Formel zu erhalten 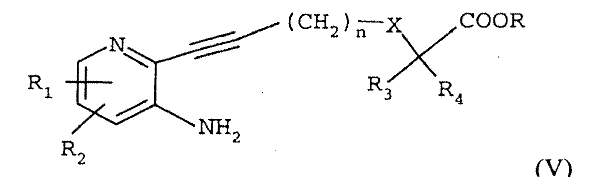
worin:
R₁, R₂, n, X, R₃, R₄ und R die gleiche Bedeutung wie bei den Ausgangsverbindungen behalten,
c) die Verbindung der Formel V mit einem Arylsulfonylchlorid folgender Formel reagieren zu lassen 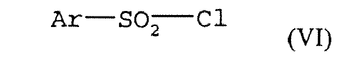 worin:
Ar einen aromatischen oder heteroaromatischen Kern darstellt, ausgewählt aus den Phenyl-, Pyrazolyl-, Imidazolyl-, Thiazolyl-, Oxazolyl-, Isoxazolyl-, Furanyl-, Thienyl-, Pyrrolyl-, Pyridinyl-, Biphenyl-, Naphtyl-, 1,2,3,4-Tetrahydronaphthalenyl-, Quinolinyl-, Isoquinolinyl-, 1,2,3,4-Tetrahydroquinolinyl-, Benzimidazolyl-, Benzopyrazinyl-, Indolyl-, 2,3-Dihydroindolyl-, Benzofuranyl-, 2,3-Dihydrobenzofuranyl-, Benzothiazolyl-, Benzothiadiazolyl-, Benzisoxazolyl-, 3,4-Dihydro-1,4-benzcaxazinyl-, 1,3-Benzodioxolyl-, 2,3-Dihydrobenzodioxinyl-, Imidazothiazolyl- oder Benzoxazolyl-Gruppen, eventuell substituiert durch einen oder mehrere (beispielsweise 2 oder 3) Substituenten, ausgewählt aus den Halogenatomen, den C₁-C₆-Alkyl-, G₁-C₄-Alkoxy-, Trifluormethyl-, Trifluormethoxy-, Nitro-, Acetyl-, Acetylamino-, Dialkylamino- oder Amino-Gruppen oder den Heterozyklen Oxazolyl, Thiazolyl, Pyrazolyl, Pyrrolidinyl, Pyridinyl, Pyrimidinyl, Methyl-Pyrimidinyl oder Morpholinyl,
und zwar in Anwesenheit von Pyridin, eventuell in einem Lösungsmittel, bei Umgebungstemperatur für 10 bis 120 Min., um die Verbindung folgender Formel zu erhalten 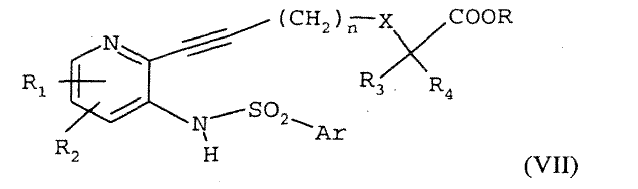
worin:
R₁, R₂, n, X, R₃, R₄, R und Ar die gleiche Bedeutung wie bei den
Ausgangsverbindungen behalten,
d) eine Zyklisierung der Verbindung der Formel VII zu vollziehen, beispielsweise durch Wirkung von Kupfer(II)-acetat, in einem Lösungsmittel, bei einer Temperatur nahe der Rückflußtemperatur des Lösungsmittels, für 4 bis 24 Stunden, um die Verbindung der folgenden Formel zu erhalten 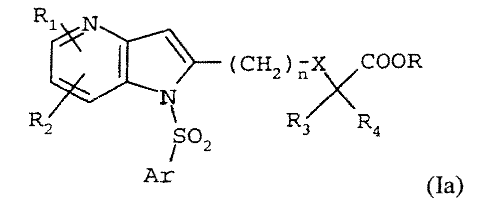 worin:
R₁, R₂, n, X, R₃, R₄, R und Ar die gleiche Bedeutung wie bei den
Ausgangsverbindungen behalten,
e) falls erforderlich die Esterfunktion der Verbindung der Formel la zu hydrolysieren, beispielsweise durch Wirkung einer mineralischen Base, um nach der Säurebehandlung die Verbindung der Formel I in ihrer Form als freie Säure zu erhalten:

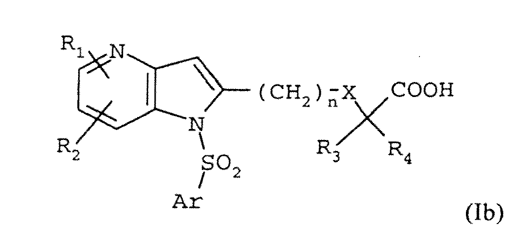

10. Verfahren zur Herstellung einer Verbindung nach Anspruch 1, **dadurch gekennzeichnet, daß** es die Schritte umfaßt, die darin bestehen:
a) die Verbindung der Formel (III) 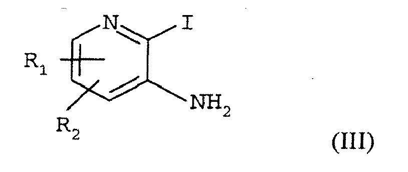 worin R₁ und R₂ jeweils unabhängig ein Wasserstoff-, Chlor- oder Fluoratom oder eine C₁-C₄-Alkyl-, C₁-C₄-Alkoxy- oder Trifluormethyl-Gruppe darstellen,
mit einem Arylsulfonylchlorid folgender Formel reagieren zu lassen 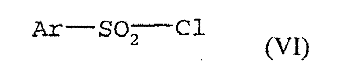 worin:
Ar einen aromatischen oder heteroaromatischen Kern darstellt, ausgewählt aus den Phenyl-, Pyrazolyl-, Imidazolyl-, Thiazolyl-, Oxazolyl-, Isoxazolyl-, Furanyl-, Thienyl-, Pyrrolyl-, Pyridinyl-, Biphenyl-, Naphtyl-, 1,2,3,4-Tetrahydronaphthalenyl-, Quinolinyl-, Isoquinolinyl-, 1,2,3,4-Tetrahydroquinolinyl-, Benzimidazolyl-, Benzopyrazinyl-, Indolyl-, 2,3-Dihydroindolyl-, Benzofuranyl-, 2,3-Dihydrobenzofuranyl-, Benzothiazolyl-, Benzothiadiazolyl-, Benzisoxazolyl-, 3,4-Bihydro-1,4-benzoxazinyl-, 1,3-Benzodioxolyl-, 2,3-Dihydrobenzodioxinyl-, Imidazothiazolyl- oder Benzoxazolyl-Gruppen, eventuell substituiert durch einen oder mehrere (beispielsweise 2 oder 3) Substituenten, ausgewählt aus den Halogenatomen, den C₁-C₆-Alkyl-, C₁-C₄-Alkoxy-, Trifluormethyl-,
Trifluormethoxy-, Nitro-, Acetyl-, Acetylamino-, Dialkylamino- oder Amino-Gruppen oder den Heterozyklen Oxazolyl, Thiazolyl, Pyrazolyl, Pyrrolidirryl, Pyridinyl, Pyrimidinyl, Methyl-Pyrimidinyl oder Morpholinyl,
und in zwar in einem Lösungsmittel, bei Umgebungstemperatur und für 1 bis 12 Stunden, um die Verbindung der Formel (VIII) zu erhalten 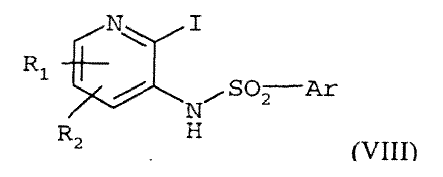
worin:
R₁, R₂ und Ar die gleiche Bedeutung wie bei den Ausgangsverbindungen behalten,
b) die Verbindung der Formel VIII mit einem Acetylenderivat folgender Formel reagieren zu lassen: 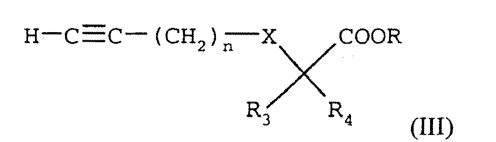 worin:
n = 1, 2 oder 3,
R₃ und R₄ jeweils unabhängig ein Wasserstoffatom oder eine C₁-C₄-Alkylgruppe darstellen,
R eine C₁-C₃-Alkylgruppe darstellt,
X eine einfache Bindung oder ein Sauerstoffatom darstellt,
und zwar unter ähnlichen Bedingungen wie sie für Schritt b) des Verfahrens nach Anspruch 9 beschrieben sind,
um die Verbindung folgender Formel zu erhalten 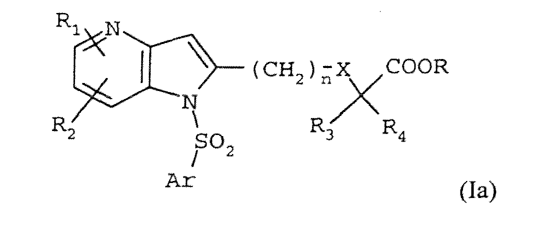 worin R₁, R₂, n, X, R₃, R₄, R und Ar die gleiche Bedeutung wie bei den Ausgangsverbindungen behalten,
c) falls erforderlich die Esterfunktion der Verbindung der Formel la zu hydrolysieren, beispielsweise durch Wirkung einer mineralischen Base, um nach der Säurebehandlung die Verbindung der Formel I in ihrer Form als freie Säure zu erhalten:
